# EUROPEAN PATENT APPLICATION

(11) **EP 4 725 969 A1**
(43) Date of publication of application: **15.04.2026**
(21) Application number: 24818770.0
(22) Date of filing: 07.06.2024
(51) Int. Cl.: C07K 16/46, A61K 39/395, A61P 35/00

(54) **ANTI-HER2 COMPLEMENTARY BISPECIFIC ANTIBODY-DRUG CONJUGATE, PREPARATION METHOD THEREFOR AND USE THEREOF**

(30) Priority: 09.06.2023 CN 202310684362; 10.08.2023 CN 202311002749; 09.11.2023 CN 202311490098; 01.03.2024 CN 202410237969
(71) Applicant: Latticon (Suzhou) Biopharmaceuticals Co., Ltd., Suzhou, Jiangsu 215164 (CN)
(72) Inventor: LI, John Yuehua, Suzhou, Jiangsu 215164 (CN)
(74) Representative: Müller Schupfner & Partner Patent- und Rechtsanwaltspartnerschaft mbB (Muc)
(86) International application number: PCT/CN2024/097980
(87) International publication number: WO 2024/251240

(57) **Abstract**

Provided are an anti-HER2 antibody or an antigen-binding fragment thereof, or an anti-HER2 complementary bispecific antibody constructed therefrom and a complementary bispecific antibody-drug conjugate (ADC), and a pharmaceutical composition and a kit comprising same. Also provided is a method of using the antibodies, ADC, pharmaceutical composition and kit for the treatment of HER2 abnormal expression-associated diseases.

## Description

### FIELD OF THE INVENTION

The present invention relates to anti-HER2 antibodies or antigen-binding fragments thereof, and biparatopic antibodies derived from such anti-HER2 antibodies and any antibody-drug conjugate comprising such biparatopic antibody. The present invention also provides methods of preparing the biparatopic antibody and the antibody-drug conjugate thereof, as well as methods of use for the threatment of diseases related to aberrant HER2 expression.

### BACKGROUND OF THE INVENTION

Human epidermal growth factor receptor 2 (HER2 or ErbB2) is a member of the receptor tyrosine kinase ErbB/HER family. The receptors of ErbB/HER family activate downstream signaling pathways after dimerization, thereby participating in the regulation of cell growth, differentiation, and survival. HER2 is a transmembrane glycoprotein with a molecular weight of approximately 185 kDa, consisting of an extracellular domain, a transmembrane domain, and an intracellular tyrosine kinase domain. Mutation and/or amplification of the ErbB2/HER2 gene can lead to HER2 overexpression, which is classified by immunohistochemistry (IHC) and fluorescence in situ hybridization (FISH) assay as IHC 3⁺ or IHC 2⁺/FISH⁺. Clinical studies have shown that HER2 is overexpressed in various cancers (e.g., breast cancer, ovarian cancer, endometrial cancer, gastric cancer, prostate cancer, and lung cancer, etc.), and HER2 overexpression is associated with tumor aggressiveness and poor prognosis.

Several HER2-targeted therapeutics have been approved for treating HER2-overexpressing breast cancer and gastric cancer, which have significantly improved patient survival. However, a considerable number of cancer patients do not respond to these therapies. Additionally, existing HER2-targeted therapies have the following drawbacks, which limited their clinical efficacy and/or posed safety risks:
(1) Current HER2-targeted therapeutics are ineffective in eradicating cancer cells expressing relatively low level of HER2, such as those classified as IHC 2⁺/FISH⁻ or IHC 1⁺ (defined as "HER2-low expression"). Tumor cells expressing low levels of HER2 often develop resistance to the existing therapies due to the high heterogeneity of HER2 expression in HER2-overexpressing tumors. As a result, most cancer patients develop drug resistance and exprience disease progression after 6-12 months of the treatment of current HER2-targeted therapies.
(2) Among the breast cancer patients, approximately 70% are classified as HER2-low-expression. Currently, DS-8201 is the only approved HER2-targeting drug in this patient population; however, in the phase III DESTINY-Breast04 clinical trial it showed only a ~50% response rate, with a 12.1% incidence of drug-related interstitial lung disease/pneumonia (Modi et al., N Engl J Med 2022, 387:9-20). Obviously, there exists substantial and urgent needs for improving the efficacy and safety of current HER2-targeted therapies to treat HER2-low-expressing tumors.
(3) HER2-mediated signal transduction pathways are critical for maintaining cardiomyocyte survival, repairing cardiomyocyte damage, and preserving the functional integrity of cardiomyocytes. Currently approved anti-HER2 therapies, including antibodies, ADCs, and small-molecule inhibitors, can disrupt HER2 signaling, leading to cardiotoxicity. For example, trastuzumab and pertuzumab may cause reduced left ventricular ejection fraction (LVEF) and congestive heart failure. Particularly, trastuzumab has a higher risk of inducing heart failure when co-administered with anthracyclines, therefore it requires strict screening of eligible patients or prophylactic cardioprotective therapy prior to the treatment with these agents.

Thus, there remains a significant unmet clinical need to develop a safer (especially addressing the cardiotoxicity caused by current HER2-targeting therapies) and more potent targeted therapy against HER2-low-expressing cancer cells to overcome the safety and resistance limitations of current therapies.

### SUMMARY OF THE INVENTION

The present invention provides a class of HER2-targeting biparatopic antibody-drug conjugate. In one aspect, the biparatopic ADC can specifically bind to two different epitopes on the extracellular domain (also referred to as the "extracellular region") of HER2, which can cross-link HER2 on the cell surface to form meshwork multimers, thereby promting rapid and efficient endocytosis and altering the intracellular endosome trafficking pathway from recycling to lysosomal trafficking. As a result, the ADC bound to the cell surface is efficiently transported (almost 100%) into lysosomes for degradation, and thus more small-molecule toxins are released into the cytoplasm to exert cytotoxicity and subsequent bystander killing effect. In this way, the biparatopic ADC exhibits a broader spectrum of target cell-killing activity. It can not only kill HER2-overexpressing cancer cells, but also can kill cancer cells expressing low levels of HER2 (IHC 2+/FISH- or IHC 1+) directly, thereby significantly reducing the probability of developing drug resistance due to the heterogeneity of HER2 expression in tumors. In another aspect, the binding epitopes of the biparatopic ADC are distinct from those of trastuzumab and pertuzumab, which do not interfere with HER2 dimerization or affect the regulation of HER2-mediatied downstream signaling pathways, thus the biparatopic ADC shall not interfere with the normal biological function of HER2 on cardiomyocytes.

In one aspect, the present invention provides isolated anti-HER2 antibodies or antigen-binding fragments thereof, wherein the anti-HER2 antibodies or antigen-binding fragments thereof can specifically bind to the extracellular domain of HER2, having high binding activity or binding affinity to HER2-expressing tumor cells (for example, the value of binding affinity constant [K_{D}] is <1×10⁻⁸ M, <5×10⁻⁹ M, or <1×10⁻⁹ M), and with no cross-reactivity to the other members of ErbB/HER family (including EGFR, HER3, and HER4). The anti-HER2 antibodies or antigen-binding fragments thereof have no antagonistic or agonistic activity on HER2 or its downstream signal transduction pathways.

In some embodiments, the anti-HER2 antibodies include anti-mouse HER2 antibody and its derived chimeric antibody, humanized antibody, and/or optimized antibody. In some embodiments, the anti-HER2 antibodies or antigen-binding fragments thereof specifically bind to subdomain I, III, or IV of the HER2 extracellular domain, preferably subdomain I or III. In some embodiments, the paired anti-HER2 antibodies or antigen-binding fragments thereof used to construct the anti-HER2 biparatopic antibody specifically recognize different epitopes on the extracellular domain of HER2 and do not compete each other for binding.

In one aspect, the present invention provides an anti-HER2 biparatopic antibody, comprising a first antigen-binding domain and a second antigen-binding domain, wherein each antigen-binding domain specifically recognizes a distinct epitope on HER2, enabling the biparatopic antibody to cross-link HER2 at the cell surface and induce cluster formation. The formation of cluster is largely dependent on the HER2 density on cell surface. Formation of cluster on cell surface will lead to a rapid internalization and lysosomal degradation. Compared to the mono-specific anti-HER2 antibodies described in the present invention and/or trastuzumab, the biparatopic antibody can elicit a significantly enhanced HER2 internalization and lysosomal trafficking/degradation.

In some embodiments, the first and second antigen-binding domains of the anti-HER2 biparatopic antibody specifically bind to two distinct (non-overlapping or non-competing) epitopes on HER2, wherein the epitopes comprise sequences located in subdomain I, III, and/or IV of the HER2 extracellular domain. In one embodiment, the first antigen-binding domain specifically binds to subdomain III of the HER2 extracellular domain, and the second antigen-binding domain specifically binds to subdomain I of the HER2 extracellular domain; both epitopes are distinct from those bound by trastuzumab or pertuzumab. In one embodiment, the biparatopic antibody neither inhibits nor activates HER2 or its downstream signaling pathways in cells. In one embodiment, the biparatopic antibody induces rapid receptor internalization and efficient lysosomal trafficking upon binding and cross-linking HER2 on cell surface. In one embodiment, the biparatopic antibody is capable of inducing degradation of HER2 on cell surface. In one embodiment, the biparatopic antibody induces HER2 internalization and lysosomal degradation by cross-linking HER2 on cell surface, resulting in significantly reduced HER2 expression on cell surface and therefore effectively inhibiting the HER2-overexpressing tumor cell proliferation.

In one aspect, the present invention provides an ADC comprising a small-molecule toxin compound conjugated to the disclosed anti-HER2 biparatopic antibody via a linker. The ADC can specifically bind to and cross-link HER2 on tumor cell surface to form aggregated "clusters", which lead to rapid internalization and lysosomal trafficking, as well as ADC degradation in the lysosomes, resulting in significantly increased release of toxin into the cytoplasm of tumor cells. Therefore, the biparatopic ADC in comparison to T-DM1 and/or DS-8201 exhibits a broader spectrum of tumor cell-killing activity, i.e., the biparatopic ADC can effectively kill both HER2-overexpressing and HER2-low-expressing tumor cells. The ADC has a formula (I) as follows:

Ab-(L-D)p (I),

wherein
Ab is an anti-HER2 biparatopic antibody of the present invention;
D is a small-molecule toxin compound (Drug);
L is a cleavable linker that conjugates Ab to D; and
p represents the copy number of (L-D) conjugated to Ab, which ranges from 2 to 8.

In some embodiments, the small-molecule toxin compound includes cytotoxins and chemotherapeutic agents. In one embodiment, the small-molecule toxin compound is a cytotoxin, including tubulin inhibitors and DNA-alkylating agents. Preferably, tubulin inhibitors include eribulin, auristatin derivatives (e.g., MMAE, MMAF, MMAD), tubulysins, cryptomycins, maytansinoid derivatives (e.g., DM1, DM2, DM3, DM4); the DNA-alkylating agents include topoisomerase inhibitors (e.g., camptothecin derivatives such as SN-38, exatecan, and DXd [exatecan derivative for ADC]), pyrrolobenzodiazepines (PBDs), calicheamicin and derivatives thereof (e.g., N-acetyl calicheamicin [CMC]), duocarmycins. In one embodiment, the small-molecule toxin compound is eribulin.

In some embodiments, the cleavable linker can be any linker comprising a cleavable moiety, wherein the cleavable moiety comprises any cleavable chemical bond. In some specific embodiments, the cleavable linker comprises a cleavable peptide moiety that can be cleaved by intracellular peptidases or proteases, wherein the cleavable peptide moiety comprises an amino acid unit, such as dipeptides, tripeptides, or tetrapeptides. In some specific embodiments, the cleavable linker comprises at least one spacer conjugating the anti-HER2 biparatopic antibody (Ab) of the present invention to the small-molecule toxin compound (D), wherein the spacer comprises one spacer conjugated to the antibody and/or the second spacer conjugated to the small-molecule toxin compound. In some embodiments, the spacer conjugated to the antibody is hydrophilic, and an exemplary spacer includes polyethylene glycol (PEG). In some examples, the spacer is attached to the anti-HER2 biparatopic antibody of the present invention via a maleimide moiety (Mal). In some embodiments, the second spacer serves to connect the cleavable moiety (e.g., a cleavable peptide) of the cleavable linker to the small-molecule toxin compound. In some examples, the second spacer conjugated to the small-molecule toxin compound is self-immolative, wherein the self-immolative spacer comprises a *p*-aminobenzyl unit. In some specific embodiments, the cleavable linker comprises a Mal-spacer and a cleavable peptide moiety. In some specific embodiments, the Mal-spacer in the cleavable linker is conjugated to one or more amino acid residues of the antibody component of the ADC of the present invention. In some embodiments, the maleimide group of the Mal-spacer can react with the thiol group of cysteine residue at specific positions in the constant and/or variable regions of the antibody. In some specific embodiments, the second spacer in the cleavable linker is conjugated to the small-molecule toxin compound of the ADC of the present invention, wherein the small-molecule toxin compound is eribulin or a derivative thereof. In some specific embodiments, the cleavable moiety (e.g., cleavable peptide) in the cleavable linker is directly conjugated to the small-molecule toxin compound of the ADC, wherein the small-molecule toxin compound is eribulin or a derivative thereof.

In some embodiments, p ranges 2 to 8, for example, 4 to 8. As used herein, p is an integer or non-integer greater than 0.

In another aspect, the present invention relates to isolated nucleic acid molecules (also referred to as "polynucleotide") encoding the anti-HER2 biparatopic antibody and its corresponding monospecific antibodies or antigen-binding fragments thereof, as well as expression vectors comprising the nucleic acid molecules, and host cells comprising the nucleic acid molecules or the expression vectors. The present invention also relates to methods for the preparation of anti-HER2 monospecific antibodies or antigen-binding fragments thereof and the anti-HER2 biparatopic antibody disclosed herein using the host cells, wherein the methods comprise culturing the host cells and recovering the antibodies or antigen-binding fragments thereof from the culture media.

In another aspect, the present invention relates to a pharmaceutical composition comprising the anti-HER2 monospecific antibodies or antigen-binding fragments thereof, the anti-HER2 biparatopic antibody, or the anti-HER2 biparatopic antibody-drug conjugate as described herein, and a pharmaceutically acceptable carrier.

In another aspect, the present invention relates to a kit comprising an effective amount of the anti-HER2 monospecific antibodies or antigen-binding fragments thereof, the anti-HER2 biparatopic antibody, the anti-HER2 biparatopic antibody-drug conjugate, or the pharmaceutical composition of the present invention, and optionally at least one additional anti-cancer therapeutic agent.

In another aspect, the present invention relates to methods of treating HER2-expressing tumors in a subject, wherein the methods comprise administering to the subject in need the anti-HER2 biparatopic antibody, the anti-HER2 biparatopic antibody-drug conjugate, the pharmaceutical composition, or the kit of the present invention. Alternatively, the present invention relates to use of the anti-HER2 biparatopic antibody, the anti-HER2 biparatopic antibody-drug conjugate, the pharmaceutical composition, or the kit as described herein in the manufacture of a medicament for treating HER2-expressing tumors in a subject. Alternatively, the present invention relates to use of the anti-HER2 biparatopic antibody, the anti-HER2 biparatopic antibody-drug conjugate, the pharmaceutical composition, or the kit as described herein in the treatment of HER2-expressing tumors in a subject. In some embodiments, the tumors include HER2-overexpressing tumor (IHC 3⁺, or IHC 2⁺/FISH⁺) and/or low HER2-expressing tumor (IHC 2⁺/FISH⁻, or IHC 1⁺). In some embodiments, the anti-HER2 biparatopic antibody-drug conjugate or pharmaceutical composition thereof exhibits cytotoxic effects against HER2-overexpressing and/or low HER2-expressing tumor cells. In some embodiments, the subject is a mammal. In some embodiments, the tumors include breast cancer, ovarian cancer, cervical cancer, colorectal cancer, gastric cancer, esophageal cancer, lung cancer, head and neck cancer, bladder cancer, melanoma, pancreatic cancer, liver cancer, cholangiocarcinoma, kidney cancer, bladder cancer, thyroid cancer, prostate cancer, endometrial cancer, etc., wherein the tumor is HER2-overexpressing or low HER2-expressing.

In some embodiments, the tumor is resistant to existing HER2-targeting therapies. In some embodiments, the tumor is resistant to the HER2-targeting therapeutic agents including trastuzumab, pertuzumab, T-DM1, and/or DS-8201. In one embodiment, the tumor is unresponsive or poorly responsive to HER2-targeting therapeutic agents including trastuzumab, pertuzumab, T-DM1, DS-8201 and/or taxanes (e.g., paclitaxel, docetaxel, cabazitaxel, etc.).

In some embodiments, the subject includes a patient who is ineligible for or refractory to existing HER2-targeting therapies or have developed resistance or relapse after receiving existing HER2-targeting therapies.

In another aspect, the present invention relates to a method of detecting and/or measuring HER2 or HER2-expressing tumor cells in a specimen, or a method of screening cancer patients who may respond to the treatment using the anti-HER2 biparatopic ADC described herein, wherein the method comprises incubating the anti-HER2 monospecific antibody or antigen-binding fragment thereof, or the anti-HER2 biparatopic antibody of the present invention with the specimen isolated from the patient and detecting whether the antibody binds to the specimen.

Other features and advantages of the present invention will become apparent from the following detailed description of examples and drawings. It should be understood, however, that the drawings and specific embodiments should not be construed as limiting the scope of the present invention, and various changes and modifications within the spirit and scope of the present invention which will become apparent to those skilled in the art from this detailed description are included in the scope of protection of the appended claims. The contents of all references, including publications, patents and published patent applications, cited in the present invention are incorporated by reference in their entirety.

### DESCRIPTION OF DRAWINGS

FIGURE 1 shows the detection of binding activity of anti-HER2 chimeric antibodies to human ErbB/HER family members EGFR, HER2, HER3, and HER4 by ELISA, with the control antibodies including trastuzumab, cetuximab, and patritumab.
FIGURE 2 shows the detection of binding activity of anti-HER2 chimeric antibodies to HER2-overexpressing cell line NCI-N87 by flow cytometry, with trastuzumab as the control antibody.
FIGURE 3 shows the schematic structures of recombinant human-mouse chimeric proteins of the HER2 extracellular domain (Fig 3A), and the results of ELISA detecting the subdomain of HER2 extracellular domain where each anti-HER2 chimeric antibody binds (Fig 3B).
FIGURE 4 shows the detection of binding competition between the anti-HER2 chimeric antibodies or with the control antibody trastuzumab by competitive ELISA; wherein Fig 4A shows the results of biotin-labeled mAb2164 competing with mAb2117, mAb2126, mAb2170, and trastuzumab, respectively, for binding of the recombinant HER2 protein; Fig 4B shows the results of mAb2117 competing with biotin-labeled mAb2128 or biotin-labeled mAb2126 for binding of the recombinant HER2 protein, and mAb2164 competing with biotin-labeled mAb2128 for binding of the recombinant HER2 protein.
FIGURE 5 shows the effect of anti-HER2 chimeric antibodies mAb2117 and mAb2126 on the phosphorylation of Y1248 site within the HER2 intracellular domain in SKBR-3 cells, with trastuzumab and pertuzumab as the control antibodies.
FIGUER 6 shows the effect of anti-HER2 chimeric antibodies mAb2117 and mAb2126 on the NRG-1-induced AKT phosphorylation in T47D cells, with trastuzumab and pertuzumab as the control antibodies.
FIGURE 7 shows the dectetion of binding activity of optimized anti-HER2 scFv muteins to BT474 cells (Fig 7A) and RT-112 cells (Fig 7B) by flow cytometry, with the parental scFv Hu2117HK as the control antibody.
FIGURE 8 shows the detection of binding activity of optimized anti-HER2 scFv molecules containing single- or multi-point mutations (including Hu2117-HK203, Hu2117-HK303, Hu2117-HK304, Hu2117-HK309, and Hu2117-HK310) to BT474 cells and RT-112 cells by flow cytometry, with the parental scFv Hu2117HK as the control antibody.

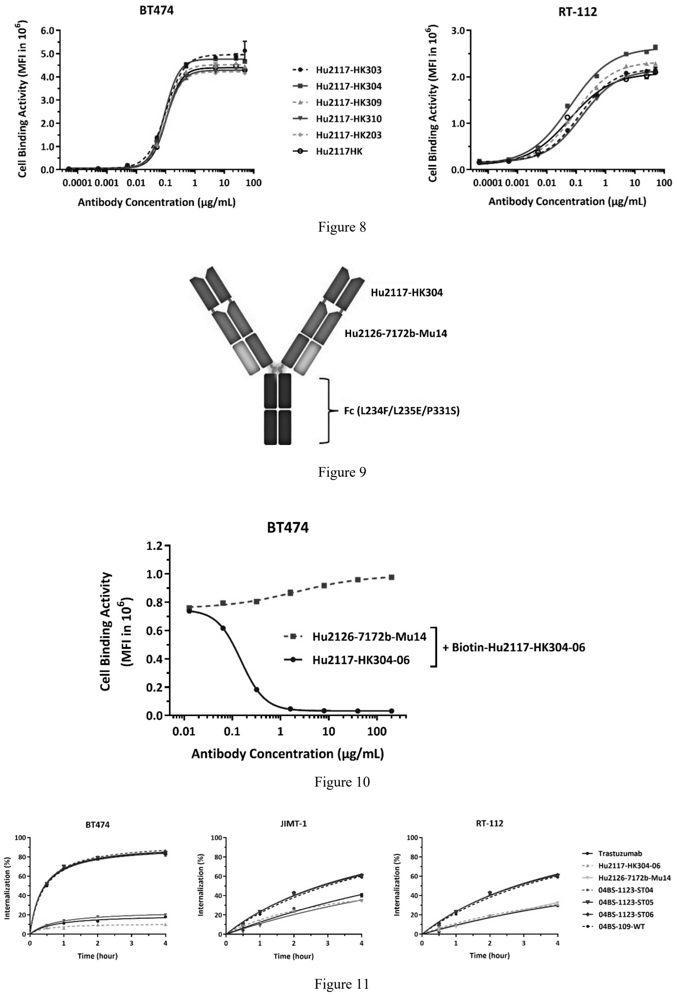

FIGURE 9 shows schematic structure of a representative anti-HER2 biparatopic antibody 04BS-109-WT. The said biparatopic antibody is constructed in DVD-IgG configuration, wherein the Fv domain comprises the variable region sequence of Hu2117-HK304 and the IgG domain comprises the full-length sequence of Hu2126-H2K1-L71-H72b-Mu14, while the heavy chain variable region of the Fv domain is connected to the heavy chain of the IgG domain via a linker (G₄S)₁, the light chain variable region of the Fv domain is connected to the light chain of the IgG domain via a linker (G₄S)₃, and the heavy chain Fc region of the antibody contains site mutations L234F/L235E/P331S (EU Numbering).
FIGURE 10 shows the detection of concurrent- or competitive-binding between the humanized anti-HER2 antibodies Hu2117-HK304-06 and Hu2126-H2K1-L71-H72b-Mu14 (abbreviated as Hu2126-7172b-Mu14) to BT474 cells by flow cytometry.
FIGURE 11 shows the detection of internalization kinetics of the anti-HER2 biparatopic antibody 04BS-109-WT and its variants in cells expressing different levels of HER2 (e.g., BT474, JIMT-1, RT-112) by flow cytometry, with the control antibodies including trastuzumab, as well as Hu2117-HK304-06 and Hu2126-H2K1-L71-H72b-Mu14 (abbreviated as Hu2126-7172b-Mu14), the two monospecific antibodies used to construct the biparatopic antibody 04BS-1123-ST06.
FIGURE 12 shows the detection of internalization and lysosomal trafficking induced by the anti-HER2 biparatopic antibody 04BS-1123-ST06 in SKBR-3 cells by confocal microscopy, with the control antibodies includind trastuzumab and human IgG isotype control. Arrowheads denote the co-localization of fluorescent signals of antibody and lysosome, demonstrating that HER2 aggregation upon cross-linking by the biparatopic antibody are internalized and transported into lysosomes. T indicates the antibody incubation time (hours).
FIGURE 13 shows the detection of HER2 protein degradation in BT474 cells after incubation with the anti-HER2 biparatopic antibody 04BS-1123-ST06 by Western-blot, with the control antibodies including trastuzumab, as well as Hu2117-HK304-06 and Hu2126-H2K1-L71-H72b-Mu14 (abbreviated as Hu2126-7172b-Mu14), the two monospecific antibodies used to construct the biparatopic antibody.
FIGURE 14 shows the effect of anti-HER2 biparatopic antibody 04BS-1123-ST06 on BT474 cell proliferation *in vitro,* with the control antibodies including trastuzumab, pertuzumab, as well as Hu2117-HK304-06 and Hu2126-H2K1-L71-H72b-Mu14 (abbreviated as Hu2126-7172b-Mu14), the two monospecific antibodies used to construct the biparatopic antibody.
FIGURE 15 shows the effect of anti-HER2 monospecific antibodies (Fig 15A) and anti-HER2 biparatopic antibodies (Fig 15B) on NRG-1-induced HER2/HER4 dimerization by a reporter gene assay, with the control antibodies including trastuzumab and pertuzumab.
FIGURE 16 shows the effect of anti-HER2 biparatopic antibody 04BS-1123-ST06 on NRG-1-induced AKT phosphorylation in human cardiomyocytes, with the control antibodies including trastuzumab and pertuzumab.
FIGURE 17 shows the detection of ADCC activity of the anti-HER2 biparatopic antibody 04BS-1123-ST06 by a reporter gene assay, with trastuzumab as the positive control antibody.
FIGURE 18 shows the detection of binding specificity of the anti-HER2 biparatopic ADC (ST06-VCP-Eribulin) to human ErbB/HER family members (including EGFR, HER2, HER3, and HER4) by ELISA.
FIGURE 19 shows the detection of *in vitro* cell-killing activity of the anti-HER2 biparatopic ADC (including ST06-GGFG-Eribulin and ST06-VCP-Eribulin) in comparison to the benchmark molecule DS-8201 against a panel of cancer cell lines expressing different levels of HER2.

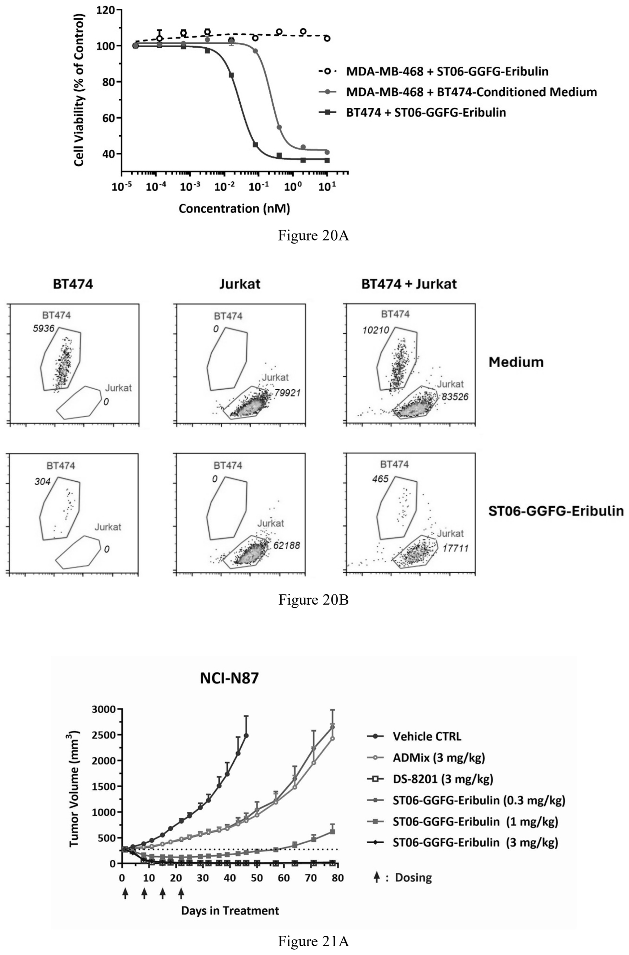

FIGURE 20 shows the bystander effect of anti-HER2 biparatopic ADC detected by *in vitro* cell-killing assay. In Fig 20A, BT474 cells were treated with ST06-GGFG-Eribulin for 3 days and the cytotoxicity was confirmed by the *in vitro* cell-killing assay, in the meantime the BT474-conditioned medium was collected and then used for incubating with MDA-MB-468 cells for 3 days. The viability of MDA-MB-468 cells was measureded by the *in vitro* cell-killing assay to detect the bystander killing effect, wherein the control group was the MDA-MB-468 cells incubated in parallel with freshly prepared ST06-GGFG-Eribulin. In Fig 20B, HER2-overexpressing BT474 cells and HER2-null Jurkat cells was treated with ST06-GGFG-Eribulin in either individual culture or co-culture, and cell viability was then detected by flow cytometry, wherein the numbers shown in the upper-left and lower-right corners are the viable cell counts for BT474 and Jurkat, respectively.
FIGURE 21 shows the detection of *in vivo* anti-tumor activity of the anti-HER2 biparatopic ADC (ST06-GGFG-Eribulin) in mouse subcutaneous xenograft tumor models established with tumor cell lines NCI-N87 (Fig 21A), JIMT-1 (Fig 21B) and RT-112 (Fig 21C), wherein the controls include the mixture of the antibody and the small molecule compound (ADMix) group, the DS-8201 group and the vehicle group. All tumor-bearing mice were dosed via intravenous injections and the arrows indicate the time of dosing.
FIGURE 22 shows the detection of *in vivo* anti-tumor activity of the anti-HER2 biparatopic ADC (ST06-GGFG-Eribulin) in a mouse subcutaneous xenograft tumor model with acquired resistance to DS-8201, with control groups including DS-8201 and vehicle.

### DETAILED DESCRIPTION OF THE INVENTION

### Definition:

Unless otherwise defined, technical and scientific terms used herein have the same meaning as those commonly understood by a person skilled in the art. For the purposes of the present invention, the following terms are defined below.

As used herein, "HER2" and "HER2 receptor" can be used interchangeably, and this protein is also referred to as ErbB2, c-ERB2, c-ERB-2, NEU, HER-2/neu, p185(erbB2), or CD340. Unless explicitly specified as deriving from a non-human species (e.g., "mouse HER2," "monkey HER2," etc.), "HER2" as used herein refers to any natural form of human HER2, which can have the amino acid sequence set forth in SEQ ID NO: 234 and/or the full-length HER2 amino acid sequence as shown in NCBI Accession No. NP_004439.2, and can be naturally expressed by cells (including tumor cells) or expressed by cells transfected with the HER2 gene or cDNA. This term includes allelic and splice variants, isoforms, homologs, and species homologues of naturally occurring HER2. HER2 can be isolated from human body, or can be produced by recombinant or synthetic methods.

HER2 extracellular domain consists of four subdomains: Subdomain 1 (D1, approximately amino acid residues 1-195), Subdomain 2 (D2, approximately amino acid residues 196-319), Subdomain 3 (D3, approximately amino acid residues 320-488), and Subdomain 4 (D4, approximately amino acid residues 489-630) (residue numbering excludes the signal peptide), among which D2 and D4 are cysteine-rich domains responsible for receptor dimeraization (Garrett et al., Mol Cell 2003, 11:495-505; Cho et al., Nature 2003, 421:756-760; Franklin et al., Cancer Cell 2004, 5:317-328).

As used herein, "cells expressing HER2" can be naturally occurring cells or cell lines (e.g., tumor cells) or can be recombinantly produced by introducing nucleic acids encoding HER2 into host cells.

As used herein, "bispecific antibody" is intended to include any antibody or antigen-binding fragment capable of specifically binding to two different epitopes (or antigenic determinants), which comprises two independent antigen-binding domains and each with unique antigen-binding specificity. For example, the "biparatopic antibody" described herein refers specifically to a class of bispecific antibodies, wherein the first antigen-binding domain and the second antigen-binding domain bind respectively to different epitopes on the same antigen.

A monospecific antibody refers to an antibody or antigen-binding fragment with only one binding specificity, meaning that the antigen-binding domain of a monospecific antibody binds to a single epitope on a single antigen. In some embodiments, examples of the monospecific antibody include the anti-HER2 monospecific antibodies of the present invention.

As used herein, "antigen-binding domain" or "antigen-binding region" or "epitope-binding domain" or "antigen-binding polypeptide" can be used interchangeably and refers to a specific region on an antibody or antigen-binding fragment or derivative thereof that is directly involved in specific interaction with a target antigen, for example, interaction with the target antigen by binding, steric hindrance, stabilizing/destabilizing, steric distribution and other mechanisms to achieve dynamic equilibrium.

In the present invention, "antigen-binding domain" also refers to a specific region on the antibody or antigen-binding fragment or derivative thereof that interacts with a specific epitope on HER2, and the interaction between the two achieves dynamic equilibrium by binding, steric hindrance, stabilizing/destabilizing, spterial distribution and other mechanisms.

As used herein, "antibody" refers to a polypeptide or protein capable of specifically recognizing and binding to an antigen, generally encoded by one or more immunoglobulin genes or fragments thereof. Recognized immunoglobulin genes include κ, λ, α, γ, δ, ε and µ constant region genes, as well as countless immunoglobulin variable region genes. Light chains are classified as κ or λ. Heavy chains are classified as y, µ, α, δ or ε, each of which in turn defines the immunoglobulin classes or isotypes as IgG, IgM, IgA, IgD, and IgE. Some of these classes can be further divided into subclasses, such as IgG1, IgG2, IgG3, IgG4, IgA1, and IgA2. The structural unit of typical immunoglobulin (e.g., antibody) is a tetramer. Each tetramer consists of two identical pairs of polypeptide chains, each pair containing a "light" chain (about 25 kDa) and a "heavy" chain (about 50-70 kDa). The N-terminus of each chain defines a variable (V) region primarily responsible for antigen recognition, having about 100 to 110 or more amino acids. The heavy chain of an antibody consists of a heavy chain variable region (VH) and a heavy chain constant region (CH), wherein the heavy chain constant region typically includes three domains: CH1, CH2, and CH3. The light chain is composed of a light chain variable region (VL) and a light chain constant domain (CL), wherein the light chain constant domain usually contains one domain, CL. The pairing of VH and VL forms a single antigen-binding site. Endogenous VL is encoded by V (variable) and J (joining) gene segments, and endogenous VH is encoded by V, D (diversity), and J gene segments. VL or VH comprises region of hypervariability or complementarity determining regions (CDR), and framework regions (FR). The term "variable region" or "V region" can be used interchangeably and refers to either a heavy chain variable region or a light chain variable region, arranged in an order from the amino to the carboxyl terminus: FR1-CDR1-FR2-CDR2-FR3-CDR3-FR4. The term "J region" refers to the subsequence that encodes the C-terminal portion of the variable region comprising CDR3 and FR4. V regions or J regions can be naturally occurring, recombinant, or synthetic. As used herein, the light chain variable region and/or heavy chain variable region of an antibody can be collectively referred to as an "antibody variable region", and the light chain and/or heavy chain of an antibody can be collectively referred to as "antibody chain". In certain embodiments, the FRs of an antibody or antigen-binding fragment thereof provided herein can be identical to the human germline sequence, or be naturally or artificially modified.

The positions of CDRs and FRs can be determined using a variety of definition methods well known in the art, e.g., Kabat, Chothia, IMGT, and Contact (e.g., Kabat et al. Sequences of Proteins of Immunological Interest, 1991, Fifth Edition, NIH Publication No. 91-3242; Johnson et al., Nucleic Acids Res 2001, 29:205-206; Chothia & Lesk, J Mol Biol 1987, 196:901-917; Chothia et al., Nature 1989, 342:877-883; Chothia et al., J Mol Biol 1992, 227:799-817; Al-Lazikani et al., J Mol Biol 1997, 273:927-748; Lefranc MP et al., Nucleic Acids Research 1999, 27:209-212; MacCallum RM et al., J Mol Biol 1996, 262:732-745). Definitions of antigen-binding sites are also described in: Ruiz et al., Nucleic Acids Res 2000, 28:219-221; Lefranc MP, Nucleic Acids Res 2001, 29:207-209; Lefranc MP, The Immunologist 1999, 7:132-136; Lefranc MP et al., Dev Comp Immunol 2003, 27:55-77; MacCallum et al., J Mol Biol 1996, 262:732-745; Martin et al., Proc Natl Acad Sci USA 1989, 86:9268-9272; Martin et al., Methods Enzymol 1991, 203:121-153; Sternberg MJE (edition), Protein Structure Prediction: A Practical Approach, 1996, Oxford University Press, 141-172. The present invention includes any one of the definition methods to determine the CDRs in the anti-HER2 biparatopic antibody or the anti-HER2 antibody or antigen-binding fragment thereof of the present invention. Table 1 shows the amino acid residues of the antibody CDRs determined by different definition methods. The exact number of amino acid residues encompassing a particular CDR varies with the CDR sequence. With the amino acid sequence of the variable region of an antibody being clarified, those skilled in the art can determine the CDR of the antibody by conventional definition methods, including but not limited to the definition methods herein.

**Table 1. CDR¹ identified by different definition methods**

| CDRs | Kabat | Chothia | IMGT | Contact |
|---|---|---|---|---|
| HCDR1 | 31-35 | 26-32 | 27-38 | 30-35 |
| HCDR2 | 50-65 | 52-56 | 56-65 | 47-58 |
| HCDR3 | 95-102 | 95-102 | 105-117 | 93-101 |
| LCDR1 | 24-34 | 24-34 | 27-38 | 30-36 |
| LCDR2 | 50-56 | 50-56 | 56-65 | 46-55 |
| LCDR3 | 89-97 | 89-97 | 89-97 | 89-96 |

| | | | | |
|---|---|---|---|---|
| ¹: All CDR definitions in Table 1 are numbered according to the numbering system proposed by Kabat et al. (Kabat et al., Sequences of Proteins of Immunological Interest, 1991, Fifth Edition, NIH Publication No. 91-3242). | | | | |

In addition, Kabat et al. have defined a numbering system for variable region sequences, which can be applied to any antibody. A person of ordinary skill in the art would be able to unambiguously apply this "Kabat numbering" system to the variable region sequence of any antibody without relying on any experimental data other than the antibody sequence itself to determine the variable region sequence. Unless otherwise stated, the numbering of specific amino acid residue positions in the variable region of the antigen-binding domains of the anti-HER2 antibodies, anti-HER2 biparatopic antibodies, or biparatopic ADCs described in the present invention is determined according to the Kabat numbering system.

An antibody exists as an intact immunoglobulin or as many well-characterized fragments produced by protease digestion. Although various antibody fragments are defined based on the digestion of intact antibodies, those skilled in the art should understand that such fragments can be generated either by chemical cleavage methods or recombinant DNA methods. As used herein, the term "antigen-binding fragment" (or abbreviated as "antibody portion" or "antibody fragment") of an antibody herein refers to a portion of an antibody comprising one or more CDRs, or any other antibody fragment that can bind to an antigen (such as HER2 or HER2 extracellular domain) without the entire antibody structure. Antigen-binding fragments can exhibit the same antigen-specific binding activity as intact antibodies. A preferred antigen-binding fragment retains the ability to be internalized into cells expressing the target antigen. In some embodiments, the antigen-binding fragment may contain one or more CDRs from a certain human antibody, grafted to one or more framework regions from a different human antibody. The antigen-binding fragment includes but is not limited to: (i) "Fab" fragments, which are monovalent antibody fragments consisting of VH, VL, CL, and CH1 domains; (ii) "F(ab')2" fragments, which are bivalent fragments containing two Fab fragments linked by disulfide bonds in the hinge region; (iii) "Fv" fragments, which consist of the VL and VH domains from a single antibody arm and are the smallest antibody fragments containing a complete antigen-binding site; (iv) "Fd" fragments, which are composed of VH and CH1 domains; (v) "single-chain Fv antibodies (scFv)" or "single-chain antibodies", referring to engineered antibodies composed of a light chain variable region linked to a heavy chain variable region directly or by a peptide chain (Huston et al., Proc Natl Acad Sci USA 1988, 85:5879-5883; Bird et al., Science 1988, 242:423-426). These scFv antibodies can be internalized into cells when binding to antigens on the cell surface (He et al., J Nucl Med 2010, 51:427-432; Fitting et al., MAbs 2015, 7:390-402). In addition, single-chain antibodies also include "linear antibodies" containing tandem Fv segments (VH-CH1-VH-CH1), which form a pair of antigen-binding regions when paired with complementary light chain polypeptides (Zapata et al., Protein Eng 1995, 8:1057-1062; US5641870); (vi) "dAb" fragments (Ward et al., Nature 1989, 341:544-546; WO90/05144A1) containing a single variable domain, such as a VH domain. Single-domain antibodies (sdAb) are independent immunoglobulin domains; (vii) "Diabodies" which are bivalent bispecific antibodies, wherein VH and VL domains are expressed on a single polypeptide chain, but with the linker too short to allow the two domains on the same chain pairing with each other, thus forcing them to pair with the complementary domains of the other chain to form two antigen-binding sites (Holliger et al., Proc Natl Acad Sci USA 1993, 90:6444-6448; Poljak et al., Structure 1994, 2:1121-1123; EP404097; WO93/11161).

As used herein, the term "Fc region" or "Fc domain" refers to the C-terminal region of an immunoglobulin heavy chain, which contains at least a portion of the constant region, such as an immunoglobulin heavy chain constant region excluding the first constant region (CH1). For IgG, the Fc region can comprise the immunoglobulin domains CH2 and CH3, as well as the hinge region between CH1 and CH2. As used herein, the Fc region includes the native Fc region and/or Fc region variants and can be a portion of the anti-HER2 antibody, anti-HER2 biparatopic antibody or ADC thereof described in the present invention. It would be understood in the art that the boundary of the Fc region may vary, and however, a human IgG heavy chain Fc region is generally defined as comprising either a cysteine residue at position 226 or a proline residue at position 230 at its amino-terminus, according to the EU numbering system/scheme as described in Kabat et al., Sequences of Proteins of Immunological Interest, 1991, 5th edition, NIH Publication No. 91-3242.

The term "anti-HER2 antibody" or "HER2-specific antibody" refers to any form of antibody or fragment thereof that specifically binds to HER2, and encompasses monoclonal antibodies (including full-length monoclonal antibodies), polyclonal antibodies, and biologically functional antibody fragments, provided that the fragments specifically bind to HER2.

The term "specifically bind/binding" or "binding specificity" or "specific for" or "binding" herein refers to a binding reaction that determines the presence of a target molecule (or protein or antigen) in a heterogeneous population of proteins and other biological agents (e.g., in biological samples, such as blood, serum, plasma or tissue samples), that is, the binding is selective for the target molecule and can distinguish from those undesired or nonspecific interactions. For example, an antibody that specifically binds to a target molecule (which may be an antigen) exhibits higher affinity, stronger binding activity, easier binding and/or prolonged binding duration to the target molecule compared to its binding to other non-target moleclules. Therefore, as needed, such selection can be achieved by eliminating antibodies that cross-react with other members of the ErbB/HER family. Various immunoassays can be employed to select antibodies that specifically react with a particular protein, such as ELISA. Typically, under defined assay conditions, a specific or selective binding reaction between an antibody or binding agent and its antigen should generate a signal at least two-fold above the background level, more generally at least 10-100 fold above the background, and demonstrate substantially no significant binding to other antigens present in the sample. In some embodiments, the antibody specifically binds to the target antigen with a dissociation equilibrium constant (K_{D}) value of <1 µM, <100 nM, <10 nM, <1 nM, or <0.1 nM.

The term "monoclonal antibody" herein refers to an antibody obtained from a population of antibodies that is substantially homogeneous, i.e., the individual antibodies that make up the population are identical except for natural mutations that may be present in minor amounts. A monoclonal antibody exhibits high binding specificity and affinity for a particular epitope. Monoclonal antibodies can be produced by the hybridoma method first described by Kohler et al., Nature 1975, 256:495, or by recombinant DNA methods (see US4816567), and can also be isolated from phage antibody libraries using techniques such as those described in Clackson et al., Nature 1991, 352:624-628; Marks et al., J Mol Biol 1991, 222:581-597.

The term "chimeric antibody" herein refers to an antibody that contains sequences derived from two different antibodies (e.g., US4,816,567), wherein the antibody is typically derived from different species. For example, a chimeric antibody comprises human and rodent antibody fragments, usually human constant regions and mouse variable regions. Methods for generating chimeric antibodies include conventional recombinant DNA and gene transfection techniques known to those skilled in the art (e.g., Morrison SL et al., Proc Natl Acad Sci USA 1984, 81:6851-6855; US5202238 and US5204244).

The term "humanized antibody or antigen-binding fragment thereof" herein refers to an antibody or antigen-binding fragment thereof comprising CDRs derived from non-human animals, FR regions derived from human, and constant regions derived from human. A humanized antibody may optionally further comprise at least a portion of the constant region of a human immunoglobulin. A humanized antibody or antigen-binding fragment thereof has reduced immunogenicity and thus can be used as a therapeutic agent for humans. In some embodiments, the non-human animal is a mammal such as a mouse, rat, rabbit, goat, sheep, guinea pig, or hamster. In some embodiments, the humanized antibody or antigen-binding fragment thereof consists essentially entirely of human sequences, except for the CDR sequences which are non-human. In some embodiments, humanized antibodies can be further modified, improved and optimized by substituting residues in the FRs of human immunoglobulins with corresponding residues from non-human species antibodies to enhance their specificity, affinity, and/or activity. In some embodiments, the human-derived FR may include the same amino acid sequence as the human antibody from which it is derived, or it may include some amino acid mutations, e.g., no more than 5, 4, 3, 2 or 1 amino acid modification. In some embodiments, the amino acid modification may be present only in the heavy chain FR, only in the light chain FR, or in both chains.

The term "corresponding human germline sequence" herein refers to an antibody variable region amino acid sequence or subsequence, which has the highest amino acid sequence identity to the amino acid sequence of the reference human germline immunoglobulin variable region, compared to the amino acid sequences of all other known human germline immunoglobulin variable region. The corresponding human germline sequence may be just a framework region, a complementarity-determining region, a framework region and a complementarity-determining region, a variable region, or other combinations containing a variable region sequence or subsequence. Sequence identity can be determined by aligning two sequences using the methods described herein, e.g., using BLAST, ALIGN, or another alignment algorithm known in the art. The corresponding human germline amino acid sequence may have at least about 90%, 91%, 92%, 93%, 94%, 95%, 96%, 97%, 98%, 99% or 100% identity to the amino acid sequence of the reference human germline immunoglobulin variable region.

The anti-HER2 antibody, anti-HER2 biparatopic antibody or the antibody component of ADC of the present invention can be selected from any one or more of the following forms, including a chimeric, non-human, humanized, or fully human form, provided that the form is capable of binding specifically to HER2.

As used herein, the term "epitope" refers to a protein determinant, which is the portion of an antigen that can be recognized and bound specifically by an antibody. An epitope usually consists of chemically active surface groupings of moleclules such as amino acids or sugar side chains and generally has specific three-dimensional structural characteristics as well as specific charge characteristics. The part of an antibody or antigen-binding fragment thereof that recognizes the epitope is called a paratope.

Competitive binding and epitope binning can be employed to determine whether different antibodies bind to identical or overlapping epitopes, for example, using the methods described in Harlow and Lane (eds.), Antibodies, A Laboratory Manual, Cold Spring Harbor Laboratory for evaluation. In some embodiments, competitive binding ELISA is used to evaluate whether an antibody or antigen-binding fragment (e.g., the antibody or antigen-binding fragment comprising CDRs and/or variable regions defined in Table 2 and Table 3) inhibits the binding activity of another antibody or antigen-binding fragment to the target antigen (e.g., HER2). When the binding activity is reduced by at least about 50% (e.g., 50%, 60%, 70%, 80%, 90%, 95%, 99% or more, or any percentage between the listed values), they are determined to be competitively binding, i.e., binding to the same epitope or overlapping epitopes. In some embodiments, competitive binding can be attributed to shared epitopes or similar epitopes (e.g., partially overlapping), or to steric hindrance caused by antibodies or antigen-binding fragments binding at adjacent epitopes (see Morris (ed.), Methods in Molecular Biology 1998, Vol. 66, pp. 55-66). In some embodiments, competitive binding can be used to group antibodies or antigen-binding fragments that bind to shared/similar epitopes. For example, antibodies or antigen-binding fragments that exhibit competitive binding can be "binned" into a group of antibodies or antigen-binding fragments targeting overlapping or adjacent epitopes, and non-competing antibodies or antigen-binding fragments are classified into another group targeting non-overlapping or non-adjacent epitopes.

The term "affinity" or "binding affinity" refers to the intrinsic binding capacity of the interaction between a molecule (e.g., a receptor or antigen) and its partner (e.g., a ligand or antibody), i.e., a total intensity of all noncovalent interactions. Unless otherwise stated, "binding affinity" as used herein is the intrinsic binding affinity which reflects a 1:1 interaction between the members of a binding pair (e.g., receptor and ligand, or antigen and antibody). The affinity of a molecule X for its binding partner Y can usually be expressed as the dissociation equilibrium constant (K_{D}), which is a ratio of the dissociation rate constant (k_{dis} or k_{off}) and the association rate constant (kₐ or kₒₙ). Affinity can be measured by conventional methods known in the art, including those used in the present invention.

As used herein, "internalization" or "endocytosis" refers to the process where an antibody or antigen-binding fragment thereof, bispecific antibody, or ADC, upon binding to a target antigen on cell surface, can enter the endosomes of the cell through internalization (i.e., endocytosis) across the cellular lipid bilayer membrane, preferably being the antibody or antigen-binding fragment, or the bispecific antibody that can traffic to the lysosomes within the cell. In some embodiments, the anti-HER2 biparatopic antibody or ADC described in the present invention, after binding to HER2 on the tumor cell membrane, can be internalized into the cell and downregulate the HER2 expression on tumor cell surface.

As used herein, the term "antibody variant" or "antibody mutant" refers to an antibody polypeptide sequence containing at least one amino acid mutation in the variable region of the original antibody. A variant can be substantially homologous or substantially identical to the unmodified antibody. In some embodiments, amino acid mutation(s) is/are introduced in one, two, three, four, five and/or six CDRs of the anti-HER2 antibody, anti-HER2 biparatopic antibody or the antibody component of ADC of the present invention to improve and optimize the performance of the said antibody or antigen-binding fragment, including but not limited to increasing the degree of humanization, enhancing binding affinity or binding activity to HER2, improving internalization efficiency, increasing production yield, and/or improving stability (e.g., reducing or eliminating the risk of aspartate isomerization and/or asparagine deamidation). In some embodiments, the anti-HER2 antibody, anti-HER2 biparatopic antibody, or the antibody component of the ADC of the present invention contains amino acid mutations in one, two, three, and/or four FRs to improve and optimize the performance of the said antibody or antigen-binding fragment, including but not limited to increasing the degree of humanization of the antibody or antigen-binding fragment, increasing binding affinity or binding activity to HER2, increasing production yield, and/or improving stability (e.g., reducing or eliminating the risk of aspartate isomerization and/or asparagine deamidation). In some embodiments, one or more amino acid mutations are introduced in both CDRs and FRs of the anti-HER2 antibody, anti-HER2 biparatopic antibody, or the antibody component of ADC of the present invention to increase the degree of humanization of the antibody or antigen-binding fragment, to enhance binding affinity or binding activity to HER2, to improve internalization efficiency, to increase production yield, and/or to improve stability (e.g., to reduce or eliminate the risk of aspartate isomerization and/or asparagine deamidation). In some embodiments, the amino acid mutations include amino acid substitution, deletion, insertion, or any combination thereof.

Among them, the amino acid substitutions include conservative amino acid substitutions and non-conservative amino acid substitutions, wherein conservative amino acid substitutions pertain to replacing with another amino acid from the same class (such as similar biochemical properties or function), and non-conservative substitutions pertain to replacing an amino acid in a class with another one from a different class (with dissimilar chemical properties or functions). A person skilled in the art can make conservative or non-conservative amino acid substitutions based on similarity in polarity, charge, solubility, hydrophobicity, hydrophilicity, and/or amphipathic properties of the residues involved. For example, (i) non-polar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); (ii) polar neutral amino acids include glycine (Gly, G), serine (Ser, S), threonine (Thr, T), cysteine (Cys, C), tyrosine (Tyr, Y), asparagine (Asn, N) and glutamine (Gln, Q); (iii) positively charged (basic) amino acids include arginine (Arg, R), lysine (Lys, K) and histidine (His, H); (iv) negatively charged (acidic) amino acids include aspartic acid (Asp, D) and glutamic acid (Glu, E). Generally, conservative amino acid substitutions do not substantially alter the functional properties of a protein, whereas non-conservative amino acid substitutions may lead to significant changes in properties or functions of a protein. Although the sites or regions for introducing amino acid mutations can be predetermined, the potential changes in protein properties or functions caused by non-conservative substitutions are unpredictable.

The term "identical" or "identity" or "percent identity" or "percent sequence identity" herein can be used interchangeably in two or more polypeptide sequences, and refers that after amino acid sequences are aligned, and if necessary, gaps are introduced to maximize the number of identical amino acids, the percentage of amino acid residues of the candidate sequence that are identical to the reference sequence in the amino acid residues of the candidate sequence. Conservative substitutions of such amino acid residues may or may not be considered as the identical residues. Percent sequence identity for amino acid sequences can be determined by aligning the sequences using the tools disclosed in the art, such as BLASTp, ClustalW2 (see, e.g., Higgins et al., Methods in Enzymology 1996, 266:383-402; Larkin et al., Bioinformatics 2007, 23:2947-2948) and ALIGN or Megalign (DNASTAR) software. Those skilled in the art can use the default parameters of the tools or appropriately adjust the parameters according to the alignment requirement, for example by selecting a suitable algorithm for sequence alignment.

The term "isolated" herein when referring to a protein means that the protein contains substantially no other cellular components bound to it in its natural state, preferably being in a homogeneous state, and for example, an isolated protein may be removed from its natural environment. The isolated protein can be lyophilized or in an aqueous solution. Generally, its purity and homogeneity can be determined by using analytical chemistry techniques such as polyacrylamide gel electrophoresis or high-performance liquid chromatography. As the main ingredient in the formulation, protein is basically obtained by purification. The term "purified" means that the protein produces substantially one band in an electrophoresis gel. In particular, this means that the protein is at least 85% purity, more preferably at least 95% purity, and most preferably at least 99% purity. For the present invention, in some embodiments, recombinant proteins expressed in host cells are considered to be isolated, and this applies to natural or recombinant proteins separated, fractionated, or partially or substantially purified by any technique well-known to those skilled in the art. In some embodiments, an "isolated antibody" refers to an antibody that is substantially free of other antibodies with different antigen specificities (e.g., an isolated antibody that specifically binds HER2 substantially does not contain other antibodies that specifically bind antigens other than HER2). However, the isolated antibody that specifically binds HER2 may be cross-reactive with other antigens, such as HER2 proteins from other species, e.g., monkey HER2. Furthermore, the isolated antibody may be substantially free of other cellular material and/or chemicals. In some embodiments, a recombinant polynucleotide encoding a polypeptide or protein of the present invention (e.g., anti-HER2 antibody, anti-HER2 biparatopic antibody) contained in a vector is considered to be isolated. Other examples of isolated polynucleotide include recombinant polynucleotide contained in heterologous host cells or (partially or substantially) purified polynucleotide in solution.

The term "engineering" herein is taken to include any manipulation of a polypeptide or protein backbone or post-translational modification of a naturally occurring or recombinant protein or polypeptide. Engineering includes introducing mutation(s) into the amino acid sequence, modification of the glycosylation pattern or the amino acid side chain, and a combination of these methods.

As used herein, the term "polypeptide" refers to a polymer of amino acids and their equivalents, rather than to a specific length of a product; thus, "peptide" and "protein" are included within the definition of polypeptide. Also included within the definition of polypeptide is an "antibody" as defined herein.

Dual Variable Domain Immunoglobulin (DVD-Ig) is a tetravalent molecule containing two distinct antigen-binding domains, which is constructed by fusing one antibody's heavy chain variable region (VH) and light chain variable region (VL) domains to the N-terminals of the heavy chain and light chain of another IgG antibody, respectively. Therefore, each half of a DVD-Ig contains one heavy chain polypeptide and one light chain polypeptide, which forms two independent antigen-binding domains after dimerization, one of them being an Fv fragment, and the other being a Fab fragment or IgG domain. DVD-Ig can be bispecific, meaning it can bind to two different epitopes on the same antigen or two different antigens.

The term "expression vector" or "vector" herein refers to a vehicle into which a polynucleotide or nucleic acid encoding a protein can be operably inserted and the protein can be expressed. A vector can be used to transform, transduce or transfect a host cell, allowing the genetic material elements it carries to be expressed in the host cell.

The term "host cell" herein is a cell to be introduced with an exogenous polynucleotide or nucleic acid and/or vector. A host cell includes a "transformant" and "transformed cell", which includes the primary transformed cell and progeny derived therefrom, regardless of the number of passages. The progeny may not be identical in nucleic acid content to the parent cell and may contain mutation. Included in the present invention are mutant progeny screened or selected for the same function or biological activity from the originally transformed cell.

The terms "subject", "patient" or "individual" herein are used interchangeably and include but are not limited to, a mammal, including, for example, human, a non-human primate (e.g., monkey), mouse, pig, cow, goat, rabbit, rat, guinea pig, hamster, horse, monkey, sheep, or other non-human mammal; a non-mammal, including, for example, non-mammalian vertebrates such as birds (e.g., chicken or duck) or fish; and non-mammalian invertebrates. In some embodiments, the subject and pharmaceutical composition involved in the use or method of the present invention are used to treat (prophylactically and/or therapeutically) non-human animals.

As used herein, "treating, treatment or to treat" for a disease or symptom means alleviating a disease or condition, reducing the rate at which a disease or condition arises or develops, and reducing the risk of developing a disease or condition, or delaying the development of symptoms associated with a disease or condition, reducing or terminating symptoms associated with a disease or condition, producing a complete or partial reversal of a disease or condition, curing a disease or condition, or any combination of the above.

The term "therapeutically effective amount" or "effective dose" refers to a dose or concentration effective to achieve prevention or amelioration of symptoms associated with a disease or disorder and/or lessening the severity of a disease or disorder, at a dosage and for a desired period of time. A therapeutically effective amount of a preparation, antibody or antigen-binding fragment, ADC, or composition thereof of the present invention may vary depending on factors such as the disease state, the age, sex and body weight of the individual, and the ability of the antibody or antibody portion or ADC to elicit a desired response in the individual. A therapeutically effective amount may also be considered as any toxic or detrimental effect of the preparation, the antibody or antigen-binding fragment, ADC, or composition thereof lower than the therapeutically beneficial effect. The term "effective amount" refers to an amount of an active ingredient or agent sufficient to provide a clinical benefit to a subject, including but not limited to, amelioration, alleviation, or relief of a disease, disorder, or associated symptoms thereof, delaying or arresting disease progression.

As used hererin, "low level HER2", "low expression of HER2", "low HER2 expression", "HER2 low expression", or "HER2 low-level expression" refers to cancer cells/tumors, subjects, or patients that have an IHC score of 1+ (faint/barely discernible membrane staining detected in greater than 10% of tumor cells, with cells stained in only a portion of the membrane) for ErbB2/HER2 protein staining intensity as determined by an immunohistochemistry (IHC) assay (e.g., HercepTest^{®}) on paraffin-embedded tissue sections from a tumor biopsy, or a negative score for the degree of HER2 gene amplification in tumor cells as further determined by the fluorescence in situ hybridization (FISH) (e.g., InformTM [Ventana], or PathVysionTM [Vysis]) when having an IHC score of 2⁺ (weak to moderate global membrane staining observed in greater than 10% of tumor cells), i.e., IHC 2+/FISH⁻. The HER2 expression level of most HER2-low-expression tumors is significantly higher than that of normal tissues. The HER2 expression level of normal tissues can be determined by any method available to those skilled in the art.

"HER2-negative," "negative HER2 expression," or "expressing limited levels of HER2" refers to cancer cells/tumors, subjects, or patients with HER2-low expression as defined above or with an IHC score of 0 (no staining observed or membrane staining present in less than 10% of tumor cells).

"HER2-positive" or "HER2 expression-positive" or "HER2 overexpression" refers to cancer cells/tumors, subjects, or patients with an IHC score of 3⁺ (strong, intact membranous staining observed in more than 10% of tumor cells). HER2-positive also includes cancer cells/tumors, subjects, or patients that have an IHC score of 2⁺ and a positive score in FISH assay (e.g., Subtractive Probe Technology Chromogenic In Situ Hybridization [SPoT-Light HER2 CISH] test, Inform Dual ISH test), i.e., IHC 2+/FISH⁺.

As used herein, the "bystander effect" or "bystander killing effect" of ADC is mediated by free membrane-permeable small-molecule toxins (e.g., anticancer agents), which are released by target molecule-positive (e.g., HER2-positive) cancer cells and passively diffuse into the tumor microenvironment, thereby killing neighboring cells, including neighboring cancer cells that do not express or express low-levels of the target molecule (e.g., HER2-negative) and are thus insensitive to ADC.

As used herein, the term "pharmaceutically acceptable" or "medicinally acceptable" refers to the indicated carrier, vehicle, diluents, adjuvant and/or salt, generally chemically and/or physically compatible with the other ingredients in the formulation and physiologically compatible with the subject.

The term "about" when used in conjunction with a numerical value is meant to encompass a numerical value within a range having a lower limit of 5% less than the specified numerical value and an upper limit of 5% greater than the specified numerical value, or in one embodiment a lower limit of 10% less than the specified numerical value and an upper limit of 10% greater than the specified numerical value, or in another embodiment a lower limit of 15% less than the specified numerical value and an upper limit of 15% greater than the specified numerical value, or in another embodiment a lower limit of 20% less than the specified numerical value and an upper limit of 20% greater than the specified numerical value.

The term "and/or" should be understood to mean any one of the alternatives or a combination of any two or more of the alternatives.

As used herein, the term "comprising" or "including" or "containing" or "having" or "involving" can be used interchangeably to mean the inclusion of an element, integer or step, but not the exclusion of any other elements, integers or steps. Herein, when the term "comprising" or "including" or "containing" or "having" or "involving" is used, unless otherwise indicated, situations consisting of the said elements, integers or steps are also encompassed.

As used herein, the term "optionally" indicates that the object it modifies is present or absent, for example, "the kit optionally comprises at least one additional tumor therapeutic agent" means that the kit may or may not comprise at least one additional tumor therapeutic agent.

As mentioned herein, "some embodiments", "one embodiment", "one specific embodiment", or "specific embodiments", or a combination thereof indicates that the specific features, structures or characteristics described in relation to the embodiment are included in at least one embodiment of the present invention. Thus, appearances of the foregoing terms in various places throughout this specification do not necessarily refer to the same embodiment. Furthermore, the specific features, structures or characteristics may be combined in any suitable manner in one or more embodiments.

Unless the context clearly dictates otherwise, singular terms encompass plural referents and vice versa.

All patents, patent applications, and other publications are expressly incorporated herein by reference for the purposes of description and disclosure. These publications are provided solely because their disclosure predates the filing date of this application. All statements regarding the dates of these documents or representations of the contents of these documents are based on information available to the applicant and do not constitute any admission as to the correctness of the dates of these documents or the contents of these documents.

Various aspects of the present invention will be described in more details in the following sections.

### 1. Anti-HER2 Antibody or Antigen-Binding Fragment thereof

In one aspect, the present invention provides an isolated anti-HER2 antibody or antigen-binding fragment thereof, wherein the anti-HER2 antibody or antigen-binding fragment thereof can specifically bind to the extracellular domain of HER2.

The anti-HER2 antibody or antigen-binding fragment thereof of the present invention can specifically bind to D1, D3 and/or D4 of the HER2 extracellular region, preferably D1 or D3 of the HER2 extracellular region. The anti-HER2 biparatopic antibody of the present invention can be constructed using the antigen-binding domains of two anti-HER2 monospecific antibodies, wherein the two monospecific antibodies can each bind to different epitopes on the HER2 extracellular region, and the two monospecific antibodies exhibit no competitive inhibition in an antigen competitive binding assay (i.e., the antigen-binding epitopes do not overlap).

In some embodiments, the antigen-binding domains of two anti-HER2 monospecific antibodies can be used to construct the anti-HER2 biparatopic antibody of the present invention, wherein the two monospecific antibodies are capable of simultaneously binding to different epitopes on the extracellular domain of HER2. Furthermore, the two monospecific antibodies can simultaneously bind to any two epitopes on D1, D3, and D4 of the HER2 extracellular region. Preferably, they can simultaneously bind to D1 and D3 of the HER2 extracellular region. For example, the antigen-binding domain of the first anti-HER2 antibody binds to D3 of the HER2 extracellular region, and the antigen-binding domain of the second anti-HER2 antibody binds to D1 of the HER2 extracellular region.

The anti-HER2 antibody or antigen-binding fragment thereof has strong binding activity to HER2-expressing tumor cells, including HER2-overexpressing tumor cells (e.g., breast cancer cell line SKBR-3, breast ductal carcinoma cell line BT474, gastric cancer cell line NCI-N87, and ovarian cancer cell line SKOV-3), tumor cells expressing intermediate levels of HER2 (e.g., breast cancer cell line JIMT-1), tumor cells expressing low levels of HER2 (e.g., human bladder cancer cell line RT-112, breast cancer cell lines ZR-75-1 and T47D), and/or tumor cells expressing a limited level of HER2 (e.g., breast cancer cell line MCF-7). The binding affinity (K_{D}) value of the anti-HER2 antibody or antigen-binding fragment thereof to HER2 is <5×10⁻⁸ M, preferably <1×10⁻⁸ M, <5×10⁻⁹ M, or <1×10⁻⁹ M, and more preferably <1×10⁻⁹ M. The anti-HER2 antibody or antigen-binding fragment thereof does not cross-react with other members of the ErbB/HER family (including EGFR, HER3 and HER4).

The anti-HER2 antibody or antigen-binding fragment thereof does not interfere with the regulation of HER2 expressed on the cell surface and its downstream signal transduction pathways. In some embodiments, the anti-HER2 antibody or antigen-binding fragment thereof does not induce, block, or inhibit the phosphorylation and/or dephosphorylation of tyrosine residues in the HER2 intracellular domain, wherein the tyrosine residue phosphorylation sites in the HER2 intracellular domain include, but are not limited to, Y877, Y1221/1222, and Y1248. In some embodiments, the anti-HER2 antibody or antigen-binding fragment thereof does not induce, block, or inhibit ligand-dependent or ligand-independent HER2 dimerization (including HER2:HER4 dimerization and/or HER2:HER3 dimerization) and its downstream signaling pathways, wherein the ligands include NRG-1 or Heregulins.

The anti-HER2 antibody of the present invention may also optionally include F(ab')2, Fab, Fab', Fv, scFv, scFv-Fc, single-domain antibody (sdAb), or be of IgG type. The antibody of the present invention can be a murine antibody, chimeric antibody, humanized antibody, or fully human antibody, and can be a monoclonal antibody, polyclonal antibody, a monospecific antibody, a bispecific antibody, a multispecific antibody, or an antibody fragment, provided that the antibody specifically recognize epitopes D1, D3, and/or D4 (preferably D1 and/or D3) of the HER2 extracellular region and does not affect HER2 expressed on the surface of tumor cells or its signaling pathways. In some embodiments, the anti-HER2 antibody is selected from a mouse anti-human HER2 antibody and a humanized and optimized antibody thereof. In some embodiments, the anti-HER2 antibody includes scFv, scFv-Fc, Fab fragment and/or IgG-type. For example, an antibody specifically binding to D3 of the HER2 extracellular domain can be scFv or scFv-Fc format, and an antibody specifically binding to D1 of the HER2 extracellular domain can be Fab or IgG format, wherein Fab and scFv formats can be interconverted as needed, and the conversion methods are known in the art (see, e.g., the method described in Zhou et al., Mol Cancer Ther 2012, 11:1167-1476).

In another aspect, the present invention provides an anti-HER2 antibody or antigen-binding fragment thereof comprising the CDRs and/or variable regions of the antibody shown in Table 2, which recognizes D3 of the HER2 extracellular domain and does not induce, block, or inhibit the phosphorylation and/or dephosphorylation of tyrosine residues in the HER2 intracellular domain, and/or does not induce, block, or inhibit NRG-1-dependent or NRG-1-independent HER2 dimerization and its downstream signaling pathways. The present invention also provides an anti-HER2 antibody or antigen-binding fragment thereof capable of recognizing D1 of the HER2 extracellular domain, which does not induce, block, or inhibit the phosphorylation and/or dephosphorylation of tyrosine residues in the HER2 intracellular domain, and/or does not induce, block, or inhibit NRG-1-dependent or NRG-1-independent HER2 dimerization and its downstream signaling pathways. Preferably, the antibody comprises the CDRs and/or variable regions of the antibody or antigen-binding fragment thereof shown in Table 3. The anti-HER2 antibody or antigen-binding fragment thereof of the present invention also comprises CDRs, variable regions, or light and heavy chains of the anti-HER2 antibody or antigen-binding fragment thereof that recognizes D4 of the HER2 extracellular domain.

The heavy chain variable region CDRs and light chain variable region CDRs of the anti-HER2 antibody of the present invention are defined by the Kabat numbering system. However, as known in the art, CDR regions can also be defined based on other numbering systems/methods for heavy/light chain variable region sequences, such as Chothia and IMGT, AbM, or Contact. The CDR regions defined by other numbering systems/methods, as well as those defined by Kabat employed in the present invention, are all within the protection scope of the present invention.

**Talbe 2. Amino acid sequence ID numbers of CDRs and the heavy and light chain variable regions of mAb2117 and humanized antibody thereof**

| Description | Antibody ID | SEQ ID No. | | | | | | | |
|---|---|---|---|---|---|---|---|---|---|
| | | HCDR1 | HCDR2 | HCDR3 | VH | LCDR1 | LCDR2 | LCDR3 | VL |
| Murine antibody, chimeric antibody | mAb2117 | 11 | 14 | 32 | 1 | 38 | 46 | 49 | 2 |
| Humanized Antibody | hu2117-H1K1 | 11 | 14 | 32 | 52 | 38 | 46 | 49 | 81 |
| | hu2117-HK-Mu01 | 11 | 14 | 32 | 52 | 38 | 46 | 49 | 82 |
| | hu2117-HK-Mu02 | 11 | 14 | 32 | 52 | 38 | 46 | 49 | 83 |
| | hu2117-HK-Mu03 | 11 | 14 | 32 | 52 | 38 | 46 | 49 | 84 |
| | hu2117-HK-Mu04 | 11 | 14 | 32 | 52 | 38 | 46 | 49 | 85 |
| | hu2117-HK-Mu05 | 11 | 14 | 32 | 53 | 38 | 46 | 49 | 81 |
| | hu2117-HK-Mu06 | 11 | 14 | 32 | 54 | 38 | 46 | 49 | 81 |
| | hu2117-HK-Mu08 | 11 | 15 | 32 | 55 | 38 | 46 | 49 | 81 |
| | hu2117-HK-Mu09 | 11 | 16 | 32 | 56 | 38 | 46 | 49 | 81 |
| | hu2117-HK-Mu10 | 11 | 17 | 32 | 57 | 38 | 46 | 49 | 81 |
| | hu2117-HK-Mu11 | 11 | 14 | 32 | 52 | 38 | 47 | 49 | 86 |
| | hu2117-HK-Mu12 | 11 | 14 | 32 | 52 | 38 | 48 | 49 | 87 |
| | hu2117-HK-Mu13 | 11 | 14 | 32 | 52 | 38 | 46 | 49 | 88 |
| | hu2117-HK-Mu14 | 11 | 18 | 32 | 58 | 38 | 46 | 49 | 81 |
| | hu2117-HK-Mu23 | 11 | 14 | 32 | 52 | 39 | 46 | 49 | 89 |
| | hu2117-HK-Mu24 | 11 | 14 | 32 | 52 | 40 | 46 | 49 | 90 |
| | hu2117-HK-Mu25 | 11 | 14 | 32 | 52 | 41 | 46 | 49 | 91 |
| | hu2117-HK-Mu27 | 11 | 14 | 32 | 52 | 42 | 46 | 49 | 92 |
| | hu2117-HK-Mu28 | 11 | 14 | 32 | 52 | 43 | 46 | 49 | 93 |
| | hu2117-HK-Mu29 | 11 | 14 | 32 | 52 | 44 | 46 | 49 | 94 |
| | hu2117-HK-Mu32 | 11 | 14 | 32 | 52 | 45 | 46 | 49 | 95 |
| | hu2117-HK-Mu34 | 11 | 14 | 32 | 52 | 38 | 46 | 50 | 96 |
| | hu2117-HK-Mu37 | 11 | 14 | 32 | 52 | 38 | 46 | 51 | 97 |
| | hu2117-HK-Mu38 | 12 | 14 | 32 | 59 | 38 | 46 | 49 | 81 |
| | hu2117-HK-Mu39 | 12 | 14 | 32 | 60 | 38 | 46 | 49 | 81 |
| | hu2117-HK-Mu40 | 13 | 14 | 32 | 61 | 38 | 46 | 49 | 81 |
| | hu2117-HK-Mu41 | 11 | 19 | 32 | 62 | 38 | 46 | 49 | 81 |
| | hu2117-HK-Mu42 | 11 | 20 | 32 | 63 | 38 | 46 | 49 | 81 |
| | hu2117-HK-Mu45 | 11 | 14 | 33 | 64 | 38 | 46 | 49 | 81 |
| | hu2117-HK-Mu46 | 11 | 14 | 34 | 65 | 38 | 46 | 49 | 81 |
| | hu2117-HK-Mu48 | 11 | 14 | 35 | 66 | 38 | 46 | 49 | 81 |
| | hu2117-HK-Mu49 | 11 | 14 | 36 | 67 | 38 | 46 | 49 | 81 |
| | hu2117-HK-Mu50 | 11 | 14 | 37 | 68 | 38 | 46 | 49 | 81 |
| | hu2117-HK201 | 11 | 21 | 32 | 69 | 42 | 46 | 49 | 92 |
| | hu2117-HK202 | 11 | 14 | 37 | 68 | 42 | 46 | 49 | 92 |
| | hu2117-HK203 | 11 | 21 | 37 | 70 | 42 | 46 | 49 | 92 |
| | hu2117-HK-301 | 11 | 22 | 37 | 71 | 42 | 46 | 49 | 92 |
| | hu2117-HK-303 | 11 | 23 | 37 | 72 | 42 | 46 | 49 | 92 |
| | hu2117-HK-304 | 11 | 24 | 37 | 73 | 42 | 46 | 49 | 92 |
| | hu2117-HK-305 | 11 | 25 | 37 | 74 | 42 | 46 | 49 | 92 |
| | hu2117-HK-306 | 11 | 26 | 37 | 75 | 42 | 46 | 49 | 92 |
| | hu2117-HK-309 | 11 | 27 | 37 | 76 | 42 | 46 | 49 | 92 |
| | hu2117-HK-310 | 11 | 28 | 37 | 77 | 42 | 46 | 49 | 92 |
| | hu2117-HK304-04 | 11 | 29 | 37 | 78 | 42 | 46 | 49 | 92 |
| | hu2117-HK304-05 | 11 | 30 | 37 | 79 | 42 | 46 | 49 | 92 |
| | hu2117-HK304-06 | 11 | 31 | 37 | 80 | 42 | 46 | 49 | 92 |

**Talbe 3. Amino acid sequence ID numbers of CDRs and the heavy and light chain variable regions of mAb2126 and humanized antibody thereof**

| Description | Antibody ID | SEQ ID No. | | | | | | | |
|---|---|---|---|---|---|---|---|---|---|
| | | HCDR1 | HCDR2 | HCDR3 | VH | LCDR1 | LCDR2 | LCDR3 | VL |
| Murine antibody, chimeric antibody | mAb2126 | 98 | 100 | 110 | 3 | 114 | 116 | 139 | 4 |
| Humanized Antibody | Hu2126-H1K1 | 98 | 100 | 110 | 170 | 114 | 116 | 139 | 171 |
| | hu2126-H2K1 | 98 | 100 | 110 | 143 | 114 | 116 | 139 | 171 |
| | 2126-Hu-L02 | 98 | 100 | 110 | 143 | 115 | 116 | 139 | 172 |
| | 2126-Hu-L05 | 98 | 100 | 110 | 143 | 114 | 116 | 139 | 173 |
| | 2126-Hu-L06 | 98 | 100 | 110 | 143 | 114 | 117 | 139 | 174 |
| | 2126-Hu-L07 | 98 | 100 | 110 | 143 | 114 | 118 | 139 | 175 |
| | 2126-Hu-L08 | 98 | 100 | 110 | 143 | 114 | 119 | 139 | 176 |
| | 2126-Hu-L09 | 98 | 100 | 110 | 143 | 114 | 120 | 139 | 177 |
| | 2126-Hu-L10 | 98 | 100 | 110 | 143 | 114 | 121 | 139 | 178 |
| | 2126-Hu-Ll1 | 98 | 100 | 110 | 143 | 114 | 122 | 139 | 179 |
| | 2126-Hu-L12 | 98 | 100 | 110 | 143 | 114 | 123 | 139 | 180 |
| | 2126-Hu-L13 | 98 | 100 | 110 | 143 | 114 | 124 | 139 | 181 |
| | 2126-Hu-L14 | 98 | 100 | 110 | 143 | 114 | 125 | 139 | 182 |
| | 2126-Hu-L15 | 98 | 100 | 110 | 143 | 114 | 126 | 139 | 183 |
| | 2126-Hu-L16 | 98 | 100 | 110 | 143 | 114 | 127 | 139 | 184 |
| | 2126-Hu-L17 | 98 | 100 | 110 | 143 | 114 | 128 | 139 | 185 |
| | 2126-Hu-L18 | 98 | 100 | 110 | 143 | 114 | 116 | 140 | 186 |
| | 2126-Hu-L19 | 98 | 100 | 110 | 143 | 114 | 116 | 141 | 187 |
| | 2126-Hu-L21 | 98 | 100 | 110 | 143 | 114 | 116 | 142 | 188 |
| | 2126-Hu-H22 | 98 | 100 | 110 | 144 | 114 | 116 | 139 | 171 |
| | 2126-Hu-H23 | 98 | 100 | 110 | 145 | 114 | 116 | 139 | 171 |
| | 2126-Hu-H24 | 99 | 100 | 110 | 146 | 114 | 116 | 139 | 171 |
| | 2126-Hu-H25 | 98 | 101 | 110 | 147 | 114 | 116 | 139 | 171 |
| | 2126-Hu-H27 | 98 | 102 | 110 | 148 | 114 | 116 | 139 | 171 |
| | 2126-Hu-H28 | 98 | 103 | 110 | 149 | 114 | 116 | 139 | 171 |
| | 2126-Hu-H29 | 98 | 104 | 110 | 150 | 114 | 116 | 139 | 171 |
| | 2126-Hu-H31 | 98 | 105 | 110 | 151 | 114 | 116 | 139 | 171 |
| | 2126-Hu-H32 | 98 | 106 | 110 | 152 | 114 | 116 | 139 | 171 |
| | 2126-Hu-H33 | 98 | 107 | 110 | 153 | 114 | 116 | 139 | 171 |
| | 2126-Hu-H34 | 98 | 108 | 110 | 154 | 114 | 116 | 139 | 171 |
| | 2126-Hu-H35 | 98 | 109 | 110 | 155 | 114 | 116 | 139 | 171 |
| | 2126-Hu-H36 | 98 | 100 | 110 | 156 | 114 | 116 | 139 | 171 |
| | 2126-Hu-H37 | 98 | 100 | 110 | 157 | 114 | 116 | 139 | 171 |
| | 2126-Hu-H38 | 98 | 100 | 110 | 158 | 114 | 116 | 139 | 171 |
| | 2126-Hu-H39 | 98 | 100 | 110 | 159 | 114 | 116 | 139 | 171 |
| | 2126-Hu-H40 | 98 | 100 | 110 | 160 | 114 | 116 | 139 | 171 |
| | 2126-Hu-H42 | 98 | 100 | 110 | 161 | 114 | 116 | 139 | 171 |
| | 2126-Hu-H43 | 98 | 100 | 110 | 162 | 114 | 116 | 139 | 171 |
| | 2126-Hu-H44 | 98 | 100 | 110 | 163 | 114 | 116 | 139 | 171 |
| | 2126-Hu-H45 | 98 | 100 | 110 | 164 | 114 | 116 | 139 | 171 |
| | 2126-Hu-H46 | 98 | 100 | 110 | 165 | 114 | 116 | 139 | 171 |
| | 2126-Hu-H50 | 98 | 100 | 111 | 166 | 114 | 116 | 139 | 171 |
| | 2126-Hu-H54 | 98 | 100 | 112 | 167 | 114 | 116 | 139 | 171 |
| | 2126-Hu-H59 | 98 | 100 | 113 | 168 | 114 | 116 | 139 | 171 |
| | 7172b-Mu10 | 98 | 102 | 110 | 184 | 115 | 129 | 142 | 189 |
| | 7172b-Mu11 | 98 | 102 | 110 | 169 | 115 | 130 | 142 | 190 |
| | 7172b-Mu12 | 98 | 102 | 110 | 169 | 115 | 131 | 142 | 191 |
| | 7172b-Mu13 | 98 | 102 | 110 | 169 | 115 | 132 | 142 | 192 |
| | 7172b-Mu14 | 98 | 102 | 110 | 169 | 115 | 133 | 142 | 193 |
| | 7172b-Mu15 | 98 | 102 | 110 | 169 | 115 | 134 | 142 | 194 |
| | 7172b-Mu16 | 98 | 102 | 110 | 169 | 115 | 135 | 142 | 195 |
| | 7172b-Mu17 | 98 | 102 | 110 | 169 | 115 | 136 | 142 | 196 |
| | 7172b-Mu18 | 98 | 102 | 110 | 169 | 115 | 137 | 142 | 197 |
| | 7172b-Mu19 | 98 | 102 | 110 | 169 | 115 | 138 | 142 | 198 |

In some embodiments, the anti-HER2 antibody or antigen-binding fragment thereof of the present invention can specifically bind to D3 of the HER2 extracellular domain, comprising VH with the amino acid sequence as set forth in SEQ ID NO: 1 and/or VL with the amino acid sequence as set forth in SEQ ID NO: 2.

In some embodiments, the anti-HER2 antibody or antigen-binding fragment thereof of the present invention is capable of specifically binding to D1 of the HER2 extracellular domain, comprising a VH with the amino acid sequence as set forth in SEQ ID NO: 3, 7, or 9 and/or a VL with the amino acid sequence as set forth in SEQ ID NO: 4, 8, or 10. In one embodiment, the anti-HER2 antibody or antigen-binding fragment thereof that specifically binds to D1 of the HER2 extracellular domain of the present invention comprises VH with the amino acid sequence as set forth in SEQ ID NO: 3 and/or VL with the amino acid sequence as set forth in SEQ ID NO: 4. In one embodiment, the anti-HER2 antibody or antigen-binding fragment thereof that specifically binds to D1 of the HER2 extracellular domain of the present invention comprises VH with the amino acid sequence as set forth in SEQ ID NO: 7 and/or VL with the amino acid sequence as set forth in SEQ ID NO: 8. In one embodiment, the anti-HER2 antibody or antigen-binding fragment thereof that specifically binds to D1 of the HER2 extracellular domain of the present invention comprises VH with the amino acid sequence as shown in SEQ ID NO: 9 and/or VL with the amino acid sequence as shown in SEQ ID NO: 10.

In some embodiments, the anti-HER2 antibody or antigen-binding fragment thereof of the present invention is capable of specifically binding to D4 of the HER2 extracellular domain, comprising VH with the amino acid sequence as set forth in SEQ ID NO: 5 and/or VL with the amino acid sequence as set forth in SEQ ID NO: 6.

In some embodiments, the anti-HER2 antibody or antigen-binding fragment thereof of the present invention that specifically binds to D3 of the HER2 extracellular domain comprises one or more CDRs of the VH with amino acid sequence as set forth in SEQ ID NO: 1 or has amino acid sequences as set forth in SEQ ID NOs: 11, 14, and 32, or variants thereof, wherein the variants include humanized antibodies or any other variants described herein.

In some embodiments, the anti-HER2 antibody or antigen-binding fragment thereof of the present invention that specifically binds to D3 of the HER2 extracellular domain further comprises one or more CDRs of the VL with amino acid sequence as set forth in SEQ ID NO: 2 or has amino acid sequences as set forth in SEQ ID NOs: 38, 46 and 49, or variants thereof, whererin the variants include humanized antibodies or any other variants described herein.

In some embodiments, the anti-HER2 antibody or antigen-binding fragment thereof comprises HCDR1 as set forth in SEQ ID NO: 11, HCDR2 as set forth in SEQ ID NO: 14, and HCDR3 as set forth in SEQ ID NO: 32, and LCDR1 as set forth in SEQ ID NO: 38, LCDR2 as set forth in SEQ ID NO: 46, and LCDR3 as set forth in SEQ ID NO: 49.

In some embodiments, the anti-HER2 antibody or antigen-binding fragment thereof of the present invention that specifically binds to D1 of the HER2 extracellular domain comprises one or more CDRs of the VH with amino acid sequence as set forth in SEQ ID NO: 3 or has amino acid sequences as set forth in SEQ ID NOs: 98, 100, and 110, or variants thereof, and/or one or more CDRs of the VH with amino acid sequence as set forth in SEQ ID NO: 7 or has amino acid sequences as set forth in SEQ ID NOs: 205, 206, and 207, or variants thereof, and/or one or more CDRs of the VH with amino acid sequence as set forth in SEQ ID NO: 9 or has amino acid sequences as set forth in SEQ ID NOs: 211, 212, and 213, or variants thereof, wherein the variants include humanized antibodies or any other variants described herein.

In some embodiments, the anti-HER2 antibody or antigen-binding fragment thereof of the present invention that specifically binds to D1 of the HER2 extracellular domain further comprises one or more CDRs of the VL with amino acid sequence as set forth in SEQ ID NO: 4 or has amino acid sequences as set forth in SEQ ID NOs: 114, 116 and 139, or variants thereof, and/or one or more CDRs of the VL with amino acid sequence as set forth in SEQ ID NO: 8 or has amino acid sequences as set forth in SEQ ID NOs: 208, 209 and 210, or variants thereof, and/or one or more CDRs of the VL with amino acid sequence as set forthe in SEQ ID NO: 10 or has amino acid sequences as set forth in SEQ ID NOs: 214, 215 and 216, or variants thereof, whererin the variants include humanized antibodies or any other variants described herein.

In some embodiments, the anti-HER2 antibody or antigen-binding fragment thereof of the present invention that specifically binds to D1 of the HER2 extracellular domain comprises HCDR1 as set forth in SEQ ID NO: 98, HCDR2 as set forth in SEQ ID NO: 100, HCDR3 as set forth in SEQ ID NO: 110, and LCDR1 as set forth in SEQ ID NO: 114, LCDR2 as set forth in SEQ ID NO: 116, LCDR3 as set forth in SEQ ID NO: 139; or, HCDR1 as set forth in SEQ ID NO: 205, HCDR2 as set forth in SEQ ID NO: 206, HCDR3 as set forth in SEQ ID NO: 207, and LCDR1 as set forth in SEQ ID NO: 208, LCDR2 as set forth in SEQ ID NO: 209, LCDR3 as set forth in SEQ ID NO: 210; or, HCDR1 as set forth in SEQ ID NO: 211, HCDR2 as set forth in SEQ ID NO: 212, HCDR3 as set forth in SEQ ID NO: 213, and LCDR1 as set forth in SEQ ID NO: 214, LCDR2 as set forth in SEQ ID NO: 215, LCDR3 as set forth in SEQ ID NO: 216.

In some embodiments, the anti-HER2 antibody or antigen-binding fragment thereof of the present invention that specifically binds to D4 of the HER2 extracellular domain comprises one or more CDRs of the VH with amino acid sequence as set forth in SEQ ID NO: 5 or has amino acid sequences as set forth in SEQ ID NOs: 199, 200, and 201, or variants thereof, wherein the variants include humanized antibodies or any other variants described herein.

In some embodiments, the anti-HER2 antibody or antigen-binding fragment thereof of the present invention that specifically binds to D4 of the HER2 extracellular domain comprises one or more CDRs of the VL with amino acid sequence as set forth in SEQ ID NO: 6 or has amino acid sequences as set forth in SEQ ID NOs: 202, 203, and 204, or variants thereof, wherein the variants include humanized antibodies or any other variants described herein.

In some embodiments, the anti-HER2 antibody or antigen-binding fragment thereof of the present invention that specifically binds to D4 of the HER2 extracellular domain comprises HCDR1 as set forth in SEQ ID NO: 199, HCDR2 as set forth in SEQ ID NO: 200, HCDR3 as set forth in SEQ ID NO: 201, and LCDR1 as set forth in SEQ ID NO: 202, LCDR2 as set forth in SEQ ID NO: 203, LCDR3 as set forth in SEQ ID NO: 204.

In another aspect, the VH and/or VL of the anti-HER2 antibody or antigen-binding fragment of the present invention can be used as starting materials for engineering to produce the antibodies described herein that are more suitable for administration to humans. The antibodies can be engineered by mutating one or more amino acid residues within one or both variable regions (i.e., VH and/or VL), e.g., by mutating one or more amino acid residues within one or more CDR regions and/or one or more framework regions.

In some embodiments, the variable regions of the anti-HER2 antibody of the present invention are engineered by CDR grafting. Antibodies interact with target antigens primarily through the amino acid residues of the six CDRs of the heavy and light chains. Therefore, amino acid sequences within the CDR regions among individual antibodies are more diverse than sequences outside the CDR regions such as FRs. Because the amino acid sequences of the CDR regions are responsible for most of the antibody-antigen interactions, recombinant antibodies can be expressed to mimic the properties of a particular naturally occurring antibody by constructing expression vectors comprising the CDR sequences from the particular naturally occurring antibody grafting onto the FR sequences of another antibody with different properties (see, e.g., Riechmann et al., Nature 1998, 332:323-327; Jones et al., Nature 1986, 321:522-525; Queen et al., Proc Natl Acad Sci USA 1989, 86:10029-10033; see also US5225539, US5530101, US5585089, US5693762, and US6180370).

The anti-HER2 antibody of the present invention may also comprise different framework region sequences. Such framework region sequences can be obtained from public DNA databases or published references relating to germline antibody gene sequences. For example, germline DNA sequences of human heavy and light chain variable region genes can be found in the "V Base" human germline sequence database (available at www.mrc-cpe.cam.ac.uk/vbase); as well as in Kabat et al., Sequences of Proteins of Immunological Interest, 5th ed. 1991, NIH Publication No. 91-3242; Tomlinson et al., J Mol Biol 1992, 227:776-798; and Cox et al., Eur J Immunol 1994, 24:827-836; the contents of each of which are expressly incorporated herein by reference. As another example, germline DNA sequences of human heavy and light chain variable region genes can be found in the IMGT database. For example, the following heavy chain germline sequences found in human immunoglobulins can be obtained by IMGT accession number: IGHV3-23 (DP47; VH26; V3-23) or IGHV7-4. As another example, the following light chain germline sequences found in human immunoglobulins can be obtained by IMGT accession number: IGKV1-39 or IGKV1-39*01.

Amino acid sequences of antibodies can be aligned based on translated protein sequences using one of the sequence similarity search methods, e.g., Gapped BLAST, known to those skilled in the art (Altschul et al., Nucleic Acids Res 1997, 25:3389-3402).

The preferred framework sequence used in the anti-HER2 antibody of the present invention is the acceptor framework region that is structurally similar (or highly homologous) to the framework sequence of the murine parental antibody as described in the present invention. In some embodiments, CDR1, CDR2, and CDR3 region sequences of either VH or VL can be grafted onto the acceptor framework region respectively, wherein the acceptor framework region has an identical or the highest homologous sequence with immunoglobulin genes in its corresponding germline. In a specific embodiment, the present invention selects and grafts the CDR regions of the VH as set forth in SEQ ID NO: 1 or 3 and the VL as set forth in SEQ ID NO: 2 or 4 onto human-derived IgG FR regions to generate a humanized antibody, wherein the humanized antibody not only maintains an antigen-binding activity similar to that of the parent antibody comprising the VH as set forth in SEQ ID NO: 1 and the VL as set forth in SEQ ID NO: 2, or the VH as set forth in SEQ ID NO: 3 and the VL as set forth in SEQ ID NO: 4, but also does not interfere with the HER2 downstream signaling pathways, for example, does not affect the phosphorylation of tyrosine residues in the HER2 intracellular domain (e.g., Y1248 phosphorylation) and/or ligand (e.g., NRG-1 or heregulins)-induced HER2 dimerization and downstream signaling pathways mediated thereby.

Additionally, the VH and VL sequences (or CDR sequences, or full-length heavy and light chain sequences) of other anti-HER2 antibodies binding to HER2 can also be "mixed and matched" with the VH and VL sequences (or CDR sequences, or full-length heavy and light chain sequences) of the anti-HER2 antibody of the present invention. Preferably, when VH and VL chains (or CDRs within these chains, or full-length heavy chain and full-length light chain sequences) are mixed and matched, the VH sequence from a particular VH/VL pair is replaced with a structurally similar VH sequence. Similarly, and preferably, the VL sequence from a specific VH/VL pair is replaced with a structurally similar VL sequence. Similarly, the full-length heavy chain sequence from a specific full-length heavy chain/light chain pair should be replaced with a structurally similar full-length heavy chain sequence. Similarly, the full-length light chain sequence from a particular full-length heavy/light chain pair should be replaced with a structurally similar full-length light chain sequence.

Thus, in one embodiment, the antibody or antigen-binding fragment thereof of the present invention comprises: (a) a heavy chain variable region comprising an amino acid sequence listed in Table 2 or Table 3; and (b) a light chain variable region comprising an amino acid sequence listed in Table 2 or Table 3, or a VL from another anti-HER2 antibody specifically binding to domain D3 or D1 of the HER2 extracellular domain.

In one embodiment, the antibody or antigen-binding fragment thereof of the present invention comprises: (a) a heavy chain variable region comprising an amino acid sequence listed in Table 2 or Table 3, or VH of another anti-HER2 antibody specifically binding to D3 or D1 of the HER2 extracellular domain; and (b) a light chain variable region comprising an amino acid sequence listed in Table 2 or Table 3.

In a specific embodiment, the humanized antibody or antigen-binding fragment thereof of the present invention comprises a VH with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 52. The antibody or antigen-binding fragment thereof specifically binds to D3 of the HER2 extracellular domain.

In a specific embodiment, the humanized antibody or antigen-binding fragment thereof of the present invention comprises a VL with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 81. The antibody or antigen-binding fragment thereof specifically binds to D3 of the HER2 extracellular domain.

In one embodiment, the humanized antibody of the present invention comprises a VH with amino acid sequence having at least 85%, 86%, 87%, 88%, 89%, 90%, 91%, 92%, 93%, 94%, 95%, 96%, 97%, 98%, 99%, or 100% identical to the sequence as ser forth in SEQ ID NO: 52, and a VL with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 81. The antibody or antigen-binding fragment thereof specifically binds to D3 of the HER2 extracellular domain.

In a specific embodiment, the humanized antibody or antigen-binding fragment thereof of the present invention comprises a VH with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 143 or 170. The antibody or antigen-binding fragment thereof specifically binds to D1 of the HER2 extracellular domain.

In a specific embodiment, the humanized antibody or antigen-binding fragment thereof of the present invention comprises a VL with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 171. The antibody or antigen-binding fragment thereof specifically binds to D1 of the HER2 extracellular domain.

In one embodiment, the humanized antibody of the present invention comprises a VH with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 143 or 170, and a VL with amino acid sequence having at least 85%, 86%, 87%, 88%, 89%, 90%, 91%, 92%, 93%, 94%, 95%, 96%, 97%, 98%, 99%, or 100% identical to the sequence as set forth in SEQ ID NO: 171. The antibody or antigen-binding fragment thereof specifically binds to D1 of the HER2 extracellular domain.

In some embodiments, the CDR sequence can be grafted onto a framework region containing one or more mutations compared to the germline sequence. For example, introducing amino acid mutations within the framework regions can maintain or enhance the antigen-binding ability of an antibody (see, e.g., US5530101, US5585089, US5693762, and US6180370). In some embodiments, grafting the CDRs of the parent antibody of the present invention onto an acceptor framework region containing one or more mutations compared to the germline sequence can increase the degree of humanization of the anti-HER2 antibody of the present invention and/or improve the antigen-binding affinity.

In some specific embodiments, the FR region of the anti-HER2 humanized antibody of the present invention that specifically binds to D3 of the HER2 extracellular domain comprises one or more amino acid mutations to improve the antigen-binding affinity of the humanized antibody. For example, one or more amino acid mutations are introduced in the FR region of the VH as set forth in SEQ ID NO: 52, and further, amino acid mutations can be introduced in HFR1, such as mutation of the serine residue at position 30 of HFR1 (corresponding to position 30 of the VH as set forth in SEQ ID NO: 52) is mutated; and/or one or more amino acid mutations are introduced in the FR region of the VL as set forth in SEQ ID NO: 81, and further, one or more amino acid mutations can be introduced in LFR1, LFR2 and/or LFR3. Specifically, one or more amino acid mutations are introduced at the aspartic acid residue at position 2, the methionine residue at position 4 of LFR1, the alanine residue at position 9 (corresponding to position 43 of the VL as set forth in SEQ ID NO: 81), and the threonine residue at position 29 of LFR3 (corresponding to position 85 of the VL as set forth in SEQ ID NO: 81).

In a specific embodiment, the anti-HER2 humanized antibody of the present invention that specifically binds to D3 of the HER2 extracellular domain comprises an amino acid mutation S30N in the HFR region relative to the VH as set forth in SEQ ID NO: 52, and/or comprises one or more amino acid mutations in the LFR region relative to the VL as set forth in SEQ ID NO: 81, including D2I, M4L, A43S, and T85V, which can significantly improve the antigen-binding affinity of the antibody.

In some specific embodiments, the FR regions of the anti-HER2 humanized antibody of the present invention that specifically binds to D3 of the HER2 extracellular region comprise one or more amino acid mutations to improve the degree of humanization of the antibody of the present invention. For example, one or more amino acid mutations are introduced in the FR region of the VH as set forth in SEQ ID NO: 52, and further, amino acid mutations can be introduced in HFR1 and/or HFR2, such as amino acid mutations of the tyrosine residue at position 29 of HFR1 and/or the alanine residue at position 14 of HFR2 (corresponding to position 49 of the amino acid sequence as set forth in SEQ ID NO: 52); and/or one or more amino acid mutations are introduced in the FR region of the VL as set forth in SEQ ID NO: 81, and further, amino acid mutations can be introduced in LFR3, such as amino acid mutation of phenylalanine residue at position 31 of LFR3 (corresponding to position 87 of the VL as set forth in SEQ ID NO: 81). Specifically, the anti-HER2 humanized antibody that specifically binds to D3 of the HER2 extracellular domain comprises the following amino acid mutations: Y29F and/or A49S in the HFR relative to the VH as set forth in SEQ ID NO: 52, and/or comprises the amino acid mutation F87Y in the LFR region relative to the VL as set forth in SEQ ID NO: 81, which can enhance the degree of humanization of the antibody.

In specific embodiments, the FR regions of the anti-HER2 humanized antibody of the present invention that specifically binds to D1 of the HER2 extracellular domain comprise one or more amino acid mutations. For example, one or more amino acid mutations can be introduced into the FR regions of the VH as set forth in SEQ ID NO: 143, and one or more amino acid mutations can be introduced in HFR1 and/or HFR3 to improve the degree of humanization of the anti-HER2 antibody of the present invention. Preferably, one or more amino acid mutations are introduced at the serine residue at position 9, the valine residue at position 18 of HFR1, and/or the valine residue at position 3 (corresponding to position 68 of the VH as set forth in SEQ ID NO: 143), the phenylalanine residue at position 4 (corresponding to position 69 of the VH as set forth in SEQ ID NO: 143), the leucine residue at position 6 (corresponding to position 71 of the VH as set forth in SEQ ID NO: 143), the valine residue at position 10 (corresponding to position 75 of the VH as set forth in SEQ ID NO: 143), the isoleucine residue at position 17 (corresponding to position 82 of the VH as set forth in SEQ ID NO: 143), and the phenylalanine residue at position 29 (corresponding to position 91 of the VH as set forth in SEQ ID NO: 143) of HFR3. One or more amino acid mutations can be introduced in the FR regions of the VL as set forth in SEQ ID NO: 171 to improve the degree of humanization of the anti-HER2 antibody of the present invention. For example, an amino acid mutation can be introduced at the asparagine residue at position 15 of LFR2 (corresponding to position 49 of the VL as set forth in SEQ ID NO: 171).

In a specific embodiment, the anti-HER2 humanized antibody of the present invention that specifically binds to D1 of the HER2 extracellular domain comprises one or more of the following amino acid mutations in the HFR region relative to the VH as set forth in SEQ ID NO: 143: S9G, V18L, V68T or V68S, F69I, L71V or L71R, V75K or V75T, I82L or I82M, F91Y. In some embodiments, the humanized antibody that specifically binds to D1 of the HER2 extracellular domain comprises one or more of the following amino acid mutations in the HFR region relative to the VH as set forth in SEQ ID NO: 143: V68T, F69I, L71R, V75K, and F91Y, which can significantly improve the degree of humanization of the antibody.

In some embodiments, the present invention introduces amino acid mutations into the CDR regions of the VH and/or VL of the humanized antibody to improve one or more properties of the antibody, such as enhancing the degree of humanization, improving antigen-binding affinity, increasing production yeild, and enhancing stability (e.g., reducing the risk of aspartic acid isomerization and/or asparagine deamidation). Site-directed mutagenesis or PCR-induced mutagenesis can be performed, and the effect of mutation(s) on the functional properties of the antibody can be evaluated using *in vitro* or *in vivo* assys known in the art. The mutation can be amino acid substitution, addition or deletion, preferably an amino acid substitution. Specifically, no more than 1, 2, 3, 4, 5, 6, 7, 8, 9, or 10 amino acid residues in the heavy chain CDR regions or light chain CDR regions are mutated.

In some specific embodiments, the anti-HER2 humanized antibody of the present invention that specifically binds to D3 of the HER2 extracellular domain comprises no more than 1, 2, 3, 4, 5, 6, 7, 8, 9, or 10 amino acid mutations in the heavy chain CDR regions or the light chain CDR regions.

In a specific embodiment, one or more amino acid mutations are introduced in the CDR regions of the VH as set forth in SEQ ID NO: 52; furthermore, one, two, three, four, or five amino acid mutations can be introduced in HCDR1, HCDR2 and/or HCDR3. For example, one or more amino acid mutations can be introduced at the serine residue at position 1 of HCDR1 (corresponding to position 31 of the VH as set forth in SEQ ID NO: 52), the glycine residue at position 5 of HCDR2 (corresponding to position 53 of the VH as set forth in SEQ ID NO: 52), the serine residue at position 7 of HCDR2 (corresponding to position 55 of the VH as set forth in SEQ ID NO: 52), the threonine residue at position 9 of HCDR2 (corresponding to position 57 of the VH as set forth in SEQ ID NO: 52), the proline residue at position 12 of HCDR2 (corresponding to position 60 of the VH as set forth in SEQ ID NO: 52), the aspartic acid residue at position 13 of HCDR2 (corresponding to position 61 of the VH as set forth in SEQ ID NO: 52), the serine residue at position 14 of HCDR2 (corresponding to position 62 of the VH as set forth in SEQ ID NO: 52), and/or the alanine residue at position 3 of HCDR3 (corresponding to position 97 of the VH as set forth in SEQ ID NO: 52).

In one embodiment, one or more amino acid mutations are introduced in HCDR1, HCDR2 and/or HCDR3 of the VH as set forth in SEQ ID NO: 52. For example, amino acid mutations are introduced at the serine residue at position 7 (corresponding to position 55 of the VH as set forth in SEQ ID NO: 52), the threonine residue at position 9 (corresponding to position 57 of the VH as set forth in SEQ ID NO: 52), the proline residue at position 12 (corresponding to position 60 of the VH as set forth in SEQ ID NO: 52), the aspartic acid residue at position 13 (corresponding to position 61 of the VH as set forth in SEQ ID NO: 52), the serine residue at position 14 (corresponding to position 62 of the VH as set forth in SEQ ID NO: 52) of HCDR2, and/or the alanine residue at position 3 of HCDR3 (corresponding to position 97 of the VH as set forth in SEQ ID NO: 52) of the VH as set forth in SEQ ID NO: 52.

In a specific embodiment, one or more amino acid mutations are introduced into the CDR regions of the VL as set forth in SEQ ID NO: 81; furthermore, one, two, or three amino acid mutations can be introduced in LCDR1, LCDR2 and/or LCDR3. For example, one or more amino acid mutations can be introduced at the valine residue at position 7 (corresponding to position 27c of the VL as set forth in SEQ ID NO: 81), the histidine residue at position 8 (corresponding to position 27d of the VL as set forth in SEQ ID NO: 81), the glycine residue at position 11 (corresponding to position 29 of the VL as set forth in SEQ ID NO: 85) of LCDR1, the phenylalanine residue at position 6 of LCDR2 (corresponding to position 55 of the VL as set forth in SEQ ID NO: 81), and/or the serine residue at position 1 (corresponding to position 89 of the VL as set forth in SEQ ID NO: 81), the tyrosine residue at position 8 of LCDR3 (corresponding to position 96 of the VL as set forth in SEQ ID NO: 81).

In one embodiment, the present invention introduces multiple amino acid mutations in the CDR regions of the VH and VL of the above-mentioned anti-HER2 humanized antibody that specifically binds to D3 of the HER2 extracellular domain, to enhance the degree of humanization, increase the production yield, and/or improve the stability (e.g., to reduce the risk of aspartic acid isomerization) of the antibody of the present invention. Specifically, one or more amino acid mutations are introduced at the serine residue at position 7 (corresponding to position 55 of the VH as set forth in SEQ ID NO: 52), the threonine residue at position 9 (corresponding to position 57 of the VH as set forth in SEQ ID NO: 52), the proline residue at position 12 (corresponding to position 60 of the VH as set forth in SEQ ID NO: 52), the aspartic acid residue at position 13 (corresponding to position 61 of the VH as set forth in SEQ ID NO: 52), the serine residue at position 14 of HCDR2 (corresponding to position 62 of the VH as set forth in SEQ ID NO: 52), and at the alanine residue at position 3 of HCDR3 (corresponding to position 97 of the VH as set forth in SEQ ID NO: 52) of the VH as set forth in SEQ ID NO: 52, and at the glycine residue at position 11 of LCDR1 (corresponding to position 29 of the VL as set forth in SEQ ID NO: 81) of the VL as set forth in SEQ ID NO: 81. Preferably, amino acid mutations are selectively introduced at any one or two of the following amino acid residues: the proline residue at position 12 (corresponding to position 60 of the VH as set forth in SEQ ID NO: 52), the aspartic acid residue at position 13 (corresponding to position 61 of the VH as set forth in SEQ ID NO: 52), and the serine residue at position 14 of HCDR2 (corresponding to position 62 of the VH as set forth in SEQ ID NO: 52), and the alanine residue at position 3 of HCDR3 (corresponding to position 97 of the VH shown in SEQ ID NO: 52) of the VH as set forth in SEQ ID NO: 52, and the glycine residue at position 11 of LCDR1 (corresponding to position 29 of the VL as set forth in SEQ ID NO: 81) of the VL as set forth in SEQ ID NO: 81.

In some specific embodiments, the heavy chain CDR regions or light chain CDR regions of the anti-HER2 humanized antibody of the present invention that specifically binds to D1 of the HER2 extracellular domain comprise no more than 1, 2, 3, 4, or 5 amino acid mutations.

In a specific embodiment, one or more amino acid mutations are introduced in the CDR regions of the VH as set forth in SEQ ID NO: 143; further, 1, 2, 3, 4, or 5 amino acid mutations can be introduced in HCDR1, HCDR2 and/or HCDR3. For example, one or more mutations can be introduced at the serine residue at position 3 of HCDR1 (corresponding to position 33 of the VH as set forth in SEQ ID NO: 143); the glutamic acid residue at position 5 (corresponding to position 53 of the VH as set forth in SEQ ID NO: 143), the glutamic acid residue at position 8 (corresponding to position 56 of the VH as ser forth in SEQ ID NO: 143), the aspartic acid residue at position 14 (corresponding to position 62 of the VH as set forth in SEQ ID NO: 143), the phenylalanine residue at position 15 (corresponding to position 63 of the VH as set forth in SEQ ID NO: 143) of HCDR2; and/or the arginine residue at position 3 (corresponding to position 97 of the VH as set forth in SEQ ID NO: 143), the tyrosine residue at position 4 (corresponding to position 98 of the VH as set forth in SEQ ID NO: 143), the aspartic acid residue at position 5 (corresponding to position 99 of the VH as set forth in SEQ ID NO: 143) of HCDR3.

In a specific embodiment, one or more amino acid mutations are introduced in the CDR regions of the VL as set forth in SEQ ID NO: 171. For example, mutations can be introduced at one or more of the following positions: the lysine residue at position 1 of LCDR1 (corresponding to position 24 of the VL as set forth in SEQ ID NO: 171); the serine residue at position 1 (corresponding to position 50 of the VL as set forth in SEQ ID NO: 171), the tyrosine residue at position 4 (corresponding to position 53 of the VL as set forth in SEQ ID NO: 171), the tyrosine residue at position 6 (corresponding to position 55 of the VL as set forth in SEQ ID NO: 171) of LCDR2; and/or the histidine residue at position 3 of LCDR3 (corresponding to position 91 of the VL as set forth in SEQ ID NO: 171).

In one embodiment, the present invention introduces multiple amino acid mutations in the CDR regions of the VH and VL of the above-mentioned anti-HER2 humanized antibody that specifically binds to D1 of the HER2 extracellular domain, to improve the humanization degree of the antibody of the present invention, enhance antigen-binding affinity, increase production yield, and/or enhance stability (e.g., to reduce the risk of potential asparagine deamidation). Specifically, mutations are introduced at the following positions: the glutamic acid residue at position 5 of HCDR2 of the VH as set forth in SEQ ID NO: 143 (corresponding to position 53 of the VH as set forth in SEQ ID NO: 143), the lysine residue at position 1 of LCDR1 of the VL as ser forth in SEQ ID NO: 171 (corresponding to position 24 of the VL as set forth in SEQ ID NO: 171), the serine residue at position 1 (corresponding to position 50 of the VL as set forth in SEQ ID NO: 171), the tyrosine residue at position 4 (corresponding to position 53 of the VL as set forth in SEQ ID NO: 171), the tyrosine residue at position 6 (corresponding to position 55 of the VL as set forth in SEQ ID NO: 171) of LCDR2, and the histidine residue at position 3 of LCDR3 (corresponding to position 91 of the VL as set forth in SEQ ID NO: 171).

In some embodiments, multiple amino acid mutations can also be introduced in the CDR regions and framework regions of the variable domains of the humanized antibody to further improve one or more properties of the antibody (e.g., enhancing the degree of humanization, increasing antigen-binding affinity, improving stability, or increasing production yield). In specific embodiments, multiple amino acid mutations are introduced into one or two CDR regions and one HFR region of the VH as set forth in SEQ ID NO: 143, and/or three CDR regions of the VL as set forth in SEQ ID NO: 171 of the above-mentioned humanized antibody that specifically binds to D1 of the HER2 extracellular domain. Preferably, multiple amino acid mutations are introduced at the glutamic acid residue at position 5 of HCDR2 of the VH as set forth in SEQ ID NO: 143 (corresponding to position 53 of the VH as set forth in SEQ ID NO: 143), the valine residue at position 3 (corresponding to position 68 of the VH as set forth in SEQ ID NO: 143), the phenylalanine residue at position 4 (corresponding to position 69 of the VH as set forth in SEQ ID NO: 143), the leucine residue at position 6 (corresponding to position 71 of the VH as set forth in SEQ ID NO: 143), the valine residue at position 10 (corresponding to position 75 of the VH as set forth in SEQ ID NO: 143), the phenylalanine residue at position 29 (corresponding to position 91 of the VH as set forth in SEQ ID NO: 143) of HFR3, and/or the lysine residue at position 1 of LCDR1 as set forth in SEQ ID NO: 171 (corresponding to position 24 of the VL as set forth in SEQ ID NO: 171), the serine residue at position 1 (corresponding to position 50 of the VL as set forth in SEQ ID NO: 171), the tyrosine residue at position 4 (corresponding to position 53 of the VL as set forth in SEQ ID NO: 171), the tyrosine residue at position 6 (corresponding to position 55 of the VL as set forth in SEQ ID NO: 171) of LCDR2, and the histidine residue at position 3 of LCDR3 (corresponding to position 91 of the VL as set forth in SEQ ID NO: 171). These mutations not only further enhance a higher degree of humanization of the humanized antibody of the present invention, but also significantly improve the binding affinity to HER2 (with a K_{D} value of approximately 8×10⁻⁹ ~ 9×10⁻¹⁰ M, preferably 5×10⁻⁹ ~ 9×10⁻¹⁰ M), and reduce the risk of potential asparagine deamidation and increase the production yield.

In other embodiments, the present invention provides an isolated anti-HER2 monoclonal antibody or antigen-binding fragment thereof, wherein the antibody and antigen-binding fragment thereof specifically binds to D3 of the HER2 extracellular domain, comprising: (1) HCDR1 having an amino acid sequence of X₁YGMS (wherein X₁ = S, N, or D; i.e., the sequence as set forth in SEQ ID NO: 217), preferably X₁=S; (2) HCDR2 having an amino acid sequence of SISGX₂GX₃YX₄KYX₅X₆X₇VKG (wherein X₂=G or S; X₃=S or N; X₄= T or A; Xs=P, A, G, or V; X₆ = D, G, E, P, Q, or R; X₇ = S, K, or N; i.e., the sequence as set forth in SEQ ID NO: 218), preferably X₂ = G, X₃ = S, X₄ = T, X₅ = P or V, X₆ = D, P, or E, X₇ = S or K; (3) HCDR3 having an amino acid sequence of DYX₈GFFDV (wherein X₈ = A, I, N, R, S, or V; i.e., the sequence as set forth in SEQ ID NO: 219), preferably X₈ = V; (4) LCDR1 having an amino acid sequence of RSSQSLX₉X₁₀SNX₁₁NTYLH (wherein X₉ = V or L; X₁₀ = H or S; X₁₁ = G, A, I, S, R, or T; i.e., the sequence as set forht in SEQ ID NO: 220), preferably X₉ = V or L, X₁₀ = H, X₁₁ = R; (5) LCDR2 having an amino acid sequence of KVSNRX₁₂S (wherein X₁₂ = F, D, or P; i.e., the sequence as set forth in SEQ ID NO: 221), preferably X₁₂ = F; (6) LCDR3 having an amino acid sequence X₁₃QSTHVPX₁₄T (wherein X₁₃ = S or Q; X₁₄ =Y or W; i.e., the sequence as set forth in SEQ ID NO: 222), preferably X₁₃ = S, X₁₄ = Y; , or amino acid sequences which are at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively. The antibody or antigen-binding fragment exhibits high humanization, high production yield, and minimal risk of aspartate isomerization.

In some specific embodiments, the above-mentioned antibody or antigen-binding fragment that specifically binds to D3 of the HER2 extracellular domain comprises: HCDR1 as set forth in SEQ ID NO: 11, 12, or 13; HCDR2 as set forth in SEQ ID NO: 14, 15, 16, 17, 18, 19, 20, 21, 22, 23, 24, 25, 26, 27, 28, 29, 30, or 31; HCDR3 as set forth in SEQ ID NO: 32, 33, 34, 35, 36, or 37; LCDR1 as set forth in SEQ ID NO: 38, 39, 40, 41, 42, 43, 44, or 45; LCDR2 as set forth in SEQ ID NO: 46, 47, or 48; and LCDR3 as set forth in SEQ ID NO: 49, 50, or 51.

In a specific embodiment, the anti-HER2 antibody or antigen-binding fragment of the present invention specifically binds to D3 of the HER2 extracellular domain, comprising the following HCDR1, HCDR2, HCDR3, LCDR1, LCDR2 and LCDR3, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2 and LCDR3, respectively:
(1) HCDR1 as set forth in SEQ ID NO: 11, HCDR2 as set forth in SEQ ID NO: 14, 15, 16, 17, 18, 19, or 20, HCDR3 as set forth in SEQ ID NO: 32, and LCDR1 as set forth in SEQ ID NO: 38, LCDR2 as set forth in SEQ ID NO: 46, LCDR3 as set forth in SEQ ID NO: 49; or
(2) HCDR1 as set forth in SEQ ID NO: 12 or 13, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 46, LCDR3 as set forth in SEQ ID NO: 49; or
(3) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 47 or 48, LCDR3 as set forth in SEQ ID NO: 49; or
(4) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 39, 40, 41, 42, 43, 44, or 45, LCDR2 as set forth in SEQ ID NO: 46, LCDR3 as set forth in SEQ ID NO: 49; or
(5) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 46, LCDR3 as set forth in SEQ ID NO: 50 or 51; or
(6) HCDR1 as set forth in SEQ ID NO: 11, HCDR2 as set forth in SEQ ID NO: 14, HCDR3 as set forth in SEQ ID NO: 33, 34, 35, 36, or 37; and LCDR1 as set forth in SEQ ID NO: 38, LCDR2 as set forth in SEQ ID NO: 46, LCDR3 as set forth in SEQ ID NO: 49; or
(7) HCDR1 as set forth in SEQ ID NO: 11, HCDR2 as set forth in SEQ ID NO: 14, 21, 22, 23, 24, 25, 26, 27, 28, 29, 30, or 31, HCDR3 as set forth in SEQ ID NO: 37; and LCDR1 as set forth in SEQ ID NO: 42, LCDR2 as set forth in SEQ ID NO: 46, LCDR3 as set forth in SEQ ID NO: 49; or
(8) HCDR1 as set forth in SEQ ID NO: 11, HCDR2 as set forth in SEQ ID NO: 21, HCDR3 as set forth in SEQ ID NO: 32; and LCDR1 as set forth in SEQ ID NO: 42, LCDR2 as set forth in SEQ ID NO: 46, LCDR3 as set forth in SEQ ID NO: 49.

In a specific embodiment, the anti-HER2 antibody or antigen-binding fragment of the present invention specifically binds to D3 of the HER2 extracellular domain, comprising: HCDR1 as set forth in SEQ ID NO: 11, HCDR2 as set forth in SEQ ID NO: 14, 17, 19, 21, 23, 24, 27, 28, 29, 30, or 31, HCDR3 as set forth in SEQ ID NO: 32 or 37, and LCDR1 as set forth in SEQ ID NO: 38 or 42, LCDR2 as set forth in SEQ ID NO: 46, LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% sequence identity to the HCDR1, HCDR2, HCDR3, LCDR1, LCDR2, and LCDR3, respectively. Preferably, the antibody or antigen-binding fragment comprises: HCDR1 as set forth in SEQ ID NO: 11, HCDR2 as set forth in SEQ ID NO: 14, 17, 19, 24, 29, 30, or 31, HCDR3 as set forth in SEQ ID NO: 32 or 37, and LCDR1 as set forth in SEQ ID NO: 38 or 42, LCDR2 as set forth in SEQ ID NO: 46, and LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% sequence identity to the HCDR1, HCDR2, HCDR3, LCDR1, LCDR2, and LCDR3, respectively. More preferably, the antibody or antigen-binding fragment comprises: HCDR1 as set forth in SEQ ID NO: 11, HCDR2 as set forth in SEQ ID NO: 24, 29, 30, or 31, HCDR3 as set forth in SEQ ID NO: 37, and LCDR1 as set forth in SEQ ID NO: 42, LCDR2 as set forth in SEQ ID NO: 46, and LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% sequence identity to the HCDR1, HCDR2, HCDR3, LCDR1, LCDR2, and LCDR3, respectively.

In other embodiments, the present invention provides an isolated anti-HER2 monoclonal antibody or antigen-binding fragment thereof, wherein the antibody and antigen-binding fragment thereof specifically binds to D1 of the HER2 extracellular domain, comprising: (1) HCDR1 having an amino acid sequence of DYX₁₅MH (wherein X₁₅=S or A; i.e., the sequence as set forth in SEQ ID NO: 223), preferably X₁₅=S; (2) HCDR2 having an amino acid sequence of WINTX₁₆TGX₁₇PTYADX₁₈X₁₉KG (wherein X₁₆=E, N, G, Y or I; X₁₇=E, D or S; X₁₈=D, K or N; X₁₉=F or V; i.e., the sequence as set forth in SEQ ID NO: 224), preferably X₁₆=G, X₁₇=E, X₁₈=D, X₁₉=F; (3) HCDR3 having an amino acid sequence of VGX₂₀X₂₁X₂₂YAMDY (wherein X₂₀=R or Y; X₂₁=Y or G; X₂₂=D or S; i.e., the sequence as set forth in SEQ ID NO: 225), preferably X₂₀=R, X₂₁=Y, X₂₂=D; (4) LCDR1 having an amino acid sequence of X₂₃ASQDVYTAVA (wherein X₂₃=K or R; i.e., the sequence as set forth in SEQ ID NO: 226), preferably X₂₃=R; (5) LCDR2 having an amino acid sequence of X₂₄ASX₂₅RX₂₆T (wherein X₂₄=S, A, D, E, K, L, Q, R, W or Y; X₂₅=Y, D, E, K, N, Q, S or T; X₂₆=Y, A, E, P or Q; i.e., the sequence as set forth in SEQ ID NO: 227), preferably X₂₄=S, A, L or Y, X₂₅=S, X₂₆=P; (6) LCDR3 having an amino acid sequence QQX₂₇YSTPPT (wherein X₂₇=H, S, A or Y; i.e., the sequence as set forth in SEQ ID NO: 228), preferably X₂₇=Y; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively. The antibody or antigen-binding fragment not only exhibits high affinity to HER2 (e.g., K_{D} value <1×10⁻⁸ M, or <5×10⁻⁹ M, or <1×10⁻⁹ M; preferably K_{D} value <5×10⁻⁹ M, or <1×10⁻⁹ M), but also demonstrates higher humanization and improved stability.

In specific embodiments, the above-mentioned antibody or antigen-binding fragment that specifically binds to D1 of the HER2 extracellular domain comprises: HCDR1 as set forth in SEQ ID NO: 98 or 99; HCDR2 as set forth in SEQ ID NO: 100, 101, 102, 103, 104, 105, 106, 107, 108, or 109; HCDR3 as set forth in SEQ ID NO: 110, 111, 112, or 113; LCDR1 as set forth in SEQ ID NO: 114 or 115; LCDR2 as set forth in SEQ ID NO: 116, 117, 118, 119, 120, 121, 122, 123, 124, 125, 126, 127, 128, 129, 130, 131, 132, 133, 134, 135, 136, 137, or 138; and LCDR3 as set forth in SEQ ID NO: 139, 140, 141, or 142.

In a specific embodiment, the anti-HER2 antibody or antigen-binding fragment of the present invention specifically binds to D1 of the HER2 extracellular domain, comprising the following HCDR1, HCDR2, HCDR3, LCDR1, LCDR2 and LCDR3, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2 and LCDR3, respectively:
(1) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 114, LCDR2 as set forth in SEQ ID NOs: 116, 117, 118, 119, 120, 121, 122, 123, 124, 125, 126, 127, or 128, LCDR3 as set forth in SEQ ID NO: 139; or
(2) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 140, 141, or 142; or
(3) HCDR1 as set forth in SEQ ID NO: 98, HCDR2 as set forth in SEQ ID NO: 101, 102, 103, 104, 105, 106, 107, 108, or 109, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 139; or
(4) HCDR1 as set forth in SEQ ID NO: 98, HCDR2 as set forth in SEQ ID NO: 100, HCDR3 as set forth in SEQ ID NO: 111, 112, or 113, LCDR1 as set forth in SEQ ID NO: 114, LCDR2 as set forth in SEQ ID NO: 116, LCDR3 as set forth in SEQ ID NO: 139; or
(5) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 115, LCDR2 as set forth in SEQ ID NO: 116, LCDR3 as set forth in SEQ ID NO: 139; or
(6) HCDR1 as set forth in SEQ ID NO: 99, HCDR2 as set forth in SEQ ID NO: 100, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 139; or
(7) HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 130, 131, 132, 133, 134, 135, 136, 137, or 138, LCDR3 as set forth in SEQ ID NO: 142.

In a specific embodiment, the anti-HER2 antibody or antigen-binding fragment of the present invention is capable of specifically binding to D1 of the HER2 extracellular domain, comprising the following HCDR1, HCDR2, HCDR3, LCDR1, LCDR2, and LCDR3, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively: HCDR1 as set forth in SEQ ID NO: 98, HCDR2 as set forth in SEQ ID NO: 100 or 102, HCDR3 as set forth in SEQ ID NO: 110, LCDR1 as set forth in SEQ ID NO: 114 or 115, LCDR2 as set forth in SEQ ID NOs: 116, 123, 127, 129, 130, 131, 132, 133, 134, 135, 136, 137, or 138, and LCDR3 as set forth in SEQ ID NO: 139 or 142. Preferably, the antibody or antigen-binding fragment comprises: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NOs: 129, 133, 136, or 138, and LCDR3 as set forth in SEQ ID NO: 142; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively. The binding affinity (K_{D}) of the antibody or antigen-binding fragment thereof to HER2 is < 5×10⁻⁹ M, or < 1×10⁻⁹ M, preferably < 1×10⁻⁹ M. More preferably, the antibody or antigen-binding fragment thereof comprises: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 133, and LCDR3 as set forth in SEQ ID NO: 142; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

In some embodiments, the anti-HER2 antibody or antigen-binding fragment thereof of the present invention further comprises a heavy chain variable region having the HCDR1, HCDR2, and HCDR3, and a light chain variable region having LCDR1, LCDR2, and LCDR3.

In specific embodiments, the antibody or antigen-binding fragment of the present invention can specifically bind to D3 of the HER2 extracellular domain, comprising: a heavy chain variable region (VH) having at least 80% (e.g., at least 85%, at least 90%, at least 95%, at least 96%, at least 97%, at least 98%, at least 99%, or 100%) identity to the amino acid sequence as set forth in SEQ ID NO: 52, 53, 54, 55, 56, 57, 58, 59, 60, 61, 62, 63, 64, 65, 66, 67, 68, 69, 70, 71, 72, 73, 74, 75, 76, 77, 78, 79, or 80; and a light chain variable region (VL) having at least 80% (e.g., at least 85%, at least 90%, at least 95%, at least 96%, at least 97%, at least 98%, at least 99%, or 100%) identity to the amino acid sequence as set forth in SEQ ID NO: 81, 82, 83, 84, 85, 86, 87, 88, 89, 90, 91, 92, 93, 94, 95, 96, or 97.

In a specific embodiment, the antibody or antigen-binding fragment of the present invention can specifically bind to D3 of the HER2 extracellular domain, comprising a heavy chain variable region having the HCDR1, HCDR2 and HCDR3 and a light chain variable region having the LCDR1, LCDR2 and LCDR3, wherein the heavy and light chain variable regions comprise amino acid sequence having at least 85%, 86%, 87%, 88%, 89%, 90%, 91%, 92%, 93%, 94%, 95%, 96%, 97%, 98%, 99%, or 100% identity to the VH as set forth in SEQ ID NO: 52 and the VL as set forth in SEQ ID NO: 81, 82, 83, 84, 85, 86, 87, 88, 89, 90, 91, 92, 93, 94, 95, 96, or 97, respectively; or comprise amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 53, 54, 55, 56, 57, 58, 59, 60, 61, 62, 63, 64, 65, 66, 67, or 68 and the VL as set forth in SEQ ID NO: 81, respectively; or comprise amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 68, 69, 70, 71, 72, 73, 74, 75, 76, 77, 78, 79, or 80 and the VL as set forth in SEQ ID NO: 92, respectively.

In some embodiments, the antibody or antigen-binding fragment thereof that specifically binds to D3 of the HER2 extracellular domain comprises amino acid sequences having at least 85%, 86%, 87%, 88%, 89%, 90%, 91%, 92%, 93%, 94%, 95%, 96%, 97%, 98%, 99%, or 100% identity to the VH as set forth in any one of SEQ ID NOs: 52, 57, 62, 68, 73, 78, 79, and 80, and the VL as set forth in SEQ ID NO: 81 or 92, respectively. Preferably, the antibody or antigen-binding fragment thereof comprises amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 73, 78, 79, or 80 and the VL as set forth in SEQ ID NO: 92, respectively. More preferably, the antibody or antigen-binding fragment thereof comprises amino acid sequences having at least 85%, 86%, 87%, 88%, 89%, 90%, 91%, 92%, 93%, 94%, 95%, 96%, 97%, 98%, 99%, or 100% identity to the VH shown in SEQ ID NO: 80 and the VL shown in SEQ ID NO: 92, respectively.

In some specific embodiments, the antibody or antigen-binding fragment thereof of the present invention can specifically bind to D1 of the HER2 extracellular domain, comprising: a heavy chain variable region VH having at least 80% (e.g., at least 85%, at least 90%, at least 95%, at least 96%, at least 97%, at least 98%, at least 99%, or 100%) identity to the amino acid sequence as set forth in SEQ ID NO: 143, 144, 145, 146, 147, 148, 149, 150, 151, 152, 153, 154, 155, 156, 157, 158, 159, 160, 161, 162, 163, 164, 165, 166, 167, 168, 169, or 170; and a light chain variable region VL having at least 80% (e.g., at least 85%, at least 90%, at least 95%, at least 96%, at least 97%, at least 98%, at least 99%, or 100%) identity to the amino acid sequence as set forth in SEQ ID NO: 171, 172, 173, 174, 175, 176, 177, 178, 179, 180, 181, 182, 183, 184, 185, 186, 187, 188, 189, 190, 191, 192, 193, 194, 195, 196, 197, or 198.

In one specific embodiment, the antibody or antigen-binding fragment thereof of the present invention can specifically bind to D1 of the HER2 extracellular domain, comprising a heavy chain variable region having the HCDR1, HCDR2 and HCDR3, and a light chain variable region having the LCDR1, LCDR2 and LCDR3, wherein the antibody or antigen-binding fragment thereof comprises an amino acid sequence having at least 85%, 86%, 87%, 88%, 89%, 90%, 91%, 92%, 93%, 94%, 95%, 96%, 97%, 98%, 99%, or 100% identity to the VH as set forth in SEQ ID NO: 143 and the VL as set forth in SEQ ID NO: 171, 172, 173, 174, 175, 176, 177, 178, 179, 180, 181, 182, 183, 184, 185, 186, 187, or 188, respectively; or an amino acid sequence having at least 85%, 86%, 87%, 88%, 89%, 90%, 91%, 92%, 93%, 94%, 95%, 96%, 97%, 98%, 99%, or 100% identity to the VH as set forth in SEQ ID NO: 144, 145, 146, 147, 148, 149, 150, 151, 152, 153, 154, 155, 156, 157, 158, 159, 160, 161, 162, 163, 164, 165, 166, 167, 168, or 170 and the VL as set forth in SEQ ID NO: 171, respectively; or an amino acid sequence having at least 85%, 86%, 87%, 88%, 89%, 90%, 91%, 92%, 93%, 94%, 95%, 96%, 97%, 98%, 99%, or 100% identity to the VH as set forth in SEQ ID NO: 169 and the VL as set forth in SEQ ID NO: 189, 190, 191, 192, 193, 194, 195, 196, 197, or 198, respectively.

In some embodiments, the antibody or antigen-binding fragment thereof that specifically binds to D1 of the HER2 extracellular domain comprises amino acid sequences having at least 85%, 86%, 87%, 88%, 89%, 90%, 91%, 92%, 93%, 94%, 95%, 96%, 97%, 98%, 99%, or 100% identity to the VH as set forth in any one of SEQ ID NOs: 143, 148, 156, 158, 160, 161, 165, and 169, and the VL as set forth in any one of SEQ ID NO: 171, 172, 180, 184, 188, 189, 190, 191, 192, 193, 194, 195, 196, 197, or 198, respectively. Preferably, the antibody or antigen-binding fragment thereof comprises amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 169 and the VL as set forth in SEQ ID NO: 189, 190, 191, 192, 193, 194, 195, 196, 197, or 198, respectively. More preferably, the antibody or antigen-binding fragment thereof comprises amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 169 and the VL as set forth in SEQ ID NO: 189, 193, 196, or 198, respectively. Further preferably, the above-mentioned antibody or antigen-binding fragment thereof of the present invention comprises the VH as set forth in SEQ ID NO: 169 and the VL as set forth in SEQ ID NO: 193.

The anti-HER2 humanized antibody or antigen-binding fragment thereof of the present invention has strong binding activity to HER2-expressing cells, has no effect on HER2-mediated signaling pathways, does not cross-react with other members of the human ErbB/HER family (including EGFR, HER3 and HER4), and has a high degree of humanization and stability.

### 2. Anti-HER2 Biparatopic Antibody

In one aspect, the present invention provides an anti-HER2 biparatopic antibody, comprising two antigen-binding domains capable of non-competitively binding to the HER2 extracellular domain simultaneously, wherein the first antigen-binding domain and the second antigen-binding domain each specifically bind to different epitopes on the HER2 extracellular domain. Further, the biparatopic antibody can simultaneously bind to any two epitopes on D1, D3 and D4 of the HER2 extracellular domain. Preferably, the biparatopic can simultaneously bind to D1 and D3 on the HER2 extracellular domain, for example, the first antigen-binding domain specifically binds to D3 of the HER2 extracellular domain, and the second antigen-binding domain specifically binds to D1 of the HER2 extracellular domain of HER2. The binding epitopes of the first and second antigen-binding domains are distinct from those of trastuzumab or pertuzumab.

The anti-HER2 biparatopic antibody of the present invention has the following functional characteristics:
(i) The anti-HER2 biparatopic antibody is a tetravalent molecule that can simultaneously bind to two different epitopes on the HER2 extracellular domain, thereby cross-linking HER2 on the cell surface to form antigen-antibody cross-linked multimers or clusters. Obviously, the efficiency and size of such multimers or clusters formation are depending on the density of target antigen on cell surface, and the formation of clusters will trigger endocytosis by the cell, with the rate and intensity of the endocytosis positively correlating with the size of the clusters. Specifically, compared to the corresponding anti-HER2 monospecific antibodies or antigen-binding fragments thereof as well as trastuzumab, the biparatopic antibody has significantly enhanced internalization, with an endocytosis rate of over 80% in HER2-overexpressing tumor cells and over 60% in HER2-low-expressing tumor cells.
(ii) Unlike the corresponding anti-HER2 monospecific antibodies or antigen-binding fragments thereof and trastuzumab, whose intracellular trafficking pathway following internalization is primarily recycling to the cell surface, the biparatopic antibody forms clusters with HER2 on cell surface and is internalized, followed by lysosomal trafficking being its dominant intracellular trafficking pathway. Therefore, the clusters formed by the biparatopic antibody and HER2 can be efficiently transported into lysosomes for degradation.
(iii) The biparatopic antibody can effectively trigger the down-regulation of HER2 on tumor cell surface, thereby significantly inhibiting the proliferation of HER2-overexpressing tumor cells.
(iv) The biparatopic antibody does not interfere with the regulation of HER2 and downstream signaling pathways mediated thereby, including not inducing, blocking, or inhibiting ligand (e.g., NRG-1)-induced HER2 dimerization and activation of its downstream signaling pathways. Therefore, the anti-HER2 biparatopic antibody does not affect the normal biological functions and regulation of HER2 and downstream signaling pathways mediated thereby in normal tissues or cells (e.g., cardiomyocytes).
(v) The anti-HER2 biparatopic antibody demonstrates high stability, high production yield, and a high monomer content (approximately 98% or more).

The formats of the biparatopic antibody include, but are not limited to: scFv- or Diabody-based bispecific formats (e.g., scFv-scFv, scFv-Fab or Fab-scFv), IgG-scFv fusion proteins, DVD-Ig, Quadroma, Knob-into-hole, Common Light Chain, CrossMab, CrossFab, SEEDbody, Leucine Zipper, Duobody, IgG1/IgG2, Dual-acting Fab (DAF)-IgG, and Mab² bispecific formats (see, e.g., Klein et al., mAbs 2012, 4:653-663 and references cited therein). The exemplary anti-HER2 biparatopic antibody of the present invention adopts the DVD-Ig format, which not only has high binding activity to HER2-expressing tumor cells but also exhibits a superior cellular internalization. In addition, it can ensure the stability of the biparatopic antibody of the present invention (e.g., a high monomer content, about 98% or more).

The anti-HER2 biparatopic antibody has strong binding activity to HER2-expressing cells and no cross-reactivity to other members of the human ErbB/HER family (including EGFR, HER3 and HER4).

The anti-HER2 biparatopic antibody demonstrates significantly enhanced internalization in HER2-expressing tumor cells. In some embodiments, the biparatopic antibody exhibits an internalization rate exceeding 80% in HER2-overexpressing tumor cells (IHC 3+, or IHC 2+/FISH⁺), compared to the corresponding anti-HER2 monospecific antibodies or antigen-binding fragments thereof and trastuzumab. In some embodiments, the biparatopic antibody exhibits an internalization rate up to 60% in HER2-low-expressing tumor cells (IHC 2⁺/FISH⁻, or IHC 1+) compared to the corresponding anti-HER2 monospecific antibody or antigen-binding fragments thereof and trastuzumab.

The anti-HER2 biparatopic antibody effectively promotes HER2 degradation in tumor cells. In some embodiments, Western blot analysis demonstrates that the biparatopic antibody induces HER2 degradation in HER2-overexpressing tumor cells (e.g., BT474 cells), whereas the corresponding anti-HER2 monospecific antibodies or antigen-binding fragments thereof and trastuzumab fail to trigger HER2 degradation in the tumor cells. Furthermore, the biparatopic antibody significantly reduces HER2 expression levels on the tumor cell surface, thereby potently inhibiting the proliferation of HER2-overexpressing tumor cells. In some embodiments, the anti-HER2 biparatopic antibody shows a marked inhibitory effect on the proliferation of HER2-overexpressing tumor cells (e.g., BT474 cells), with potency comparable to trastuzumab, whereas the corresponding anti-HER2 monospecific antibodies or antigen-binding fragments thereof cannot inhibit tumor cell proliferation.

The anti-HER2 biparatopic antibody has no effect on HER2-mediated signaling pathways, maintaining the characteristics of the corresponding anti-HER2 monospecific antibodies or antigen-binding fragments thereof that do not interfere with the biological functions of HER2, therefore having no adverse effects on the normal biological functions of HER2 in normal tissues or cells (e.g., cardiomyocytes). In some embodiments, the anti-HER2 biparatopic antibody does not induce, block, or inhibit ligand (e.g., NRG-1)-induced HER2 dimerization and phosphorylation and/or dephosphorylation of AKT in the downstream signaling pathway. Thus, unlike existing HER2-targeting therapeutics (e.g., trastuzumab- and/or pertuzumab-based therapeutics), the anti-HER2 biparatopic antibody exhibits minimal risk of inducing cardiotoxic side effects.

In another aspect, the first and second antigen-binding domains of the anti-HER2 biparatopic antibody of the present invention can be derived from any two anti-HER2 monospecific antibodies or antigen-binding fragments thereof of the present invention that do not compete with each other for binding of HER2, or can also be derived from currently known anti-HER2 antibodies or antigen-binding fragments thereof.

The exemplary anti-HER2 biparatopic antibody of the present invention can be constructed based on the anti-HER2 antibody or antigen-binding fragment thereof that specifically binds to D3 of the HER2 extracellular domain as shown in Table 2 and the anti-HER2 antibody or antigen-binding fragment thereof that specifically binds to D1 of the HER2 extracellular domain as shown in Table 3. The exemplary anti-HER2 biparatopic antibody of the present invention comprises two different antigen-binding domains, wherein the first antigen-binding domain comprises at least one of the CDRs and/or any variable region of the anti-HER2 antibodies or antigen-binding fragments thereof that specifically bind to D3 of the HER2 extracellular domain as shown in Table 2, and the second antigen-binding domain comprises at least one of the CDRs and/or any variable region of the anti-HER2 antibodies or antigen-binding fragments thereof that specifically bind to D1 of the HER2 extracellular domain as shown in Table 3.

In some embodiments, the first or second antigen-binding domain of the anti-HER2 biparatopic antibody comprises heavy chain variable region CDRs and/or light chain variable region CDRs, wherein the heavy chain variable region CDRs of the first antigen-binding domain comprise the amino acid sequence of any one, two or three CDR regions selected from the heavy chain variable region CDRs listed in Table 2, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2 and HCDR3, respectively; the light chain variable region CDRs of the first antigen-binding domain comprise the amino acid sequence of any one, two or three CDR regions selected from the light chain variable region CDRs listed in Table 2, or amino acid sequences having at least 85%, 86%, 87%, 88%, 89%, 90%, 91%, 92%, 93%, 94%, 95%, 96%, 97%, 98%, 99%, or 100% identity to the LCDR1, LCDR2 and LCDR3, respectively; the heavy chain variable region CDRs of the second antigen-binding domain comprise the amino acid sequence of any one, two or three CDR regions selected from the heavy chain variable region CDRs listed in Table 3, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2 and HCDR3, respectively; the light chain variable region CDRs of the second antigen-binding domain comprise the amino acid sequence of any one, two or three CDR regions selected from the light chain variable region CDRs listed in Table 3, or amino acid sequences having at least 85%, 86%, 87%, 88%, 89%, 90%, 91%, 92%, 93%, 94%, 95%, 96%, 97%, 98%, 99%, or 100% identity to the LCDR1, LCDR2 and LCDR3, respectively.

In some specific embodiments, the first antigen-binding domain of the anti-HER2 biparatopic antibody specifically binds to D3 of the HER2 extracellular domain, comprising: HCDR1 as set forth in SEQ ID NO: 11, HCDR2 as set forth in SEQ ID NO: 21, 23, 24, 27, 28, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

In some specific embodiments, the first antigen-binding domain of the anti-HER2 biparatopic antibody specifically binds to D3 of the HER2 extracellular domain, comprising: HCDR1 as set forth in SEQ ID NO: 11, HCDR2 as set forth in SEQ ID NO: 24, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

In some specific embodiments, the second antigen-binding domain of the anti-HER2 biparatopic antibody specifically binds to D1 of the HER2 extracellular domain, comprising: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 130, 131, 132, 133, 134, 135, 136, 137 or 138, and LCDR3 as set forth in SEQ ID NO: 142; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

In some specific embodiments, the second antigen-binding domain of the anti-HER2 biparatopic antibody specifically binds to D1 of the HER2 extracellular domain, comprising: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 133, and LCDR3 as set forth in SEQ ID NO: 142; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

In some embodiments, the first or second antigen-binding domain of the anti-HER2 biparatopic antibody further comprises a heavy chain variable region having the HCDR1, HCDR2 and HCDR3 and/or a light chain variable region having the LCDR1, LCDR2 and LCDR3, wherein the heavy chain variable region of the first antigen-binding domain comprises the amino acid sequence of any one of the VH listed in Table 2, or an amino acid sequence having at least 85%, 86%, 87%, 88%, 89%, 90%, 91%, 92%, 93%, 94%, 95%, 96%, 97%, 98%, 99%, or 100% identity thereto; the light chain variable region of the first antigen-binding domain comprises the amino acid sequence of any one of the VL listed in Table 2, or an amino acid sequence having at least 85%, 86%, 87%, 88%, 89%, 90%, 91%, 92%, 93%, 94%, 95%, 96%, 97%, 98%, 99%, or 100% identity thereto; the heavy chain variable region of the second antigen-binding domain comprises the amino acid sequence of any one of the VH listed in Table 3, or an amino acid sequence having at least 85%, 86%, 87%, 88%, 89%, 90%, 91%, 92%, 93%, 94%, 95%, 96%, 97%, 98%, 99%, or 100% identity thereto; the light chain variable region of the second antigen-binding domain comprises the amino acid sequence of any one of the VL listed in Table 3, or an amino acid sequence having at least 85%, 86%, 87%, 88%, 89%, 90%, 91%, 92%, 93%, 94%, 95%, 96%, 97%, 98%, 99%, or 100% identity thereto.

In some specific embodiments, the first antigen-binding domain of the anti-HER2 biparatopic antibody specifically binds to D3 of the HER2 extracellular domain, comprising: VH as set forth in SEQ ID NO: 70, 72, 73, 76, 77, 78, 79, or 80, and VL as set forth in SEQ ID NO: 92; or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

In a specific embodiment, the first antigen-binding domain of the anti-HER2 biparatopic antibody specifically binds to D3 of the HER2 extracellular domain of HER2, comprising: VH as set forth in SEQ ID NO: 73, 78, 79, or 80, and VL as set forth in SEQ ID NO: 92; or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

In some specific embodiments, the second antigen-binding domain of the anti-HER2 biparatopic antibody specifically binds to D1 of the HER2 extracellular domain, comprising: VH as set forth in SEQ ID NO: 169, and VL as set forth in SEQ ID NO: 189, 190, 191, 192, 193, 194, 195, 196, 197 or 198; or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

In a specific embodiment, the second antigen-binding domain of the anti-HER2 biparatopic antibody specifically binds to D1 of the HER2 extracellular domain of HER2, comprising: VH as set forth in SEQ ID NO: 169, and VL as set forth in SEQ ID NO: 193; or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

In some specific embodiments, the anti-HER2 biparatopic antibody comprises first and second antigen-binding domains, wherein the first antigen-binding domain comprises the following heavy chain variable region CDRs and light chain variable region CDRs, or amino acid sequences having at least 85%, 86%, 87%, 88%, 89%, 90%, 91%, 92%, 93%, 94%, 95%, 96%, 97%, 98%, 99%, or 100% identity to each of the CDRs, respectively: HCDR1 as set forth in SEQ ID NO: 11, HCDR2 as set forth in SEQ ID NO: 21, 23, 24, 27, 28, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; and the second antigen-binding domain comprises the following heavy chain variable region CDRs and light chain variable region CDRs, or amino acid sequences having at least 85%, 86%, 87%, 88%, 89%, 90%, 91%, 92%, 93%, 94%, 95%, 96%, 97%, 98%, 99%, or 100% identity to each of the CDRs, respectively: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 130, 131, 132, 133, 134, 135, 136, 137, or 138, and LCDR3 as set forth in SEQ ID NO: 142.

In some specific embodiments, the anti-HER2 biparatopic antibody comprises first and second antigen-binding domains, wherein the first antigen-binding domain comprises the following heavy chain variable region CDRs and light chain variable region CDRs, or amino acid sequences having at least 85%, 86%, 87%, 88%, 89%, 90%, 91%, 92%, 93%, 94%, 95%, 96%, 97%, 98%, 99%, or 100% identity to each of the CDRs, respectively: HCDR1 as set forth in SEQ ID NO: 11, HCDR2 as set forth in SEQ ID NO: 24, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; and the second antigen-binding domain comprises the following heavy chain variable region CDRs and light chain variable region CDRs, or amino acid sequences having at least 85%, 86%, 87%, 88%, 89%, 90%, 91%, 92%, 93%, 94%, 95%, 96%, 97%, 98%, 99%, or 100% identity to each of the CDRs, respectively: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 133, and LCDR3 as set forth in SEQ ID NO: 142.

In some specific embodiments, the anti-HER2 biparatopic antibody comprises first and second antigen-binding domains. The first antigen-binding domain comprises a heavy chain variable region VH and a light chain variable region VL, wherein the VH and VL comprise the VH as set forth in SEQ ID NO: 70, 72, 73, 76, 77, 78, 79, or 80 and the VL as set forth in SEQ ID NO: 92, or amino acid sequences having at least 85%, 86%, 87%, 88%, 89%, 90%, 91%, 92%, 93%, 94%, 95%, 96%, 97%, 98%, 99%, or 100% identity to the VH and VL, respectively. The second antigen-binding domain comprises a heavy chain variable region VH and a light chain variable region VL, wherein the VH and VL comprise the VH as set forth in SEQ ID NO: 169 and the VL as set forth in SEQ ID NO: 193, or amino acid sequences having at least 85%, 86%, 87%, 88%, 89%, 90%, 91%, 92%, 93%, 94%, 95%, 96%, 97%, 98%, 99%, or 100% identity with the VH and VL, respectively.

In some specific embodiments, the anti-HER2 biparatopic antibody comprises first and second antigen-binding domains. The first antigen-binding domain comprises a heavy chain variable region VH and a light chain variable region VL, wherein the VH and VL comprise the VH as set forth in SEQ ID NO: 73, 78, 79, or 80 and the VL as set forth in SEQ ID NO: 92, or amino acid sequences having at least 85%, 86%, 87%, 88%, 89%, 90%, 91%, 92%, 93%, 94%, 95%, 96%, 97%, 98%, 99%, or 100% identity to the VH and VL, respectively. The second antigen-binding domain comprises a heavy chain variable region VH and a light chain variable region VL, wherein the VH and VL comprise the VH as set forth in SEQ ID NO: 169 and the VL as set forth in SEQ ID NO: 193, or amino acid sequences having at least 85%, 86%, 87%, 88%, 89%, 90%, 91%, 92%, 93%, 94%, 95%, 96%, 97%, 98%, 99%, or 100% identity with the VH and VL, respectively.

The exemplary anti-HER2 biparatopic antibody of the present invention can also be constructed based on the anti-HER2 antibodies or antigen-binding fragments thereof that specifically bind to domains D1 and D4 of the HER2 extracellular domain as described herein. The exemplary anti-HER2 biparatopic antibody of the present invention comprises first and second antigen-binding domains, and the first and second antigen-binding domains are capable of non-competitively binding to HER2 simultaneously. The first antigen-binding domain comprises the VH as set forth in SEQ ID NO: 7 and the VL as set forth in SEQ ID NO: 8; or the first antigen-binding domain comprises the VH as set forth in SEQ ID NO: 9 and the VL as set forth in SEQ ID NO: 10; or the first antigen-binding domain comprises at least one of the CDRs and/or any one variable region of the anti-HER2 antibodies or antigen-binding fragments thereof as shown in Table 3, and can specifically bind to D1 of the HER2 extracellular domain. The second antigen-binding domain comprises the VH as set forth in SEQ ID NO: 5 and the VL as set forth in SEQ ID NO: 6, and can specifically bind to D4 of the HER2 extracellular domain.

In some embodiments, the first or second antigen-binding domain of the anti-HER2 biparatopic antibody comprises heavy chain variable region CDRs and/or light chain variable region CDRs. The heavy chain variable region CDRs of the first antigen-binding domain comprise one, two, or three CDRs from the VH as set forth in SEQ ID NO: 7, or the amino acid sequences as set forth in SEQ ID NOs: 205, 206, and 207, or variants thereof, wherein the variants include humanized antibodies or any other variants; the light chain variable region CDRs of the first antigen-binding domain comprise one, two, or three CDRs from the VL as set forth in SEQ ID NO: 8, or the amino acid sequences as set forth in SEQ ID NOs: 208, 209, and 210, or variants thereof, wherein the variants include humanized antibodies or any other variants. Alternatively, the heavy chain variable region CDRs of the first antigen-binding domain comprise one, two, or three CDRs from the VH as set forth in SEQ ID NO: 9, or the amino acid sequences as set forth in SEQ ID NOs: 211, 212, and 213, or variants thereof, wherein the variants include humanized antibodies or any other variants; the light chain variable region CDRs of the first antigen-binding domain comprise one, two, or three CDRs from the VL as set forth in SEQ ID NO: 10, or the amino acid sequences as set forth in SEQ ID NOs: 214, 215, and 216, or variants thereof, wherein the variants include humanized antibodies or any other variants. Alternatively, the heavy chain variable region CDRs of the first antigen-binding domain comprise the amino acid sequences of any one, two, or three CDR regions from the heavy chain variable region CDRs listed in Table 3, or amino acid sequences having at least 85%, 86%, 87%, 88%, 89%, 90%, 91%, 92%, 93%, 94%, 95%, 96%, 97%, 98%, 99%, or 100% identity thereto; the light chain variable region CDRs of the first antigen-binding domain comprise the amino acid sequences of any one, two, or three CDR regions from the light chain variable region CDRs listed in Table 3, or amino acid sequences having at least 85%, 86%, 87%, 88%, 89%, 90%, 91%, 92%, 93%, 94%, 95%, 96%, 97%, 98%, 99%, or 100% identity thereto. The heavy chain variable region CDRs of the second antigen-binding domain comprise one, two, or three CDRs from the VH as set forth in SEQ ID NO: 5, or the amino acid sequences as set forth in SEQ ID NOs: 199, 200, and 201, or variants thereof, wherein the variants include humanized antibodies or any other variants; the light chain variable region CDRs of the first antigen-binding domain comprise one, two, or three CDRs from the VL as set forth in SEQ ID NO: 6, or the amino acid sequences as set forth in SEQ ID NOs: 202, 203, and 204, or variants thereof, wherein the variants include humanized antibodies or any other variants.

The exemplary anti-HER2 biparatopic antibody of the present invention can also be constructed based on the anti-HER2 antibodies or antigen-binding fragments thereof that specifically bind to domains D3 and D4 of the HER2 extracellular domain as described herein. The exemplary anti-HER2 biparatopic antibody of the present invention comprises first and second antigen-binding domains, and the first and second antigen-binding domains can non-competitively bind to HER2 simultaneously, wherein the first antigen-binding domain comprises at least one of the CDRs and/or any one variable region of the anti-HER2 antibodies or antigen-binding fragments thereof that specifically bind to D3 of the HER2 extracellular domain as shown in Table 2; the second antigen-binding domain comprises the VH as set forth in SEQ ID NO: 5 and the VL as set forth in SEQ ID NO: 6, and can specifically bind to D4 of the HER2 extracellular domain.

In some embodiments, the first or second antigen-binding domain of the anti-HER2 biparatopic antibody comprises heavy chain variable region CDRs and/or light chain variable region CDRs. The heavy chain variable region CDRs of the first antigen-binding domain comprise the amino acid sequences of any one, two, or three CDR regions from the heavy chain variable region CDRs listed in Table 2, or amino acid sequences having at least 85%, 86%, 87%, 88%, 89%, 90%, 91%, 92%, 93%, 94%, 95%, 96%, 97%, 98%, 99%, or 100% identity thereto; the light chain variable region CDRs of the first antigen-binding domain comprise the amino acid sequences of any one, two, or three CDR regions from the light chain variable region CDRs listed in Table 2, or amino acid sequences having at least 85%, 86%, 87%, 88%, 89%, 90%, 91%, 92%, 93%, 94%, 95%, 96%, 97%, 98%, 99%, or 100% identity thereto; the heavy chain variable region CDRs of the second antigen-binding domain comprise one, two, or three CDRs from the VH as set forth in SEQ ID NO: 5, or the amino acid sequences as set forth in SEQ ID NOs: 199, 200, and 201, or variants thereof, wherein the variants include humanized antibodies or any other variants; the light chain variable region CDRs of the first antigen-binding domain comprise one, two, or three CDRs from the VL as set forth in SEQ ID NO: 6, or the amino acid sequences as set forth in SEQ ID NOs: 202, 203, and 204, or variants thereof, wherein the variants include humanized antibodies or any other variants.

In another aspect, the exemplary anti-HER2 biparatopic antibody of the present invention can be constructed in a DVD-Ig format (see US7612181). The first or second antigen-binding domain of the anti-HER2 biparatopic antibody can be in either an Fv form/domain or a Fab/IgG form/domain, i.e. if the first antigen-binding domain is Fv, the second antigen-binding domain is Fab or IgG; if the second antigen-binding domain is Fv, the first antigen-binding domain is Fab or IgG. The Fv domain is fused to or operably linked to the Fab or IgG domain via a linker, wherein the linker not only has low immunogenicity but also ensures the stability of the biparatopic antibody, with a flexible peptide being preferably used as the linker.

As used herein, the term "linker" refers to a moiety that connects two compounds, such as two polypeptide molecules (including but not limited to unmodified or modified amino acids or amino acid sequences). A linker can consist of one or more linking units, or can comprise a linking unit and at least one spacer designed to separate the linking unit and the compound by a specific distance.

The term "operably linked" refers to the connection of amino acid sequences, peptides, or proteins with different functional characteristics, such as the connection of an Fv domain to a Fab or IgG domain via a linker as described herein.

In some embodiments, the first antigen-binding domain of the anti-HER2 biparatopic antibody specifically binds to D3 of the HER2 extracellular domain and is an Fv domain, comprising the heavy chain variable region CDRs and the light chain variable region CDRs, and/or heavy chain variable region VH and light chain variable region VL of the anti-HER2 antibody or antigen-binding fragment thereof shown in Table 2. The second antigen-binding domain of the anti-HER2 biparatopic antibody specifically binds to D1 of the HER2 extracellular domain and is a Fab domain or IgG domain, comprising the heavy chain variable region CDRs and the light chain variable region CDRs, and/or heavy chain variable region VH and light chain variable region VL of the anti-HER2 antibody or antigen-binding fragment thereof shown in Table 3. The C-terminus of the VH domain in the Fv domain is fused to or operably linked to the N-terminus of the VH domain in the Fab or IgG domain via a linker sequence, and the C-terminus of the VL domain in the Fv domain is fused to or operably linked to the N-terminus of the VL domain in the Fab or IgG domain via a linker sequence.

In some specific embodiments, the linker comprises different copy numbers of the GGGGS sequence (G₄S), such as 1, 2, 3, 4, or 5 copies. The copy number of the G₄S connecting the VH domain in the Fv domain of the anti-HER2 biparatopic antibody to the VH domain in the Fab or IgG domain of the bipratopic antibody can be the same as or different from that of the G₄S connecting the VL domain in the Fv domain of the biparatopic antibody to the VL domain in the Fab or IgG domain of the biparatopic antibody. Preferably, the copy number of the G₄S connecting the VH domains between the Fv domain and the Fab or IgG domain is different from that of the G₄S connecting the VL domains between the two, wherein the copy number of the linker connecting the two VH domains is preferably 1, and the copy number of the linker connecting the two VL domains is preferably 3, which can effectively ensure the structural stability of the anti-HER2 biparatopic antibody of the present invention and having enhanced internalization in HER2-expressing tumor cells.

In a specific embodiment, the anti-HER2 biparatopic antibody comprises first and second antigen-binding domains, wherein the first antigen-binding domain comprises the VH and VL of the anti-HER2 antibody or antigen-binding fragment thereof shown in Table 2, and the second antigen-binding domain comprises the VH and VL of the anti-HER2 antibody or antigen-binding fragment thereof shown in Table 3. The first antigen-binding domain is an Fv domain, and the second antigen-binding domain is a Fab or IgG domain. The C-terminus of the VH domain in the Fv domain is connected to the N-terminus of the VH domain in the Fab or IgG domain via a linker (e.g., (G₄S)ₙ, wherein n is an integer > 0, such as 1-3; preferably (G₄S)₁ to the N-terminus of the VH domain in the Fab or IgG domain). The C-terminus of the VL domain in the Fv domain is connected to the N-terminus of the VL domain in the Fab or IgG domain via a linker (e.g., (G₄S)ₙ, wherein n is an integer of 1-3; preferably (G₄S)₃ to the N-terminus of the VL domain in the Fab or IgG domain).

In another aspect, the anti-HER2 biparatopic antibody of the present invention can further comprise a constant region, including a heavy chain constant region and a light chain constant region of an antibody. The heavy chain constant region of the present invention includes native and mutant forms of the Fc region of human IgG heavy chain constant region, and also includes truncated polypeptide forms containing a hinge region that facilitate dimer formation. In some embodiments, the Fc region comprises CH2 and CH3 domains of an antibody. A fusion protein containing a Fc moiety (and an oligomer formed therefrom) offers the advantages of facile purification by Protein A or Protein G affinity chromatography, as well as extended serum half-life. Preferably, the Fc region is derived from human IgGs, including IgG1, IgG2, IgG3, and IgG4. Herein, the positions of specific amino acid residues in the Fc region are determined according to the EU numbering system.

One function of the Fc region of an antibody is to produce "effector functions" with the immune system when the antibody binds to its target, including antibody-dependent cellular cytotoxicity (ADCC), antibody-dependent cellular phagocytosis (ADCP), and/or complement-dependent cytotoxicity (CDC). ADCC and ADCP are mediated by the binding of Fc to Fc receptors (FcRs) on the surface of immune cells (Raghavan et al., Annu Rev Cell Dev Biol 1996, 12:181-220; Ghetie et al., Annu Rev Immunol 2000, 18:739-766; Ravetch et al., Annu Rev Immunol 2001, 19:275-290), wherein the immune cells include monocytes, macrophages, neutrophils, dendritic cells, eosinophils, mast cells, platelets, B cells, large granular lymphocytes, Langerhans cells, NK cells, and T cells. CDC is mediated by the binding of Fc to proteins of the complement system such as C1q (Ward et al., Ther Immunol 1995, 2:77-94).

In some embodiments, the anti-HER2 biparatopic antibody of the present invention comprises an engineered IgG Fc region to reduc Fc-mediated effector functions. An exemplary antibody with reduced effector functions comprises Fc regions containing the following amino acid mutations:
N297A or N297Q (IgG1)
S267E/L328F (IgG1)
L234A/L235A (IgG1)
L234F/L235E/P331S (IgG1)
C220S/C226S/C229S/P238S (IgG1)
C226S/C229S/E233P/L234V/L235A (IgG1)
V234A/G237A (IgG2)
H268Q/V309L1A330S/A331S (IgG2)
L235A/G237A/E318A (IgG4)

Preferably, the engineered Fc region is the human IgG1 Fc with amino acid substitutions L234F/L235E/P331S (EU numbering system), which can reduce the binding of the Fc region to one or more FcyRs and C1q (Oganesyan et al., Acta Crystallogr D Biol Crystallogr 2008, 64:700-704; US5624821; US6194551). The FcyR family includes FcγRI (also known as CD64), including the isoforms FcγRIa, FcγRIb, and FcyRIc; FcγRII (also known as CD32), including isoforms FcγRIIa, FcγRIIb, and FcyRIIc; and FcγRIII (also known as CD16), including isoforms FcγRIIIa and FcγRIIIb (Jefferis et al., Immunol Lett 2002, 82:57-65). Among the FcyRs, FcyRI, FcyRIIa, FcyRIIc, and FcγRIIIa can induce ADCC, endocytosis, phagocytosis, and/or cytokine release. Binding properties include, but are not limited to, binding specificity, binding affinity constant (K_{D}), and dissociation and association rates (k_{dis} and kₐ, respectively). A person skilled in the art can analyze whether the engineered Fc region has altered ADCC and/or CDC activity based on any one or more of these binding properties. In some embodiments, the heavy chain constant region and light chain constant region in the anti-HER2 biparatopic antibody of the present invention can be derived from the heavy chain constant region of human IgG1 or IgG4 (either wild-type or mutated, such as the wild-type human IgG1 constant region as set forth in SEQ ID NO: 231), and the human Kappa (κ) or Lambda (λ) constant region.

In some specific embodiments, the heavy chain constant region of the anti-HER2 biparatopic antibody comprising a human IgG1 Fc with the amino acid substitutions L234F/L235E/P331S (EU numbering system, as set forth in SEQ ID NO: 232) has reduced binding affinity to one or more FcyRs (e.g., FcγRI, FcγRIIa, FcγRIIb, FcyRIIIa, and FcγRIIIa) and C1q. In some embodiments, introduction of the L234F/L235E/P331S mutations into the heavy chain constant region of the anti-HER2 biparatopic antibody results in loss of binding to FcyRI, FcγRIIa (167H), FcyRIIb, FcγRIIIa (176V), and/or FcγRIIIa (176F). In some embodiments, introduction of the L234F/L235E/P331S mutations into the heavy chain constant region of the anti-HER2 biparatopic antibody results in loss of binding to C1q. In some embodiments, introduction of the L234F/L235E/P331S mutations into the heavy chain constant region of the anti-HER2 biparatopic antibody does not affect the binding affinity of the biparatopic antibody to FcRn. In some embodiments, introducing the L234F/L235E/P331S mutations into the heavy chain constant region of the anti-HER2 biparatopic antibody can significantly reduce the ADCC activity of the biparatopic antibody. In one embodiment, the biparatopic antibody has a significantly attenuated or undetectable ADCC activity as compared to the antibody without the amino acid mutations in its Fc region. Such attenuation or elimination of the ADCC activity is likely caused by the significant reduction in binding affinity of the biparatopic antibody to FcyR.

In some specific embodiments, the anti-HER2 biparatopic antibody comprises a light chain constant region, wherein the light chain constant region is selected from the Kappa constant region (as set forth in SEQ ID NO: 233).

In one aspect, the anti-HER2 biparatopic antibody of the present invention comprises four polypeptide chains, wherein two polypeptide chains comprise VH1-L1-VH2-C-(Fc)n, in which VH1 represents the heavy chain variable region of the first antigen-binding domain specifically binding to D3 of the HER2 extracellular domain, L1 represents a linker, VH2 represents the heavy chain variable region of the second antigen-binding domain specifically binding to D1 of the HER2 extracellular domain, C represents the heavy chain constant region CH1, Fc represents the heavy chain constant region Fc domain, and n is 0 or 1; and the other two polypeptide chains comprise VL1-L2-VL2-CL, in which VL1 represents the light chain variable region of the first antigen-binding domain specifically binding to D3 of the HER2 extracellular domain, L2 represents a linker, VL2 represents the light chain variable region of the second antigen-binding domain specifically binding to D1 of the HER2 extracellular domain, and CL is the IgG light chain constant region.

In some embodiments, the VH1 comprises HCDR1 having an amino acid sequence as set forth in SEQ ID NO: 11, HCDR2 having an amino acid sequence as set forth in SEQ ID NO: 24, 29, 30, or 31, and HCDR3 having an amino acid sequence as set forth in SEQ ID NO: 37, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, and HCDR3, respectively; L1 comprises a linker having an amino acid sequence as set forth in SEQ ID NO: 229; VH2 comprises HCDR1 having an amino acid sequence as set forth in SEQ ID NO: 98, HCDR2 having an amino acid sequence as set forth in SEQ ID NO: 102, and HCDR3 having an amino acid sequence as set forth in SEQ ID NO: 110, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, and HCDR3, respectively; Fc comprises an engineered Fc domain, including a human IgG1 Fc with the amino acid substitutions L234F/L235E/P331S (EU numbering system) having an amino acid sequence as set forth in SEQ ID NO: 232.

In some specific embodiments, the VH1 comprises VH having an amino acid sequence as set forth in SEQ ID NO: 73, 78, 79, or 80, or an amino acid sequence having at least 85%, 86%, 87%, 88%, 89%, 90%, 91%, 92%, 93%, 94%, 95%, 96%, 97%, 98%, 99%, or 100% identity thereto; the VH2 comprises VH having an amino acid sequence as set forth in SEQ ID NO: 169, or an amino acid sequence having at least 85%, 86%, 87%, 88%, 89%, 90%, 91%, 92%, 93%, 94%, 95%, 96%, 97%, 98%, 99%, or 100% identity thereto.

In some embodiments, the VL1 comprises LCDR1 having an amino acid sequence as set forth in SEQ ID NO: 42, LCDR2 having an amino acid sequence as set forth in SEQ ID NO: 46, and LCDR3 having an amino acid sequence as set forth in SEQ ID NO: 49, or amino acid sequences having at least 85%, 86%, 87%, 88%, 89%, 90%, 91%, 92%, 93%, 94%, 95%, 96%, 97%, 98%, 99%, or 100% identity to the LCDR1, LCDR2, and LCDR3, respectively; L2 comprises a linker having an amino acid sequence as set forth in SEQ ID NO: 230; VL2 comprises LCDR1 having an amino acid sequence as set forth in SEQ ID NO: 115, LCDR2 having an amino acid sequence as set forth in SEQ ID NO: 133, and LCDR3 having an amino acid sequence as set forth in SEQ ID NO: 142, or amino acid sequences having at least 85%, 86%, 87%, 88%, 89%, 90%, 91%, 92%, 93%, 94%, 95%, 96%, 97%, 98%, 99%, or 100% identity to the LCDR1, LCDR2, and LCDR3, respectively; CL is selected from human κ constant region or human λ constant region, preferably human κ constant region (the amino acid sequence as set forth in SEQ ID NO: 233).

In some specific embodiments, the VL1 comprises VL having an amino acid sequence as set forth in SEQ ID NO: 92, or an amino acid sequence having at least 85%, 86%, 87%, 88%, 89%, 90%, 91%, 92%, 93%, 94%, 95%, 96%, 97%, 98%, 99%, or 100% identity thereto; the VL2 comprises VL having an amino acid sequence as set forth in SEQ ID NO: 193, or an amino acid sequence having at least 85%, 86%, 87%, 88%, 89%, 90%, 91%, 92%, 93%, 94%, 95%, 96%, 97%, 98%, 99%, or 100% identity thereto.

The anti-HER2 biparatopic antibody of the present invention can specifically and simultaneously bind to two different epitopes on the HER2 extracellular domain, which can effectively cross-link HER2 on the cell surface to form antigen-antibody cross-linked multimers or clusters, thereby inducing endocytosis and promoting trafficking of internalized clusters to lysosomes for degradation. As a result, the biparatopic antibody can significantly downregulate the HER2 expression on cell surface, reduce HER2 dimerization, and thus inhibit the proliferation of HER2-overexpressing tumor cells. Moreover, the anti-HER2 biparatopic antibody of the present invention does not interfere with the HER2-mediated signaling pathways, and thus does not disturb the normal biological functions of HER2 in normal tissues/cells.

### 3. Anti-HER2 Biparatopic Antibody-Drug Conjugate (ADC)

The present invention further provides an anti-HER2 biparatopic ADC, wherein the biparatopic ADC comprises a small molecule toxin compound conjugated to the anti-HER2 biparatopic antibody of the present invention via a cleavable linker, possessing one or more of the following functional characteristics:
(i) The anti-HER2 biparatopic ADC is a tetravalent molecule capable of specifically binding to two non-overlapping epitopes of HER2 on the surface of tumor cells, thus effectively cross-linking HER2 and forming cross-linked multimers or clusters on the tumor cell membrane. In one aspect, the formation of clusters promotes rapid and efficient endocytosis of the ADC by the cell; furthermore, the intracellular trafficking pathway following endocytosis is altered from recycling to lysosomal trafficking, significantly enhancing the efficiency of ADC being transported into lysosomes. Following degradation in lysosomes, more small-molecule toxin compounds can be released into the cytoplasm. Therefore, the biparatopic ADC exhibits stronger targeted cytotoxicity, demonstrating superior killing activity in HER2-overexpressing tumor cells (IHC 3+ or IHC 2⁺/FISH⁺) compared to existing HER2-targeted therapies (e.g., trastuzumab, pertuzumab, T-DM1, DS-8201), and exerting direct killing effects in HER2-low-expressing tumor cells (IHC 2+/FISH⁻ or IHC 1+), highlighting its potential for expanding the indications of HER2-targeting therapies. In another aspect, the released small molecule toxin compounds are hydrophobic and cell membrane-permeable, allowing them to passively diffuse into the tumor microenvironment and exert a bystander effect, i.e., killing adjacent lower- or non-HER2-expressing tumor cells. In summary, compared to conventional ADCs, the biparatopic ADC demonstrates broader spectrum of tumor cell killing activity and a reduced likelihood of developing drug resistance.
(ii) The anti-HER2 biparatopic ADC exhibits cytotoxic activity in tumors resistant to or relapsed from the treatment of trastuzumab, pertuzumab, T-DM1, or DS-8201. Consequently, it has the potential to address the drug resistance issues associated with existing HER2-targeting therapies.
(iii) The anti-HER2 biparatopic ADC does not interfere with HER2 dimerization and the regulation of HER2-mediated downstream signaling pathways. As a result, it does not disrupt the normal biological functions of HER2 in cardiomyocytes, therefore significantly reducing the risk of cardiotoxic side effects.
(iv) The anti-HER2 biparatopic ADC exhibits significantly reduced Fc receptor binding affinity, therefore it has very low safety risks associated with the Fc receptors.

The ADC described herein can be represented by Formula (I):

Ab-(L-D)p (I)

wherein Ab represents the anti-HER2 biparatopic antibody of the present invention;
D represents a small-molecule toxin compound (Drug);
L represents a cleavable linker that conjugates Ab to D; and
p represents the copy number of (L-D) conjugated to Ab, ranging from 2 to 8.

Ab of the ADC described herein is the anti-HER2 biparatopic antibody of the present invention, comprising a first and a second antigen-binding domain, wherein the first antigen-binding domain comprises the VH and VL of the anti-HER2 antibody or antigen-binding fragment thereof as shown in Table 2, which specifically binds to D3 of the HER2 extracellular region and is an Fv domain; the second antigen-binding domain comprises the VH and VL of the anti-HER2 antibody or antigen-binding fragment thereof as shown in Table 3, which specifically binds to D1 of the HER2 extracellular region and is a Fab domain or an IgG domain.

In some embodiments, the first antigen-binding domain of the anti-HER2 biparatopic ADC comprises a heavy chain variable region VH and a light chain variable region VL, wherein the VH and VL comprise: (i) HCDR1 as set forth in SEQ ID NO: 11; (ii) HCDR2 as set forth in SEQ ID NO: 24, 29, 30, or 31; (iii) HCDR3 as set forth in SEQ ID NO: 37; (iv) LCDR1 as set forth in SEQ ID NO: 42; (v) LCDR2 as set forth in SEQ ID NO: 46; and (vi) LCDR3 as set forth in SEQ ID NO: 49; or amino acid sequences having at least 85%, 86%, 87%, 88%, 89%, 90%, 91%, 92%, 93%, 94%, 95%, 96%, 97%, 98%, 99%, or 100% sequence identity to the HCDR1, HCDR2, HCDR3, LCDR1, LCDR2 and LCDR3, respectively.

In some embodiments, the second antigen-binding domain of the anti-HER2 biparatopic ADC comprises a heavy chain variable region VH and a light chain variable region VL, wherein the VH and VL comprise: (i) HCDR1 as set forth in SEQ ID NO: 98; (ii) HCDR2 as set forth in SEQ ID NO: 102; (iii) HCDR3 as set forth in SEQ ID NO: 110; (iv) LCDR1 as set forth in SEQ ID NO: 115; (v) LCDR2 as set forth in SEQ ID NO: 133; and (vi) LCDR3 as set forth in SEQ ID NO: 142; or amino acid sequences having at least 85%, 86%, 87%, 88%, 89%, 90%, 91%, 92%, 93%, 94%, 95%, 96%, 97%, 98%, 99%, or 100% sequence identity to the HCDR1, HCDR2, HCDR3, LCDR1, LCDR2 and LCDR3, respectively.

In some embodiments, the first antigen-binding domain of the anti-HER2 biparatopic ADC comprises: a VH as set forth in SEQ ID NO: 73, 78, 79, or 80, or an amino acid sequence having at least 85%, 86%, 87%, 88%, 89%, 90%, 91%, 92%, 93%, 94%, 95%, 96%, 97%, 98%, 99%, or 100% identity thereto; and/or a VL as set forth in SEQ ID NO: 92, or an amino acid sequence having at least 85%, 86%, 87%, 88%, 89%, 90%, 91%, 92%, 93%, 94%, 95%, 96%, 97%, 98%, 99%, or 100% identity thereto.

In some embodiments, the second antigen-binding domain of the anti-HER2 biparatopic ADC comprises: a VH as set forth in SEQ ID NO: 169, or an amino acid sequence having at least 85%, 86%, 87%, 88%, 89%, 90%, 91%, 92%, 93%, 94%, 95%, 96%, 97%, 98%, 99%, or 100% identity thereto; and/or a VL as set forth in SEQ ID NO: 193, or an amino acid sequence having at least 85%, 86%, 87%, 88%, 89%, 90%, 91%, 92%, 93%, 94%, 95%, 96%, 97%, 98%, 99%, or 100% identity thereto.

The D of the ADC is a small-molecule toxin compound that exhibits cytotoxicity or cell growth inhibitory effects on both tumor cells and normal cells when not conjugated to an antibody. However, when the small-molecule toxin compound is conjugated to the anti-HER2 biparatopic antibody of the present invention to form the ADC, it exerts its cytotoxicity and/or bystander killing effect only after the ADC is internalized and transported into the lysosomes of HER2-expressing target cells and released from the ADC through enzymatic action, hydrolysis, oxidation, or any other mechanism. The small-molecule toxin compound includes cytotoxins and chemotherapeutic agents.

In some embodiments, the cytotoxins include tubulin inhibitors and DNA-alkylating agents. The tubulin inhibitors include eribulin, auristatin derivatives (e.g., MMAE, MMAF, MMAD), tubulysins, cryptomycins, and maytansinoid derivatives (e.g., DM1, DM2, DM3, DM4). The DNA-alkylating agents include topoisomerase inhibitors (e.g., camptothecin derivatives such as SN-38, exatecan, and DXd), pyrrolobenzodiazepines (PBDs), calicheamicin and derivatives thereof (e.g., N-acetyl calicheamicin [CMC]), and duocarmycins. A preferred small-molecule toxin compound is eribulin.

The molecular structure of eribulin is represented by Formula (II):

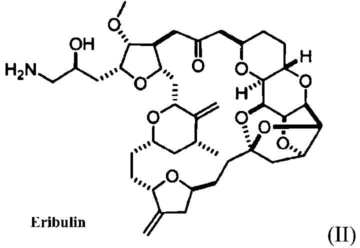

The term "eribulin" refers to a synthetic analogue of halichondrin B that is a macrocyclic compound isolated from the marine sponge *Halichondria okadai.* As a microtubule dynamics inhibitor, eribulin binds to tubulin, thereby inhibiting the formation of mitotic spindles and leanding to cell cycle division arrest at the G2/M phase. Exemplary structures of eribulin or analogues thereof, as well as methods for their synthesis, are described in WO1999065894, WO2004034990, ZL201910197071.8, and/or ZL201910509222.9, the disclosures of which are incorporated herein by reference.

In some embodiments, the small-molecule toxin compound is eribulin, which is characterized by a wide clinical therapeutic window and low off-target cytotoxicity.

In some embodiments, the chemotherapeutic agents can be natural or synthetic compounds, including but not limited to alkylating chemotherapeutic agents and other compounds with alkylating activity (e.g., nitrogen mustards, ethylenimine compounds, alkyl sulfonates, nitrosoureas, cisplatin, dacarbazine), antimetabolites (e.g., folate, purine, or pyrimidine antagonists), mitotic inhibitors (e.g., vinca alkaloids and derivatives of podophyllotoxin), and cytotoxic antibiotics (e.g., anthracycline antibiotics including daunorubicin and doxorubicin, as well as actinomycins and bleomycins).

The cleavable linker is stable outside HER2-expressing tumor cells, enabling the ADC of the present invention to maintain structural stability *in vitro* or in the blood circulation without causing systemic toxicity due to non-targeted or random release of the small-molecule toxin compound (off-target effects). Meanwhile, after being endocytosed into HER2-expressing tumor cells and trafficked to the lysosomes, the linker is rapidly cleaved and the small-molecule toxin compound is released to kill the tumor cells. The cleavable linker refers to any linker containing a cleavable moiety. As used herein, the term "cleavable moiety" refers to any cleavable chemical bond, for example, as well known in the art, including but are not limited to acid-labile bonds, protease/peptidase-labile bonds, photolabile bonds, esterase-labile bonds, and disulfide bonds. A linker containing a cleavable moiety allows the small-molecule toxin compound to be released from the ADC via cleavage at a specific site within the linker.

In some embodiments, the cleavable linker comprises a cleavable peptide moiety, after it is cleaved by intracellular proteases (e.g., endosomal proteases, lysosomal proteases, or tumor-associated proteases), the small-molecule toxin compound is released from the ADC to kill tumor cells. In some embodiments, the cleavable peptide moiety comprises amino acid units that can be cleaved by lysosomal cysteine cathepsins (e.g., cathepsin B, C, F, H, K, L, O, S, V, X, or W). The amino acid units can include naturally occurring amino acid residues and/or non-natural amino acid analogs, such as citrulline (abbreviated as Cit). In some embodiments, the amino acid units include dipeptides, tripeptides, or tetrapeptides, for example, having an amino acid sequence such as Phe-Lys, Val-Cit (VC), Glu-Val-Cit, or Gly-Gly-Phe-Gly (GGFG), which can be cleaved by cathepsin B. Preferred amino acid units include VC and GGFG.

In some embodiments, the cleavable linker can comprise at least one spacer that connects the small-molecule toxin compound (D) to the anti-HER2 biparatopic antibody (Ab) of the present invention, wherein the spacer includes a spacer connected to the antibody and/or a second spacer connected to the small-molecule compound.

In some embodiments, the spacer connected to the antibody can be hydrophilic to enhance the hydrophilicity of the ADC, thereby improving its stability, reducing aggregation of ADC products, and decreasing immunogenicity. Exemplary spacer comprises one or more polyethylene glycol (PEG), such as 1, 2, 3, 4, 5, or 6 PEG, with 2 PEG being preferred. In some examples, the spacer is attached to the antibody via maleimide (Mal), wherein the spacer attached to the antibody via Mal can also be referred to herein as a "Mal-spacer". In some examples, the Mal-spacer comprises one or more PEG moieties (e.g., 2 PEG).

In some embodiments, the second spacer connected to the small-molecule compound is used to attach the cleavable moiety (e.g., a cleavable peptide) of the cleavable linker to the small-molecule compound. In some examples, the second spacer connected to the small-molecule compound exhibits self-immolation, which facilitates the complete release of the small-molecule compound in target cells, i.e., the small-molecule compound released in target cells dose not carry any residual spacer moiety or other modifying groups attached to it, ensuring that the anti-tumor activity of the released small-molecule compound is not affected by the presence of modifying groups. In some examples, the self-immolative spacer comprises a p-aminobenzyl unit, where p-aminobenzyl alcohol (PABOH) is attached to the cleavable moiety of the cleavable linker (e.g., an amino acid unit of the cleavable peptide) via an amide bond, and a carbamate, methylcarbamate, or carbonate group is formed between PABOH and the small-molecule compound (Hamann et al., Expert Opinion on Therapeutic Patents 2005, 15:1087-1103). Furthermore, the self-immolative spacer comprises a p-aminobenzyl carbonyl (PAB) as shown in Formula (III), and the self-immolation of PAB involves a spontaneous 1,6-elimination reaction (Jain et al., Pharmaceutical Research 2015, 32:3526-3540):

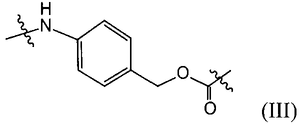

In some embodiments, the cleavable linker comprises a Mal-spacer and a cleavable peptide moiety. In some embodiments, the spacer comprises a PEG moiety (e.g., 2 PEG), and the cleavable peptide moiety comprises an amino acid unit (e.g., the dipeptide VC or the tetrapeptide GGFG). In some embodiments, the cleavable linker comprises a covalently linked Mal-spacer-amino acid unit, wherein the amino acid unit is Phe-Lys, VC, Glu-Val-Cit, or GGFG. In some embodiments, the cleavable linker comprises a covalently linked Mal-spacer-amino acid unit-PAB, wherein the amino acid unit is Phe-Lys, VC, Glu-Val-Cit, or GGFG. In some examples, the spacer is (PEG)ₘ, wherein m is an integer from 1 to 5, preferably 2. In some examples, the cleavable linker comprises Mal-(PEG)₂ and VC. In some examples, the cleavable linker comprises Mal-(PEG)₂ and GGFG.

In some embodiments, the cleavable linker comprises the structure: Mal-spacer-cleavable peptide moiety. In some examples, the cleavable linker comprises the structures: Mal-(PEG)₂-VC and Mal-(PEG)₂-GGFG.

In some embodiments, the cleavable linker comprises a Mal-spacer, a cleavable peptide moiety, and a second spacer. In some examples, the spacer comprises a PEG moiety (e.g., 2 PEG), the cleavable peptide moiety comprises an amino acid unit (e.g., the dipeptide VC), and the second spacer is self-immolative (e.g., PAB). In some examples, the cleavable linker comprises Mal-(PEG)₂, VC, and PAB.

In some embodiments, the cleavable linker comprises the structure: Mal-spacer-cleavable peptide moiety-the second spacer. In some examples, the cleavable linker comprises the structure: Mal-(PEG)₂-VC-PAB.

In some embodiments, the Mal-spacer of the cleavable linker is conjugated to one or more amino acid residues of the antibody component in the ADC of the present invention. For example, the Mal-spacer can be conjugated to the antibody via thiol groups, including conjugation to the sulfhydryl groups of one or more cysteines of the antibody. In some embodiments, the maleimide group of the Mal-spacer reacts with the sulfhydryl group of a cysteine residue of the antibody, thereby conjugating the Mal-spacer to the antibody. In some embodiments, the maleimide group of the Mal-spacer can react with the sulfhydryl groups of cysteine residues at specific positions in the constant region and/or variable region of the antibody. Furthermore, the maleimide group of the Mal-spacer can be conjugated to free cysteine residues released upon reduction of disulfide bonds in the antibody hinge region and/or interchain disulfide bonds. In some examples, the maleimide group of the Mal-spacer is conjugated to the cysteine residues in the antibody hinge region. Multiple free cysteine residues can be generated by subjecting the interchain disulfide bonds in the antibody hinge region to chemical reactions (e.g., reduction, pH adjustment, or hydrolysis). Alternatively, engineered cysteine residues can be generated by engineering one or more amino acid residues at specific sites in the antibody constant region using DNA recombination technology (e.g., by substituting or inserting cysteine residues).

In some embodiments, the second spacer of the cleavable linker is conjugated to the small-molecule compound, wherein the small-molecule compound is eribulin or a derivative thereof. In some embodiments, the second spacer conjugates the cleavable peptide moiety in the cleavable linker to the C-35 amine of eribulin, wherein the second spacer is self-immolative (e.g., PAB), and the cleavable peptide moiety comprises VC. The self-immolative spacer spontaneously undergoes self-immolation after enzymatic cleavage of the cleavable peptide moiety, resulting in the release of eribulin from the ADC in its naturally active form. In some examples, the self-immolative spacer is PAB, which conjugates the cleavable peptide moiety VC to the C-35 amine of eribulin to construct the ADC with the structure of Mal-(PEG)₂-VC-PAB-Eribulin.

In some embodiments, the cleavable moiety (e.g., a cleavable polypeptide) of the cleavable linker can be directly conjugated to the small-molecule toxin compound component of the ADC, wherein the small-molecule toxin compound is eribulin or a derivative thereof. In some embodiments, the cleavable polypeptide comprises an amino acid unit, wherein the amino acid unit comprises GGFG and this tetrapeptide is conjugated to the C-35 amine of eribulin via its carboxyl group, thereby constructing the ADC with the structure of Mal-(PEG)₂-GGFG-Eribulin. The approach of directly conjugating the GGFG tetrapeptide in the cleavable linker to eribulin can also ensure that, after the cleavable linker of the ADC is cleaved in target cells, eribulin can be released in its naturally active form, allowing eribulin to exert the target tumor cell-killing effect and/or the bystander effect.

In some embodiments, the ADC structure of the present invention comprises the anti-HER2 biparatopic antibody of the present invention conjugated to eribulin or a derivative thereof via a cleavable linker, wherein the cleavable linker comprises a Mal-spacer, a cleavable peptide moiety and/or a second spacer. In some embodiments, the ADC structure comprises the anti-HER2 biparatopic antibody of the present invention conjugated to the C-35 amine of eribulin via a cleavable linker, wherein the cleavable linker includes Mal-(PEG)₂-VC-PAB or Mal-(PEG)₂-GGFG. The free cysteine residues released upon the reduction of disulfide bonds in the antibody hinge region and/or interchain disulfide bonds are conjugated to the Mal group of the cleavable linker.

In some embodiments, the ADC of the present invention comprises the following components: 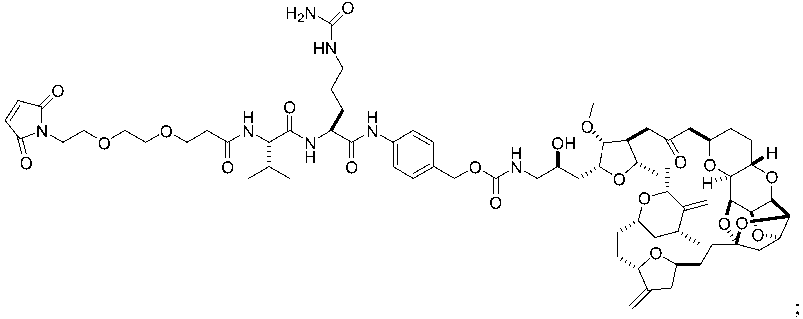 or

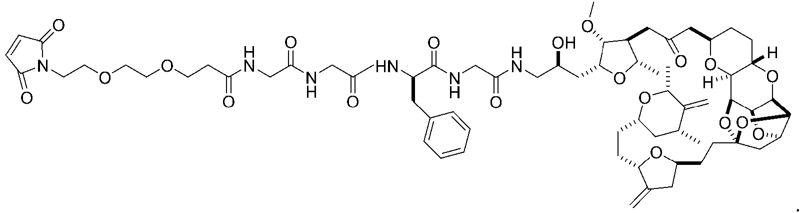

In some embodiments, the ADC of the present invention comprises the anti-HER2 biparatopic antibody of the present invention conjugated to a drug payload, wherein the drug payload includes Mal-(PEG)₂-VC-PAB-Eribulin and Mal-(PEG)₂-GGFG-Eribulin. In some embodiments, the ADC comprises the anti-HER2 biparatopic antibody of the present invention conjugated via its cysteine residues to the Mal group of the drug payload. More specifically, the free cysteine residues released upon the reduction of disulfide bonds in the hinge region and/or interchain disulfide bonds of the biparatopic antibody are conjugated to the Mal group of the drug payload.

In some embodiments, the present invention provides an ADC having the following formula: 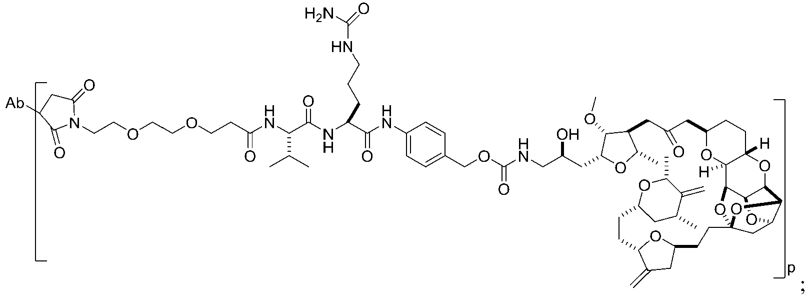 or

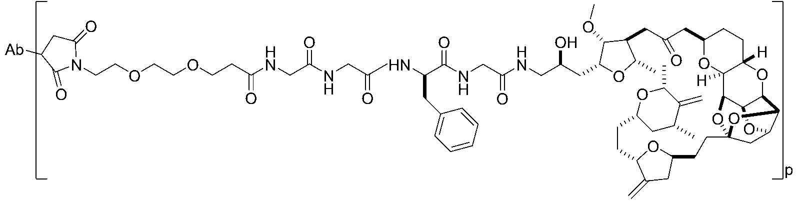

Wherein Ab represents the anti-HER2 biparatopic antibody of the present invention, and p is from 2 to 8.

In formula (I), p is also referred to herein as the Drug-to-Antibody Ratio (DAR) or the number of payload molecules, i.e., the number of small-molecule compounds conjugated to the antibody, wherein p ranges from 2 to 8, for example, from 4 to 8. The DAR value of the ADC of the present invention wherein the anti-HER2 biparatopic antibody is conjugated with eribulin via a cleavable linker exhibits desirable properties, i.e., the ADC demonstrates not only sufficient killing activity but also adequate stability. In general, a higher DAR value (e.g., p > 8) may lead to aggregation and precipitation of the ADC due to the strong hydrophobicity of the small-molecule toxin compound which may promote hydrophobic interactions in aqueous environment, potentially even raising safety risks *in vivo*; a lower DAR value (e.g., p < 2) may result in reduced killing activity of the ADC against tumor cells, thereby affecting the therapeutic efficacy. In some embodiments, the optimal DAR value is approximately 4 to 8. The average DAR value (i.e., the average number of payload molecules or average p) of the anti-HER2 biparatopic ADC of the present invention can be calculated using the data derived from conventional analysis known in the art (e.g., reversed-phase LC-MS mass spectrometry and/or HIC-HPLC).

### 4. Methods for Preparing Antibodies of the Present Invention

### 4.1 Polynucleotide, vector and host cell

In one aspect, the present invention provides a nucleic acid encoding an anti-HER2 antibody or antigen-binding fragment thereof, or an anti-HER2 biparatopic antibody. The present invention also includes a polynucleotide variant encoding the amino acid sequence described herein.

Nucleotide sequences corresponding to the amino acid sequences described in the present invention, probes or primers for use of nucleic acid isolation, or queryable sequences in the databases can be obtained by back-translation of the amino acid sequences.

Polymerase chain reaction (PCR) procedures can be used to isolate and amplify DNA sequences encoding the anti-HER2 antibody or antigen-binding fragment thereof, or the anti-HER2 biparatopic antibody of the present invention. Oligonucleotides that define the desired ends of the DNA fragment assembly are used as 5' and 3' primers. The oligonucleotides may additionally contain recognition sites for restriction endonucleases to facilitate insertion of the amplified DNA fragments in combination into an expression vector. PCR techniques are described in, for example, Saiki et al., Science 1988, 239:487-491; Wu et al., (eds), Recombinant DNA Methodology, 1989, Academic Press, pp.189-196; Innis et al., (eds) PCR Protocols: A Guide to Methods and Applications, 1990, Academic Press.

The nucleic acids of the present invention include both single- and double-stranded forms of DNA and RNA, and their corresponding complementary sequences, including isolated nucleic acids, preferably DNA or RNA derived from at least one isolation in substantially pure form and in a quantity or concentration sufficient to allow identification, manipulation, and recovery of their component nucleotide sequences using standard biochemical methods (e.g., as described in Sambrook et al., Molecular Cloning: A Laboratory Manual, 2nd Edition, Cold Spring Harbor Laboratory). Preferably, such sequences include those that are provided and/or constructed in the form of internal untranslated sequences or open reading frames interrupted by introns that are not commonly present in eukaryotic genes. Sequences of untranslated DNA may be present at 5' or 3' end of the open reading frames, wherein the sequences do not interfere with manipulation or expression of the coding regions.

The anti-HER2 antibody or antigen-binding fragment thereof, or the anti-HER2 biparatopic antibody of the present invention can be prepared by the following steps: performing site-specific mutagenesis to the nucleotides in the DNA encoding the anti-HER2 antibody or antigen-binding fragment thereof, or the anti-HER2 biparatopic antibody using PCR mutagenesis or other techniques known to those of ordinary skill in the art, to generate DNA encoding variants, followed by expression of the recombinant DNA in cell culture as outlined herein. In addition, the anti-HER2 antibody or antigen-binding fragment thereof, or the anti-HER2 biparatopic antibody can also be prepared by *in vitro* synthesis using established techniques.

As known to those skilled in the art, due to the degeneracy of the genetic codes, the anti-HER2 antibody or antigen-binding fragment thereof, or the anti-HER2 biparatopic antibody of the present invention can be encoded by an extremely large number of nucleic acids, each of which is within the scope of the present invention and can be produced using standard techniques. Thus, once a specific amino acid sequence is identified, those skilled in the art can prepare a variety of different nucleic acids by simply making one or more codon modifications to their respective coding sequences, without altering the amino acid sequences of the the anti-HER2 antibody or antigen-binding fragment thereof, or the anti-HER2 biparatopic antibody of the present invention.

In another aspect, the present invention also provides an expression vector for the nucleic acid encoding the the anti-HER2 antibody or antigen-binding fragment thereof, or the anti-HER2 biparatopic antibody described herein.

The nucleic acid encoding the anti-HER2 antibody or antigen-binding fragment thereof, or the anti-HER2 biparatopic antibody of the present invention can be constructed into a suitable vector, for introduction into a host cell to express the target protein. Vector components typically include, but are not limited to, one or more of the following: a signal sequence, an origin of replication, one or more marker genes, an enhancer element, a promoter, and a transcription termination sequence. The nucleic acid encoding the target protein in the vector is operatively linked to the promoter.

As used herein, the term "operatively linked" refers to the functional linkage between a nucleic acid expression control sequence (e.g., a promoter, a signal sequence, or an array of transcription factor binding sites) and another nucleic acid sequence, and the control sequence thus regulates the transcription and/or translation of the other nucleic acid sequence.

Suitable vectors include plasmids, phagemids, cosmids, artificial chromosomes such as yeast artificial chromosomes (YACs), bacterial artificial chromosomes (BACs) or P1-derived artificial chromosomes (PACs), bacteriophages such as λ phage or M13 phage, and animal virus, etc. Animal virus species used as vectors include retroviruses (including lentiviruses), adenoviruses, adeno-associated viruses, herpesviruses (e.g., herpes simplex virus), poxviruses, baculoviruses, papillomaviruses, papovaviruses (e.g., SV40). Vectors may contain a variety of elements to control expression, including promoter sequences, transcription initiation sequences, enhancer sequences, selectable elements, and reporter genes. Additionally, the vectors may also contain an origin of replication. The vectors may also include components that facilitate their entry into cells, including but not limited to viral particles, liposomes, or protein coats.

In another aspect, the present invention also provides a host cell comprising a nucleic acid or expression vector encoding the anti-HER2 antibody or antigen-binding fragment thereof, or the anti-HER2 biparatopic antibody of the invention.

The cell can be eukaryotic cells, e.g., mammalian host cells, including but not limited to, the SV40-transformed monkey kidney cell line CV1 (COS-7, ATCC, CRL-1651), human embryonic kidney cell lines (293 or subclones of 293 cells in suspension culture, Graham et al., J Gen Virol 1977, 36:59-74), baby hamster kidney cells (BHK-21, ATCC, CCL-10), Chinese hamster ovary cells/-DHFR (CHO, Urlaub et al., Proc Natl Acad Sci USA 1980, 77:4216-4220), mouse Sertoli cells (TM4, Mather, Biol Reprod 1980, 23:243-251), monkey kidney cells (CV1, ATCC, CCL-70), African Green monkey kidney cells (VERO-76, ATCC, CRL-1587), human cervical carcinoma cells (HELA, ATCC, CCL-2), canine kidney cells (MDCK, ATCC, CCL-34), Buffalo rat hepatocytes (BRL 3A, ATCC, CRL-1442), human lung cells (W138, ATCC, CCL-75), human hepatoma cell lines ( HepG2, ATCC, HB-8065), mouse mammary tumor cells (MMT060562, ATCC, CCL-51), TRI cells (Mather et al., Ann NY Acad Sci 1982, 383:44-68), MRC5 cells, or FS4 cells.

### 4.2 Screening of anti-HER2 antibody of the present invention

The anti-HER2 antibody provided by the present invention can be a murine antibody, a chimeric antibody, a humanized antibody, or a fully human antibody. Methods for generating monoclonal antibodies are known in the art, and any known method (e.g., hybridoma technology, phage display technology, single lymphocyte gene cloning technology, etc.) can be used in the present invention to prepare monoclonal antibodies that specifically bind to HER2.

In some embodiments, a polypeptide of any fragment in the HER2 amino acid sequence (preferably the HER2 extracellular domain) can be used as the target antigen to immunize mice. Antibody variable region genes are amplified from mature B cells of the immunized mice, and a phage display library comprising the mouse antibody variable regions being displayed on the surface of phages is constructed using the phage display technology. The library is subsequently panned against a specific target molecule (e.g., the target antigen used to immunize mice), and the interaction between the antibody variable regions displayed on the phage surface and the target antigen is detected. The antibody variable region library is screened and amplified by an *in vitro* selection method analogous to natural selection.

In some embodiments, the present invention involves constructing a phage display library and performing panning against the target antigen to screen for antibodies that recognize the target antigen from the antibody library. Various phage display methods known in the art may be utilized to generate the antibodies of the invention. For example, US5223409, US5622699, and US6068829 disclose methods for preparing phage library. Alternatively, phage display library can also be constructed according to the methods described in "Antibody Phage Display: Methods and Protocols (edited by O'Brien and Aitken)." In some embodiments, nucleic acids encoding antibody variable regions can be inserted into a phage coat protein gene, enabling the phage to display the antibody variable regions on its surface. Simultaneously, the phage contains nucleic acids encoding the antibody variable regions, thereby achieving a link between the phenotype and the genotype of the antibody variable regions.

Regarding the antibody (or single-chain antibody) screening via phage display, VH and/or VL regions of the antibody (or single-chain antibody) from a sizeable library can be expressed on the surface of filamentous phage particles, thereby allowing them to pair and form binding domains. Phages can be selected from the library based on the recognition and binding of the target antigen by the binding domains they display.

In some embodiments, panning can be achieved by infecting host bacteria with phages and propagating and amplifying in the host. Phages displaying single-chain antibody fragments on their surface, which are secreted by the host bacteria, are collected and subjectied to multiple rounds of panning as needed until phages capable of selectively or specifically binding the target antigen are obtained. Finally, the amino acid sequences of the antibody variable regions are determined by sequencing the antibody genes within the phage genome (Arap et al., Science 1998, 279:377-380; Smith et al., Science 1985, 228:1315-1317).

In a specific embodiment, the present invention uses a recombinant protein of the HER2 extracellular domain as the target antigen to immunize mice. Gene fragments encoding the heavy chain variable region family and light chain variable region family of antibodies are isolated from the spleenocytes of immunized mice, and scFv fragments are then prepared and inserted to the phage surface protein III gene. Through co-expression, these fragments are participated in phage assembly and displayed on the phage surface, thus constructing a phage display library. Using the phage display library, multiple rounds of adsorption-elution-amplification (panning) are performed against a specific target antigen (e.g., the antigen used to immunize mice) to enrich phages capable of specifically binding to the target antigen. The corresponding DNA sequence information is then obtained via gene sequencing technology, from which the amino acid sequences of the antibody variable regions are further deduced.

### 5. Method of Preparation

### 5.1 Method for preparing anti-HER2 antibody or antigen-binding fragment thereof, or anti-HER2 biparatopic antibody

The present invention provides a method for preparing the anti-HER2 antibody or antigen-binding fragment thereof, or the anti-HER2 biparatopic antibody of the present invention using the host cell.

The method comprises transfecting a nucleic acid or expression vector encoding the anti-HER2 antibody or antigen-binding fragment thereof, or the anti-HER2 biparatopic antibody of the present invention into a host cell, and culturing the host cell in a culture medium for a period of time to express the anti-HER2 antibody or antigen-binding fragment thereof, or the anti-HER2 biparatopic antibody of the present invention. Without limitation, commercially available media can be used as the culture medium.

Preferably, the expressed anti-HER2 antibody or antigen-binding fragment thereof, or anti-HER2 biparatopic antibody can be secreted into the culture medium where the host cells are grown. Antibodies are recovered from the culture medium using conventional protein purification methods, such as centrifugation or ultrafiltration to remove impurities, or affinity chromatography to purify the resulting product; other purification techniques can also be used, such as anion or cation exchange chromatography, hydrophobic interaction chromatography, and hydroxyapatite chromatography.

### 5.2 Method for preparing the anti-HER2 biparatopic ADC

The ADC of the present invention can be prepared by any method known in the art, including but not limited to: (1) reacting a nucleophilic or electrophilic group of the antibody with a cleavable linker to form an antibody-cleavable linker intermediate (Ab-L) via a covalent bond, followed by reaction with a small-molecule toxin compound (D), wherein the intermediate Ab-L may or may not undergo purification step(s) before reacting with the small-molecule toxin compound; (2) reacting a nucleophilic or electrophilic group of the small-molecule toxin compound with a cleavable linker to form a small-molecule toxin compound-cleavable linker intermediate (D-L) via a covalent bond, followed by reaction with a nucleophilic or electrophilic group of the antibody, wherein the intermediate D-L may or may not undergo purification step(s) before reacting with the antibody; or (3) mixing and reacting the antibody, a cleavable linker, and a small-molecule toxin compound simultaneously, allowing covalent bonds to be formed between the antibody and the cleavable linker and between the cleavable linker and the small-molecule toxin compound at the same time, thereby preparing the ADC of the present invention. Several specific examples of methods for preparing ADC are known in the art, such as those described in US Pat. NO. 8,624,003 (one-pot method), US Pat. NO.8,163,888 (one-step method), and US Pat. NO. 5,208,020 (two-step method).

In some embodiments, the antibody is subjected to reducing conditions prior to the conjugation reaction to generate one or more free cysteine residues. In some implementations, a reducing agent (e.g., dithiothreitol [DTT], 2-mercaptoethanol, or tris(2-carboxyethyl)phosphine [TCEP]) is used to interact with the antibody to reduce the interchain disulfide bonds in the hinge region of the antibody, thereby generating a partially or fully reduced antibody with free sulfhydryl groups. The reducing agent preferentially reduces the interchain disulfide bonds in the hinge region of the antibody while leaving the intrachain disulfide bonds of the antibody intact. In some embodiments, in a buffer containing a chelating agent, the reducing agent TCEP is used to react with the antibody to obtain a partially or fully reduced antibody with free sulfhydryl groups. The chelating agent includes, but is not limited to, ethylenediaminetetraacetic acid (EDTA) and diethylenetriaminepentaacetic acid (DTPA), and the buffer includes, but is not limited to, histidine-hydrochloride, sodium phosphate, sodium borate, and sodium acetate solutions.

In some embodiments, the partially or fully reduced antibody with free sulfhydryl groups described above can react with a reactive functional group (e.g., a maleimide group) of a cleavable linker (L) or a small-molecule toxin compound-cleavable linker intermediate (D-L) to form a covalent bond (e.g., a thioether bond), thereby obtaining an antibody-cleavable linker intermediate (Ab-L) or an ADC. Herein, the term "small-molecule toxin compound-cleavable linker intermediate (D-L)" is used interchangeably with "drug payload" or "payload".

The purification methods for the ADC prepared by the aforementioned procedures can be any biochemical techniques known in the art for protein purification or any combination thereof, including but not limited to affinity chromatography, ion exchange chromatography, mixed-mode chromatography (e.g., ceramic hydroxyapatite chromatography), hydrophobic interaction chromatography, size exclusion chromatography, dialysis, filtration, selective precipitation, or any combination thereof.

### 6. Pharmaceutical Composition

The present invention provides a pharmaceutical composition comprising the anti-HER2 antibody or antigen-binding fragment thereof, the anti-HER2 biparatopic antibody, or the anti-HER2 biparatopic ADC of the present invention, and a pharmaceutically acceptable carrier.

The pharmaceutical composition can contain any type of pharmaceutically acceptable carrier. Suitable carriers include excipients, surfactants, thickening or emulsifying agents, solid binders, dispersing or suspending auxiliaries, solubilizers, colorants, flavoring agents, coating agents, disintegrants, lubricants, sweeteners, preservatives, isotonic agents, or combinations thereof. For example, the selection and use of appropriate carriers are taught in: Gennaro, Ed., Remington: The Science and Practice of Pharmacy, 20th Edition, 2003 (Lippincott Williams & Wilkins), the disclosure of which is incorporated herein by reference.

Preferably, the pharmaceutical composition is suitable for intravenous, intramuscular, subcutaneous, parenteral, spinal, or epidermal administration. As used herein, the term "parenteral administration" refers to non-enteral and topical modes of administration, including but not limited to intravenous, intramuscular, intra-arterial, intrathecal, intracapsular, intraorbital, intracardiac, intradermal, intraperitoneal, intratracheal, subcutaneous, subepidermal, intra-articular, subcapsular, subarachnoid, intraspinal, epidural, and intrasternal injection and infusion. Alternatively, the antibody of the present invention can also be administered via non-parenteral routes (e.g., topical, epidermal, or mucosal administration routes), such as intranasal, oral, vaginal, rectal, sublingual, or topical administration. In view of the different routes of administration, the active ingredient can be encapsulated in a material to protect it from the effects of acids/bases and other natural conditions that may inactivate it.

The pharmaceutical composition can be a sterile aqueous solution or dispersion. They are also capable of forming microemulsions, liposomes, or other ordered structural combinations suitable for high drug concentrations.

The amount of the active ingredient that can be combined with a carrier material to form a single dosage form is determined based on the subject and the specific mode of administration, and is generally the amount of the composition that can produce a therapeutic effect. Typically, the composition formed with a pharmaceutically acceptable carrier contains about 0.01% to about 99% of the active ingredient, preferably from about 0.1% to about 70%, and most preferably from about 1% to about 30% of the active ingredient.

The dosage regimens can be adjusted to provide the optimal desired response (e.g., a therapeutic response). For example, a single dose may be administered, several divided doses may be administered, or the dose may be proportionally reduced or increased as indicated by the therapeutic situation. It is particularly advantageous to formulate compositions for parenteral administration in dosage unit for ease of administration and uniformity of dosage. As used herein, "dosage unit" refers to a physically discrete unit suitable as unitary dose for treating a subject; each unit dose contains a predetermined quantity of the active ingredient obtained by calculation of the active ingredient co-administered with the required pharmaceutical carrier to produce the desired therapeutic effect. In addition, the anti-HER2 antibody or antigen-binding fragment thereof, the anti-HER2 biparatopic antibody, or the anti-HER2 biparatopic ADC of the present invention can also be administered as a sustained-release formulation, which can reduce the frequency of administration.

The anti-HER2 antibody or antigen-binding fragment thereof, the anti-HER2 biparatopic antibody, or the anti-HER2 biparatopic ADC of the present invention, or a composition comprising the anti-HER2 antibody or antigen-binding fragment thereof, the anti-HER2 biparatopic antibody, or the anti-HER2 biparatopic ADC, can be administered at a dosage ranging from about 0.0001 mg/kg to 100 mg/kg body weight, typically from 0.001 mg/kg to 50 mg/kg body weight.

A "therapeutically effective dose" of the anti-HER2 antibody or antigen-binding fragment thereof, the anti-HER2 biparatopic antibody, or the anti-HER2 biparatopic ADC of the present invention preferably results in a reduction in the severity of disease symptoms, an increase in the frequency and duration of progression-free periods, or prevention of physical impairment or disability caused by the disease. For example, a "therapeutically effective dose" for tumor-bearing subjects preferably inhibits tumor growth by at least about 20%, more preferably by at least about 40%, even more preferably by at least about 60%, and still more preferably by at least about 80%, compared to untreated subjects. A therapeutically effective amount of the therapeutic antibody or ADC can reduce tumor size or alleviate symptoms of a subject, wherein the subject is typically a human or other mammal. A "therapeutically effective dose" can also be determined differently based on various factors including but not limited to formulation method, administration method, age, physical condition, body weight, gender or pathological conditions of the patient, diet, administration time, dosing interval, excretion rate, and response sensitivity.

The pharmaceutical composition may be selected from a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. See, for example, Robinson, Ed., Sustained and Controlled Release Drug Delivery Systems, 1978 (Marcel Dekker).

The therapeutic pharmaceutical composition can be delivered by a medical device selected from the group consisting of: (1) needle-free subcutaneous injection devices (e.g., US5399163, US5383851, US5312335, US5064413, US4941880, US4790824, and US4596556); (2) microinfusion pumps (US4487603); (3) transdermal devices (US4486194); (4) infusion sets (US4447233 and US4447224); and (5) osmotic devices (US4439196 and US4475196); and the disclosures of which are incorporated herein by reference.

In some embodiments, the anti-HER2 antibody or antigen-binding fragment thereof, the anti-HER2 biparatopic antibody, or the anti-HER2 biparatopic ADC of the present invention can be formulated to ensure biodistribution *in vivo.* For example, to ensure that the therapeutic antibody or ADC of the present invention crosses the blood-brain barrier, the therapeutic antibody or ADC can be formulated into liposomes, which can additionally contain targeting moieties to enhance selective delivery to specific cells or organs. See, for example, US4522811, US5374548, US5416016, and US5399331; Ranade, J Clin Pharmacol 1989, 29:685-694; Umezawa et al., Biochem Biophys Res Commun 1988, 153:1038-1044; Bloeman et al., FEBS Lett 1995, 357:140-144; Owais et al., Antimicrob Agents Chemother 1995, 39:180-184; Briscoe et al., Am J Physiol 1995, 268:L374-380; Schreier et al., J Biol Chem 1994, 269:9090-9098; Keinanen and Laukkanen, FEBS Lett 1994, 346:123-126; and Killion and Fidler, Immunomethods 1994, 4:273-279.

### 7. Kit

The present invention provides a kit comprising an effective amount of the anti-HER2 antibody or antigen-binding fragment thereof, the anti-HER2 biparatopic antibody, the anti-HER2 biparatopic ADC, or a pharmaceutical composition of the present invention, and optionally at least one additional tumor therapeutic agent (i.e., the kit may or may not contain at least one additional tumor therapeutic agent). Preferably, the tumor therapeutic agents may include, but are not limited to: another antagonist of ErbB2/HER2; an EGFR antagonist, a HER3 antagonist; a MET antagonist, a small-molecule inhibitor of MET (e.g., capmatinib); an IGF1R antagonist (e.g., an anti-IGF1R antibody); a B-Raf inhibitor (e.g., vemurafenib, sorafenib, GDC-0879, PLX-4720); a PDGFR-α inhibitor (e.g., an anti-PDGFR-α antibody); a PDGFR-β inhibitor (e.g., anti-PDGFR-β antibodies or small-molecule kinase inhibitors [e.g., imatinib mesylate or sunitinib malate]); PDGF ligand inhibitors (e.g., an anti-PDGF-A antibody, an anti-PDGF-B antibody, an anti-PDGF-C antibody, an anti-PDGF-D antibody, aptamer, siRNA, etc.); a VEGF antagonist (e.g., VEGF-Trap, such as aflibercept); an anti-VEGF antibody (e.g., bevacizumab); a VEGF receptor kinase inhibitor (e.g., sunitinib, sorafenib, or pazopanib); a DLL4 antagonist (e.g., REGN421); an Ang2 antagonist (e.g., an anti-Ang2 antibody disclosed in WO2011014469, such as H1H685P); a FOLH1 antagonist (e.g., an anti-FOLH1 antibody); a STEAP1 or STEAP2 antagonist; a TMPRSS2 antagonist; a MSLN antagonist; a MUC16 antagonist; a CLEC12A antagonist; a PD-1 or PD-L1 blocker (e.g., pembrolizumab or nivolumab); a hormone receptor modulator (e.g., an estrogen receptor modulator [such as tamoxifen]; an androgen receptor modulator); an aromatase inhibitor (e.g., letrozole, anastrozole, exemestane); a kinase inhibitor (e.g., a tyrosine kinase inhibitor [such as lapatinib]); a cytokine agonist; a cytokine inhibitor (including a small-molecule cytokine inhibitor and an antibody binding to cytokines such as IL-1, IL-2, IL-3, IL-4, IL-5, IL-6, IL-8, IL-9, IL-11, IL-12, IL-13, IL-17, IL-18, or their respective receptors); a chemotherapeutic agent (including but not limited to microtubule disruptors, antimetabolites, topoisomerase inhibitors, DNA intercalators, alkylating agents), etc.

In some embodiments, another antagonist of ErbB2/HER2 includes an anti-HER2 monoclonal antibody (e.g., trastuzumab, pertuzumab) and/or ADC (e.g., T-DM1, DS-8201), or an anti-ErbB2/HER2 small-molecule inhibitor (e.g., tyrosine kinase inhibitors such as lapatinib, pyrotinib, and neratinib); the EGFR antagonist includes an anti-EGFR antibody (e.g., cetuximab, panitumumab), an anti-EGFR small-molecule inhibitor (e.g., gefitinib, erlotinib), and an anti-EGFRvIII antagonist (e.g., an anti-EGFRvIII antibody); the HER3 antagonist includes but is not limited to an anti-HER3 antibody (e.g., patritumab); the MET antagonist includes but is not limited to an anti-MET antibody (e.g., onartuzumab, emibetuzumab, and H4H14639D).

### 8. Methods and Uses for Treating HER2-Expressing Cancers

In one aspect, the present invention relates to a method of treating HER2-expressing cancers, comprising administering to a subject in need thereof an effective amount of the anti-HER2 biparatopic antibody, the anti-HER2 biparatopic ADC, or the pharmaceutical composition or the kit of the present invention. Alternatively, the present invention relates to the use of the aforementioned antibody, ADC, pharmaceutical composition or kit in the preparation of a medicament for treating HER2-expressing cancers. Alternatively, the present application relates to the aforementioned antibody, ADC, pharmaceutical composition or kit for use in treating HER2-expressing cancers.

The cancers include, but are not limited to, breast cancer, ovarian cancer, cervical cancer, colorectal cancer, gastric cancer, esophageal cancer, lung cancer, head and neck cancer, melanoma, pancreatic cancer, liver cancer, bile duct cancer, kidney cancer, bladder cancer, thyroid cancer, prostate cancer, and endometrial cancer, wherein the cancers also include cancers at all stages, such as early-stage cancer, non-metastatic cancer, primary cancer, advanced cancer, locally advanced cancer, metastatic cancer, or cancer in remission. The cancers can be HER2-overexpression or HER2-low-expression. The subject can be a human, a non-human primate, or other mammals such as dogs, mice, or rats.

As known in the art, HER2-expressing cancers can be characterized by the level of HER2 expression on the surface of cancer cells (i.e., by "HER2 status"). The level of HER2 expression can be assessed using methods such as immunohistochemistry (IHC) and fluorescence in situ hybridization (FISH). In some embodiments, the cancers include HER2-overexpressing cancers (e.g., IHC 3+, or IHC 2⁺/FISH⁺) and/or HER2-low-expressing cancers (IHC 2⁺/FISH⁻, or IHC 1+).

In some embodiments, the anti-HER2 biparatopic ADC or pharmaceutical composition thereof exhibits cytotoxic effects on both HER2-overexpressing tumors/cancers and HER2-low-expressing tumors/cancers.

In some embodiments, the anti-HER2 biparatopic antibody or pharmaceutical composition thereof has inhibitory activity against the growth or proliferation of HER2-overexpressing tumor cells.

In some embodiments, administering an effective amount of the anti-HER2 biparatopic antibody, anti-HER2 biparatopic ADC, pharmaceutical composition, or kit of the present invention to a subject in need can reduce tumor volume of the subject, inhibit tumor growth, prolong the disease-free survival or progression-free survival of the subject, increase the overall survival of the subject, reduce tumor metastasis, or improve the quality of life the subject.

In another aspect, the present invention also relates to a method of treating cancers that are resistant to or relapsed from existing HER2-targeting therapeutics, whererin the method comprises administering to a patient in need thereof an effective amount of the anti-HER2 biparatopic antibody, anti-HER2 biparatopic ADC, pharmaceutical composition, or kit of the present invention. Alternatively, the present invention relates to the use of the antibody, ADC, pharmaceutical composition, or kit in the preparation of a medicament for treating cancers that are resistant to or relapsed from HER2-targeted therapeutics.

In some embodiments, the patient exhibits non-response or poor response to one or more existing HER2-targeting therapeutics, including trastuzumab, pertuzumab, T-DM1, and DS-8201. Non-response is manifested as tumor growth, increased tumor volume, metastasis formation, or an increase in the number of metastases in the patient. Non-response can also be manifested as the development of metastasis or a shortened time to disease progression. A poor response refers to tumor growth or metastasis occurring in the patient during or shortly after standard therapy with a HER2-targeting therapeutic.

In another aspect, the present invention also relates to a method of treating patients who are ineligible for or refractory to existing HER2-targeting therapies, or resistant to or relapsed from existing HER2-targeting therapies, wherein the method comprises administering to a patient in need thereof an effective amount of the anti-HER2 biparatopic antibody, anti-HER2 biparatopic ADC, pharmaceutical composition, or kit of the present invention. Alternatively, the present invention relates to the use of the antibody, ADC, pharmaceutical composition, or kit in the preparation of a medicament for treating patients who are ineligible for or refractory to existing HER2-targeting therapies, or resistant to or relapsed from existing HER2-targeting therapies. The HER2-targeting therapies include treatment with trastuzumab, pertuzumab, T-DM1, or DS-8201.

In some embodiments, the anti-HER2 biparatopic antibody, anti-HER2 biparatopic ADC, pharmaceutical composition, or kit of the present invention can be used to treat progressive cancer when a patient has experienced disease progression after receiving the existing HER2-targeting therapies.

In another aspect, the anti-HER2 biparatopic antibody, anti-HER2 biparatopic ADC, pharmaceutical composition, or kit of the present invention can be used alone or in combination with other types of cancer therapies known in the art, such as surgery, chemotherapy, radiotherapy, gene therapy, immunotherapy, photodynamic therapy, radiofrequency ablation, etc.

### 9. Detection Uses

The present invention also relates to the use of the anti-HER2 antibody or antigen-binding fragment thereof, or the anti-HER2 biparatopic antibody, for detecting and/or measuring HER2 or HER2-expressing tumor cells in a sample, and for screening cancer patients responsive to treatment with the anti-HER2 biparatopic ADC of the present invention. Alternatively, the present application relates to a method for detecting and/or measuring HER2 or HER2-expressing tumor cells in a sample, and a method for screening cancer patients responsive to the aforementioned ADC treatment, comprising incubating the anti-HER2 antibody or antigen-binding fragment thereof, or the anti-HER2 biparatopic antibody, with the sample or a biological specimen isolated from the patient, and detecting whether the antibody binds to the sample or biological specimen. Alternatively, the present application relates to the aforementioned anti-HER2 antibody or antigen-binding fragment thereof, or the anti-HER2 biparatopic antibody, used for detecting and/or measuring HER2 or HER2-expressing tumor cells in a sample, or for screening cancer patients responsive to the aforementioned ADC treatment.

In some embodiments, the anti-HER2 antibody or antigen-binding fragment thereof, or the anti-HER2 biparatopic antibody can be used for diagnosing conditions or diseases associated with abnormal HER2 expression (e.g., overexpression, underexpression, or lack of expression) to facilitate the determination of treatment regimens. For example, the antibody can be conjugated with a detectable label or a reporter molecule, and the labeled antibody can be incubated with a sample obtained from a patient to diagnose and determine the HER2 expression. The detectable label or reporter molecule can be a radioisotope, such as ³H, ¹⁴C, ³²P, ³⁵S, or ¹²⁵I; a fluorescent material, such as umbelliferone, fluorescein, rhodamine, fluorescein isothiocyanate, dichlorotriazinylamine fluorescein, dansyl chloride, or phycoerythrin; a chemiluminescent material, such as luminol; a bioluminescent material, such as luciferase, luciferin, or aequorin; or an enzyme, such as alkaline phosphatase, β-galactosidase, acetylcholinesterase, horseradish peroxidase, or luciferase. Specific exemplary assays that can be used to detect or measure HER2 in a sample include enzyme-linked immunosorbent assay (ELISA), radioimmunoassay (RIA), immune-PET (e.g., using ⁸⁹Zr, ⁶⁴Cu, etc.), and fluorescence-activated cell sorting (FACS).

The present invention is further illustrated by the following examples, which should not be construed as further limiting. The contents of all drawings and all references, patents and patent applications cited throughout the present invention are expressly incorporated herein by reference. Unless otherwise specified, the materials, reagents, and equipment involved in the following examples are commercially available.

### EXAMPLES

### Example 1. Construction of Anti-HER2 Antibody Library and Screening of Antibodies

### 1.1 Construction of anti-HER2 antibody library

Two BALB/c mice were immunized with the recombinant human HER2 extracellular domain protein (sequence derived from positions 23-652 of NP_004439.2). After three rounds of immunization, blood was collected from the mice, and the antibody titer was measured by ELISA. Results showed that the titer reached levels above 1:1,000,000 (data not shown). The mice were subsequently given a booster immunization, and 3 days later, their spleens were aseptically isolated and single splenocyte suspensions were prepared from each spleen. The collected mouse splenocytes were lysed and total RNA was extracted using the Animal Total RNA Isolation Kit (FuGene). The extracted RNA was served as a template for reverse transcription using a reverse transcription kit (PrimeScript^{™} II First Strand cDNA Synthesis Kit, Takara) to generate cDNA. The cDNA was then used as a template for PCR with specific primers to amplify the full repertoire of mouse antibody gene fragments encoding heavy chain variable regions and light chain variable regions. The light and heavy chain variable region gene fragments were spliced and amplified by overlap extension PCR (SOE-PCR) to generate single-chain antibody variable region (scFv) gene fragments. The scFv sequences were digested and ligated into a phagemid display vector, and the ligation product was electroporated into TG1 competent cells. After the cell culture was infected with the helper phage M13KO7, two phage display libraries were obtained.

### 1.2 Screening and sequence analysis of anti-HER2 antibodies

The two phage display libraries described in Section 1.1 of this example were panned using the recombinant HER2 extracellular domain protein as the target antigen. After 3-5 rounds of "adsorption-elution-amplification" panning, followed by colony ELISA screening and PCR digestion verification, and subsequent sequencing analysis of candidate clones, 15 sequence-specific anti-HER2 murine monoclonal antibodies were finally obtained.

### 1.3 Preparation of anti-HER2 chimeric antibodies

The light and heavy chain genes of the 15 murine anti-HER2 antibodies described above were homologously recombined with the linearized expression vector pcDNA3.1 (the heavy chain variable region VH was ligated to the pcDNA3.1 vector containing the human IgG1 constant region, and the light chain variable region VL was ligated to the pcDNA3.1 vector containing the human CK constant region). Sequence-confirmed chimeric antibody expression vectors were obtained through colony PCR and DNA sequencing. The chimeric antibody expression vectors were extracted and purified using conventional methods. The 15 groups of chimeric antibody expression vectors containing the light and heavy chain gene segments were then transiently transfected into ExpiCHO-S cells (Thermo). Anti-HER2 chimeric antibodies were expressed in serum-free cultures and then purified by Protein A affinity chromatography using the AKTA Pure system.

### 1.4 Detection of binding activity of anti-HER2 chimeric antibodies to ErbB/HER family members

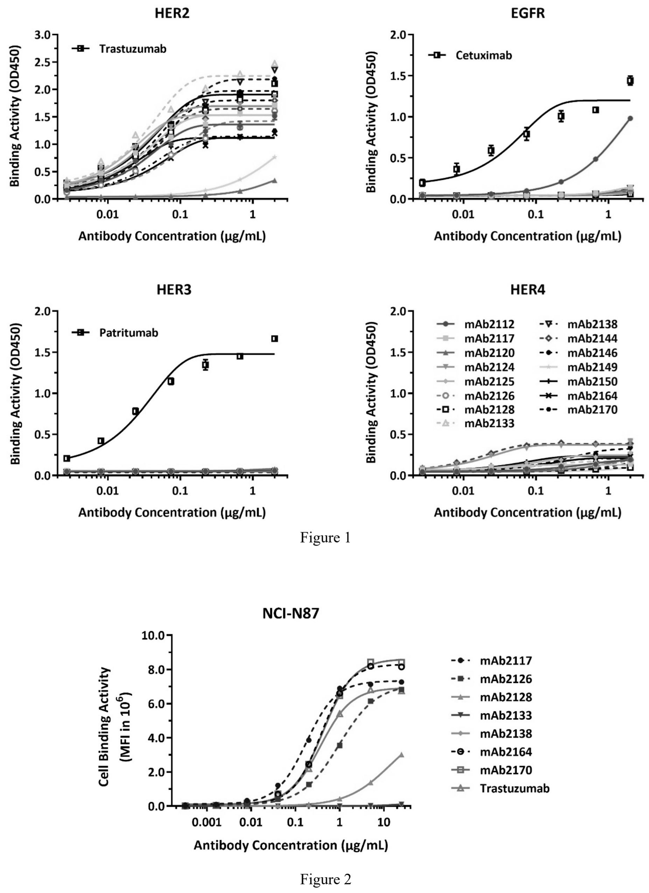

The binding activity of the anti-HER2 chimeric antibodies to human ErbB/HER family members was detected by ELISA. The specific method was as follows: the recombinant proteins of the extracellular domain of human HER2 and the other human ErbB/HER family members (EGFR, HER3, and HER4) were diluted in PBS, and then added to a 96-well ELISA plate at 100 µL per well, followed by incubation at 4°C overnight. After washing the plate with PBS, 200 µL of blocking solution (PBST [PBS + 0.1% Tween-20] containing 1% BSA) was added to each well and incubated at room temperature for 1 hour. After washing with PBST, 100 µL of the testing antibodies (including anti-HER2 chimeric antibodies and the control antibodies trastuzumab, cetuximab, or patritumab) serially diluted in blocking buffer were added to each well, and the plate was incubated at room temperature for 2 hours. After washing the plate with PBST, 100 µL of anti-human IgG (H+L)-HRP antibody (Jackson Immuno) diluted at 1:10,000 in blocking buffer was added to each well and incubated at room temperature for 1 hour. After washing the plate with PBST, 100 µL of TMB substrate solution was added to each well and incubated at room temperature in the dark for 3-10 minutes; 50 µL of stop solution (2 M HCl) was then added to each well and the OD450 value was read using a multimode microplate reader (Varioskan, Thermo). The raw data were analyzed using GraphPad Prism 9 software. As shown in Figure 1, mAb2117, mAb2126, mAb2128, mAb2133, mAb2138, mAb2164, and mAb2170 exhibited strong binding activity to HER2 and no cross-reactivity with other human ErbB/HER family members.

### 1.5 Detection of binding activity of anti-HER2 chimeric antibodies to HER2-expressing tumor cells

The binding activity of the anti-HER2 chimeric antibodies to NCI-N87 cells was detected by flow cytometry. The detailed procedure was as follows: NCI-N87 cells were harvested, washed once with ice-cold FACS buffer (PBS + 1% BSA) and then resuspended to 6-10×10⁶ cells/mL; 50 µL of the cell suspension was added to each well of a 96-well U-bottom plate, followed by the addition of 50 µL per well of serially diluted testing chimeric antibodies or the positive control antibody trastuzumab (with FACS buffer as the negative blank control), and the plate was incubated on ice for 60 minutes. After washing the cells twice with pre-cooled FACS buffer, 100 µL per well of anti-human IgG (H+L)-AF488 antibody (Jackson Immuno) diluted at 1:1,000 in FACS buffer was added, and the cells were incubated on ice in the dark for 40 minutes. After washing the cells twice with pre-cooled FACS buffer, cells were resuspended in 100 µL of pre-cooled FACS buffer and then analyzed using a flow cytometer (NovoCyte 3005, Agilent). As shown in Figure 2, the antibodies mAb2117, mAb2126, mAb2164, and mAb2170 exhibited strong binding activity to NCI-N87 cells.

### 1.6 Analysis of antigen-binding epitopes of anti-HER2 chimeric antibodies

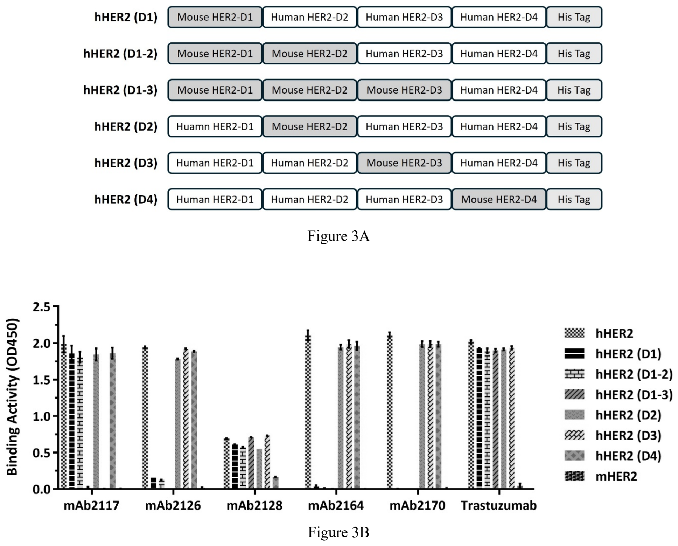

Based on the structures of the human and murine HER2 extracellular region subdomains, the gene sequences encoding different subdomains of the human and mouse HER2 were recombinantly spliced and ligated into the pcDNA3.1 vector via homologous recombination to construct expression plasmids for human-mouse chimeric HER2 extracellular recombinant proteins (Figure 3A). Transient transfection was performed to produce supernatants containing the human-mouse chimeric HER2 extracellular domain recombinant proteins (each recombinant protein contained a 6×His tag at the C-terminus). Using the human-murine chimeric recombinant proteins as antigens, the binding epitopes of the chimeric antibodies within the HER2 extracellular domain were detected by ELISA. The specific method was as follows: the testing chimeric antibodies were diluted in PBS and added to a 96-well ELISA plate at 100 µL per well, followed by incubation at 4°C overnight. After washing the plate with PBS, 200 µL of blocking buffer (PBST containing 1% BSA) was added to each well and incubated at room temperature for 1 hour. After washing the plate with PBST, 100 µL of human HER2, murine HER2, or human-murine chimeric HER2 recombinant proteins diluted at 1:200 in blocking buffer were added to each well. The chimeric proteins include HER2(D1), HER2(D1-2), HER2(D1-3), HER2(D2), HER2(D3), and HER2(D4), wherein the suffix in parentheses indicates that the human sequence of one or more extracellular subdomains in the HER2 chimeric antigen were replaced by the corresponding murine sequence. For example, D1 indicates that extracellular subdomain 1 was replaced with the murine sequence. After incubation at room temperature for 2 hours, the plate was washed with PBST, and 100 µL of anti-6×His-HRP antibody (Proteintech) diluted at 1:10,000 in blocking buffer was added to each well, followed by incubation at room temperature for 1 hour. After washing the plate with PBST, 100 µL of TMB substrate solution was added to each well and incubated at room temperature in the dark for 3-10 minutes. Then, 50 µL of stop solution (2M HCl) was added to each well, and the OD450 value was read using a multimode microplate reader (Varioskan, Thermo). The raw data were analyzed using GraphPad Prism 9 software. As shown in Figure 3B, the epitope recognized by mAb2117 is located in D3 of the HER2 extracellular domain; the epitopes recognized by mAb2126, mAb2164, and mAb2170 are located in D1 of the HER2 extracellular domain; and the epitope recognized by mAb2128 is located in D4 of the HER2 extracellular domain.

### 1.7 Analysis of antigen-binding competition of the anti-HER2 chimeric antibodies

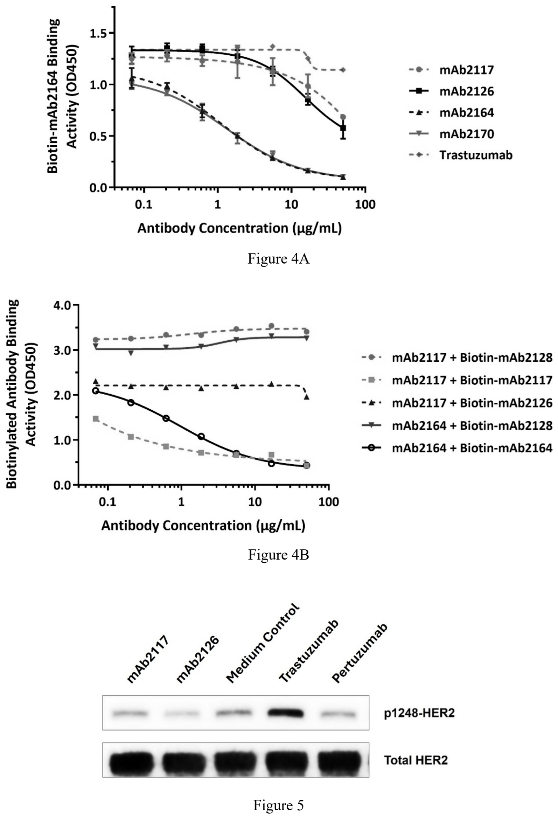

To further verify whether the epitopes bound by the chimeric antibodies on HER2 overlap or cross-react, the competitive antigen-binding activity between the chimeric antibodies was detected by a competitive ELISA. The specific method is as follows: the HER2 extracellular domain recombinant protein was diluted in PBS and added to a 96-well ELISA plate at 100 µL per well, followed by overnight incubation at 4°C. After washing the plate with PBS, 200 µL of blocking buffer (PBST containing 1% BSA) was added to each well and incubated at room temperature for 1 hour. After washing with PBST, 50 µL of the testing chimeric antibodies or the control antibody trastuzumab serially diluted in blocking buffer (starting at 50 µg/mL, followed by 3-fold serial dilutions, with a total of 7 concentration gradients) was added to each well. After incubation at room temperature for 1 hour, 50 µL of a biotin-labeled chimeric antibody (all at a final concentration of 0.5 µg/mL) was added to each well, followed by another hour of incubation at room temperature. After washing with PBST, 100 µL of HRP-Streptavidin (Jackson Immuno) diluted at 1:10,000 in blocking buffer was added to each well and incubated at room temperature for 1 hour. After washing with PBST, 100 µL of TMB substrate solution was added to each well and incubated at room temperature in the dark for 3-10 minutes. Then, 50 µL of stop solution (2 M HCl) was added to each well and the OD450 values were measured using a multimode microplate reader (Varioskan, Thermo). Results showed that mAb2164 and mAb2170 completely competed for binding of the HER2 extracellular domain recombinant protein (Figure 4A); mAb2164 partially competed with mAb2117 and mAb2126 for binding of the HER2 extracellular domain recombinant protein, while mAb2164 did not compete with trastuzumab for binding of the HER2 extracellular domain recombinant protein (Figure 4A); mAb2164 did not compete with mAb2128 for binding of the HER2 extracellular domain recombinant protein, and mAb2117 did not compete with mAb2126 or mAb2128 for binding of the HER2 extracellular domain recombinant protein (Figure 4B).

### 1.8 Analysis of antigen-binding kinetics of anti-HER2 chimeric antibodies

The binding kinetics of each chimeric antibody to the recombinant HER2 extracellular domain protein was determined using a molecular interaction analyzer (ForteBio, Model R8). The specific method was as follows: the testing chimeric antibodies (mAb2117, mAb2126, mAb2128, mAb2164, and mAb2170) were diluted to 5 µg/mL in PBS and added to a 96-well black-wall plate at 200 µL per well. The recombinant HER2 extracellular domain protein was diluted to 200 nM in PBS and subjected to 1:2 serial dilutions before being added to the 96-well black-wall plate at 200 µL per well. The 96-well black-wall plate containing the test samples and Protein A sensor were placed in the analyzer. The program was set to allow the Protein A sensor to capture the antibody, followed by detection of the association and dissociation interations with the antigen. After each cycle of association and dissociation, the sensor was regenerated by immersion in 10 mM glycine buffer (pH 1.5) before the next cycle. After the detection, the association rate constant and dissociation rate constant between the antibody and antigen were fitted and extrapolated using the analytical software, and the value of affinity constant (K_{D}) was calculated. As shown in Table 4, the binding affinity constants (K_{D}) of the chimeric antibodies (mAb2117, mAb2126, mAb2128, mAb2164, and mAb2170) to the recombinant HER2 extracellular domain protein were all approximately 10⁻⁹ M.

**Table 4. Results of binding kinetics of the chimeric antibodies to the recombinant HER2 extracellular domain protein**

| Antibody ID | K_{D} (M) | kₐ (1/Ms) | k_{dis} (1/s) | Full R² |
|---|---|---|---|---|
| mAb2117 | 4.278×10⁻⁹ | 3.877×10⁵ | 1.658×10⁻³ | 0.9557 |
| mAb2126 | 1.437×10⁻⁹ | 6.731×10⁴ | 9.671×10⁻⁵ | 0.9983 |
| mAb2128 | 3.440×10⁻⁹ | 2.759×10⁵ | 9.491×10⁻⁴ | 0.9432 |
| mAb2164 | 3.066×10⁻⁹ | 9.872×10⁴ | 3.026×10⁻⁴ | 0.9974 |
| mAb2170 | 2.349×10⁻⁹ | 1.122×10⁵ | 2.636×10⁻⁴ | 0.9965 |

| | | | | |
|---|---|---|---|---|
| Full R² represents the goodness-of-fit between the fitted curve and the measured curve. | | | | |

### 1.9 Effect of anti-HER2 chimeric antibodies on HER2 phosphorylation at Y1248

Published study has demonstrated that trastuzumab can induce phosphorylation of the HER2 intracellular domain at Y1248 in cardiomyocytes, triggering a series of signaling cascades that ultimately disrupt cardiomyocyte homeostasis (Mohan et al., Molecular Cancer Therapeutics 2016, 15:1321-1331), which may be one of the underlying mechanisms for trastuzumab-induced cardiotoxicity. Therefore, this example evaluated whether anti-HER2 antibodies have trastuzumab-like cardiotoxicity by detecting their ability to induce phosphorylation of the HER2-Y1248 in SKBR-3 cells. The specific method is as follows: SKBR-3 cells were harvested and resuspended in RPMI-1640 serum-free medium (containing 0.1% BSA) and seeded in 6-well plates, and then cultured overnight at 37°C. The testing antibodies or the control antibodies (trastuzumab and pertuzumab) were added to each well at a final concentration of 4 µg/mL and incubated at 37°C for 30 minutes. Cells from each well were collected into a 1.5-mL EP tube, washed twice with pre-cooled PBS, and then lysed in 150 µL of RIPA cell lysis buffer (Porteintech) to extract the total protein. The cell lysis supernatant was collected by high-speed centrifugation at 4°C for 10 minutes. Protein concentration in the supernatant was determined by the BCA assay, and reducing buffer was added to prepare samples for protein electrophoresis, followed by SDS-PAGE gel electrophoresis. After electrophoresis, the protein bands were transferred to a PVDF membrane at 80 V for 90 minutes. The PVDF membrane was blocked with 5% skim milk at room temperature. Rabbit anti-human HER2 (Y1248) antibody (CST) or rabbit anti-human HER2 antibody (Abcam) was added at a dilution of 1:1000 and incubated overnight at 4°C. After wahing the PVDF membrane three times with PBST, goat anti-rabbit IgG-HRP antibody (Jacskon Immuno) diluted at 1:5000 in 5% skim milk was added and incubated at room temperature for 1 hour. After washing the PVDF membrane three times with PBST, developing solution was added, and detection was performed using the Bio-Rad imaging system. As shown in Figure 5, compared to the positive control trastuzumab, the chimeric antibodies mAb2117 and mAb2126 did not induce phosphorylation of HER2-Y1248, indicating that they may not have trastuzumab-like cardiotoxicity.

### 1.10 Effect of anti-HER2 chimeric antibodies on NRG-1-induced activation of HER2 downstream signaling pathways

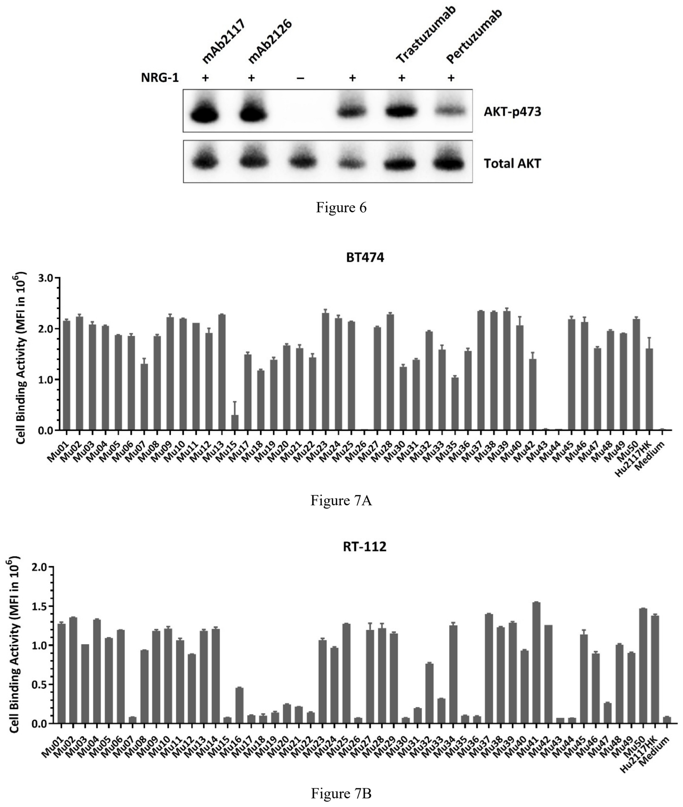

NRG-1 can induce HER2 heterodimerization and activate downstream signaling pathways, with AKT phosphorylation being a hallmark event in this signaling cascade. This example evaluated the effect of the antibodies on HER2 receptor signaling by assessing their inhibitory activity on NRG-1-induced AKT phosphorylation. The specific method is as follows: T47D cells were seeded in a 6-well plate and cultured until reaching approximately 80% confluence. The medium was removed and replaced with serum-free RPMI-1640 for overnight starvation. The testing antibodies or the control antibodies (trastuzumab, pertuzumab) were added to each well at a final concentration of 100 nM and incubated at 37°C for 20 minutes. Then, NRG-1 (NOVUS) was added to each well at a final concentration of 100 ng/mL and incubated for an additional 10 minutes. After washing the cells twice with pre-cooled PBS, total protein extraction and Western blot analysis were performed as described in Section 1.9 of this example. The primary antibodies used were rabbit anti-p-AKT (Ser465) antibody (CST) or rabbit anti-pan-AKT antibody (CST), and the secondary antibody was goat anti-rabbit IgG-HRP antibody (Jackson Immuno). As shown in Figure 6, compared to the positive control antibody pertuzumab, the chimeric antibodies mAb2117 and mAb2126 had no inhibitory effect on NRG-1-induced AKT phosphorylation, indicating that they did not interfere with NRG-1-induced HER2 heterodimerization and activation of its downstream signaling pathways.

### Example 2 Humanization and Optimization of the Anti-HER2 Chimeric Antibody mAb2117

### 2.1 Humanization of the anti-HER2 chimeric antibody mAb2117

Based on the analysis with MOE software, the heavy chain framework region sequence of the chimeric antibody mAb2117 was replaced with the framework region sequence of Germline IGHV3-23 by CDR grafting, designated as Hu2117-H1; the light chain framework region sequence of mAb2117 was replaced with the framework region sequence of Germline IGKV1-39, designated as Hu2117-K1. The chimeric antibody mAb2117 and its humanized antibodies were constructed as single-chain antibody fusion proteins with hIgG1 Fc (scFv-hIgG1 Fc fusion proteins). The scFv antibody gene fragments were generated via gene synthesis, including the humanized single-chain antibodies Hu2117HK and Hu2117KH, and the chimeric single-chain antibody controls Mu2117HK and Mu2117KH, wherein "HK" represents the single-chain antibody structure arranged as "(N-terminus) heavy chain variable region nucleic acid sequence-linker (G₄S)₃-light chain variable region nucleic acid sequence (C-terminus)", and "KH" represents the single-chain antibody structure arranged as "(N-terminus) light chain variable region nucleic acid sequence-linker (G₄S)₃-heavy chain variable region nucleic acid sequence (C-terminus)". The scFv gene fragments were homologously recombined into the pcDNA3.1 vector (containing a human IgG1 Fc sequence) to construct antibody expression plasmids. After extracting the expression plasmids, they were transiently transfected into ExpiCHO-S cells (Thermo) for serum-free culture. The antibodies were purified from the culture supernatant by Protein A affinity chromatography using an AKTA Pure system to obtain single-chain antibody fusion proteins. Binding kinetics of the single-chain antibody fusion proteins to the recombinant HER2 protein were analyzed using ForteBio. As shown in Table 5, results demonstrated that the humanized antibodies retained its parental antibody's binding activity to HER2, with affinity constants (K_{D}) of approximately 10⁻⁷ M.

**Table 5. Results of binding kinetics of the single-chain antibody fusion proteins to the recombinant HER2 protein**

| Antibody ID | K_{D} (M) | kₐ (1/Ms) | k_{dis} (1/s) | Full R² |
|---|---|---|---|---|
| Hu2117HK | 1.777×10⁻⁷ | 1.664×10⁴ | 2.956×10⁻³ | 0.9322 |
| Hu2117KH | 1.471×10⁻⁷ | 2.589×10⁴ | 3.809×10⁻³ | 0.8669 |
| Mu2117HK | 7.599×10⁻⁸ | 6.880×10⁴ | 5.228×10⁻³ | 0.9249 |
| Mu2117KH | 7.722×10⁻⁸ | 7.062×10⁴ | 5.453×10⁻³ | 0.9233 |

Full R² represents the goodness-of-fit between the fitted curve and the measured curve.

According to the method described in Section 1.5 of Example 1, the binding activities of the single-chain antibody fusion proteins Hu2117HK and Hu2117KH to BT474 cells were detected using flow cytometry, wherein the chimeric antibody mAb2117 and trastuzumab served as positive controls. Results demonstrated that mAb2117, Hu2117HK, and Hu2117KH can all bind to BT474 cells, among which the binding activity of Hu2117HK to BT474 cells was relatively higher than that of Hu2117KH (data not shown).

### 2.2 Optimization of the humanized anti-HER2 antibody Hu2117HK by single- or multiple-point mutation

Using the online software abYsis (http://abysis.org/abysis/index.html) and the software MOE, amino acid residues with low humaness level, potential stability liability sites, and murine-derived amino acid residues in the framework regions within the light and heavy chain variable region sequences of the humanized antibody Hu2117HK were identified. A total of 50 single- or multi-point mutations were designed to improve the humanization, antigen-binding affinity, and/or stability of the antibody. Specifically, each of the 50 designed single- or multi-point mutations was respectively introduced into the sequence of single-chain antibody Hu2117HK via PCR, and the mutant proteins of the single-chain antibody were expressed.

The transient expression levels of these 50 mutant proteins of the single-chain antibody and their parent single-chain antibody Hu2117HK were detected using the ForteBio Molecular Interaction Analyzer. The specific method is as follows: after transient expression in ExpiCHO-S cells, the culture supernatant was harvested and added to a 96-well black-wall plate at 200 µL/well. The testing samples and the Protein A sensor were placed in the ForteBio analyzer, and the binding rate of each antibody molecule in the culture supernatant to the Protein A sensor was measured using a pre-set quantitative program. After each cycle, the sensor was regenerated by immersion in 10 mM glycine buffer (pH 1.5) before the next cycle. After detection, the expression level of each single-chain antibody was calculated based on the standard curve using the analytical software. The binding kinetics of each single-chain antibody mutant to HER2 were also analyzed using ForteBio according to the method described in Section 1.8 of Example 1. The single-/multi-point mutation information, mutated amino acids, results of transient expression levels and antigen-binding kinetics are shown in Table 6.

**Table 6. Summary of single-/multi-point mutations of the single-chain antibody Hu2117HK, and results of transient expression levels and HER2-binding kinetics**

| Antibody ID | Single-/multi-point mutations | | Transient Expression Level (mg/L) | Antigen Binding Kinetics | | | |
|---|---|---|---|---|---|---|---|
| | Mutation Site | Mutation | | K_{D} (M) | kₐ (1/Ms) | k_{dis} (1/s) | Full R² |
| Hu2117HK-Mu01 | L43 | S43A | 184 | 8.33×10⁻⁹ | 8.62×10⁴ | 7.18×10⁻⁴ | 0.9277 |
| Hu2117HK-Mu02 | L85 | V85T | 135 | 8.32×10⁻⁹ | 8.86×10⁴ | 7.37×10⁻⁴ | 0.9177 |
| Hu2117HK-Mu03 | L2 | V2I | 81 | 8.69×10⁻⁹ | 8.16×10⁴ | 7.10×10⁻⁴ | 0.9289 |
| Hu2117HK-Mu04 | L4 | L4M | 94 | 9.88×10⁻⁹ | 9.06×10⁴ | 8.95×10⁻⁴ | 0.9108 |
| Hu2117HK-Mu05 | H29 | Y29F | 148 | 6.76×10⁻⁹ | 7.83×10⁴ | 5.29×10⁻⁴ | 0.9394 |
| Hu2117HK-Mu06 | H49 | A49S | 133 | 8.18×10⁻⁹ | 8.26×10⁴ | 6.76×10⁴ | 0.9311 |
| Hu2117HK-Mu07 | H58 | K58Y | 138 | 1.17×10⁻⁸ | 3.98×10⁴ | 4.65×10⁻⁴ | 0.9904 |
| Hu2117HK-Mu08 | H60 | P60A | 94 | 6.65×10⁻⁹ | 7.45×10⁴ | 4.95×10⁻⁴ | 0.9441 |
| Hu2117HK-Mu09 | | P60G | 119 | 6.38×10⁻⁹ | 7.34×10⁴ | 4.68×10⁻⁴ | 0.9573 |
| Hu2117HK-Mu10 | | P60V | 113 | 7.05×10⁻⁹ | 7.74×10⁴ | 5.46×10⁻⁴ | 0.9493 |
| Hu2117HK-Mu11 | L55 | F55D | 126 | 9.41×10⁻⁹ | 8.54×10⁴ | 8.03×10⁻⁴ | 0.9176 |
| Hu2117HK-Mu12 | | F55P | 192 | 6.40×10⁻⁹ | 7.08×10⁴ | 4.53×10⁻⁴ | 0.9652 |
| Hu2117HK-Mul3 | L87 | F87Y | 362 | 6.05×10⁻⁹ | 8.01×10⁴ | 4.84×10⁻⁴ | 0.9486 |
| Hu2117HK-Mu14 | H61 | D61G | 277 | 7.16×10⁻⁹ | 8.72×10⁴ | 6.24×10⁻⁴ | 0.9328 |
| Hu2117HK-Mu15 | L28 | N28D | 410 | 4.02×10⁻⁹ | 6.08×10³ | 2.44×10⁻⁷ | 0.6555 |
| Hu2117HK-Mu16 | | N28G | 449 | 8.76×10⁻⁹ | 5.82×10⁴ | 5.10×10⁻⁴ | 0.9882 |
| Hu2117HK-Mu17 | | N28I | 417 | 1.49×10⁻⁸ | 4.95×10⁴ | 7.38×10⁻⁴ | 0.9822 |
| Hu2117HK-Mu18 | | N28L | 408 | 1.14×10⁻⁸ | 4.26×10⁴ | 4.83×10⁻⁴ | 0.9726 |
| Hu2117HK-Mu19 | | N28Q | 388 | 1.39×10⁻⁸ | 6.01×10⁴ | 8.37×10⁻⁴ | 0.9590 |
| Hu2117HK-Mu20 | | N28S | 349 | 1.33×10⁻⁸ | 4.65×10⁴ | 6.16×10⁻⁴ | 0.9889 |
| Hu2 117HK-Mu21 | | N28T | 321 | 1.20×10⁻⁸ | 4.68×10⁴ | 5.60×10⁻⁴ | 0.9915 |
| Hu2117HK-Mu22 | | N28V | 409 | 1.34×10⁻⁸ | 4.39×10⁴ | 5.90×10⁻⁴ | 0.9927 |
| Hu2117HK-Mu23 | L29 | G29A | 372 | 6.67×10⁻⁹ | 8.12×10⁴ | 5.42×10⁻⁴ | 0.9320 |
| Hu2117HK-Mu24 | | G29I | 317 | 6.65×10⁻⁹ | 6.37×10⁴ | 4.23×10⁻⁴ | 0.9652 |
| Hu2117HK-Mu25 | | G29S | 356 | 6.73×10⁻⁹ | 8.31×10⁴ | 5.60×10⁻⁴ | 0.9288 |
| Hu2117HK-Mu26 | | G29V | No Expression | N/A | N/A | N/A | N/A |
| Hu2117HK-Mu27 | | G29R | 303 | 7.38×10⁻⁹ | 8.02×10⁴ | 5.92×10⁻⁴ | 0.9341 |
| Hu2117HK-Mu28 | | G29T | 350 | 7.15×10⁻⁹ | 8.28×10⁴ | 5.92×10⁻⁴ | 0.9294 |
| Hu2117HK-Mu29 | L27C | V27L | 374 | 7.62×10⁻⁹ | 8.77×10⁴ | 6.68×10⁻⁴ | 0.9179 |
| Hu2117HK-Mu30 | L27D | H27D | 399 | 8.27×10⁻⁹ | 3.64×10⁴ | 3.01×10⁻⁴ | 0.9813 |
| Hu2117HK-Mu31 | | H27F | 338 | 6.20×10⁻⁹ | 3.74×10⁴ | 2.32×10⁻⁴ | 0.9939 |
| Hu2117HK-Mu32 | | H27S | 357 | 6.09×10⁻⁹ | 6.01×10⁴ | 3.66×10⁻⁴ | 0.9712 |
| Hu2117HK-Mu33 | | H27Y | 395 | 2.14×10⁻⁸ | 5.08×10⁴ | 1.08×10⁻³ | 0.9823 |
| Hu2117HK-Mu34 | L89 | S89Q | 307 | 1.26×10⁻⁸ | 9.35×10⁴ | 1.17×10⁻³ | 0.9065 |
| Hu2117HK-Mu35 | | S89L | 326 | 5.98×10⁻⁸ | 6.19×10⁴ | 3.70×10⁻³ | 0.9441 |
| Hu2117HK-Mu36 | | S89M | 357 | 3.14×10⁻⁸ | 6.32×10⁴ | 1.98×10⁻³ | 0.9534 |
| Hu2117HK-Mu37 | L96 | Y96W | 250 | 1.76×10⁻⁸ | 1.08×10⁴ | 1.91×10⁻³ | 0.8891 |
| Hu2117HK-Mu38 | H30, H31 | S30N, S31N | 240 | 1.21×10⁻⁸ | 1.00×10⁵ | 1.21×10⁻³ | 0.8951 |
| Hu2117HK-Mu39 | H31 | S31N | 292 | 1.23×10⁻⁸ | 9.77×10⁴ | 1.20×10⁻³ | 0.9017 |
| Hu2117HK-Mu40 | H31 | S31D | 272 | 1.19×10⁻⁸ | 7.27×10⁴ | 8.67×10⁻⁴ | 0.9389 |
| Hu2117HK-Mu41 | H55, H57 | S55N, T57A | 291 | 9.66×10⁻⁹ | 6.24×10⁴ | 6.03×10⁻⁴ | 0.9725 |
| Hu2117HK-Mu42 | H53 | G53S | 282 | 3.35×10⁻⁸ | 5.30×10⁴ | 1.78×10⁻³ | 0.9635 |
| Hu2117HK-Mu43 | H97 | A97D | 343 | 5.67×10⁻⁸ | 7.17×10⁴ | 4.06×10⁻³ | 0.8786 |
| Hu2117HK-Mu44 | | A97E | 377 | 4.25×10⁻⁸ | 8.00×10⁴ | 3.40×10⁻³ | 0.8906 |
| Hu2117HK-Mu45 | | A97I | 353 | 1.28×10⁻⁸ | 9.80×10⁴ | 1.26×10⁻³ | 0.8998 |
| Hu2117HK-Mu46 | | A97N | 328 | 1.03×10⁻⁸ | 7.74×10⁴ | 7.94×10⁻⁴ | 0.9425 |
| Hu2117HK-Mu47 | | A97Q | 334 | 1.53×10⁻⁸ | 5.43×10⁴ | 8.28×10⁻⁴ | 0.9842 |
| Hu2117HK-Mu48 | | A97R | 303 | 1.21×10⁻⁸ | 9.79×10⁴ | 1.18×10⁻³ | 0.9026 |
| Hu2117HK-Mu49 | | A97S | 288 | 1.18×10⁻⁸ | 6.45×10⁴ | 7.61×10⁻⁴ | 0.9704 |
| Hu2117HK-Mu50 | | A97V | 381 | 1.60×10⁻⁸ | 1.00×10⁵ | 1.60×10⁻³ | 0.9111 |
| Hu2117HK | N/A | N/A | 412 | 1.14×10⁻⁸ | 9.17×10⁴ | 1.05×10⁻³ | 0.9145 |

| | | | | | | | |
|---|---|---|---|---|---|---|---|
| L indicates light chain; H indicates heavy chain; N/A indicates not applicable; Full R² represents the goodness-of-fit between the fitted curve and the measured curve. | | | | | | | |

According to the method described in Section 1.5 of Example 1, the binding activities of the single-chain antibodies containing single-/multi-point mutations to BT474 cells and RT-112 cells were detected by flow cytometry, wherein the parent single-chain antibody Hu2117HK served as the positive control. As shown in Figure 7, compared to the parent single-chain antibody Hu2117HK, most single-chain antibody mutants retained their binding activity to BT474 cells, and some antibodies even exhibited slightly improved cell binding activities, including Hu2117HK-Mu01, -Mu02, -Mu03, -Mu04, -Mu05, -Mu06, -Mu08, -Mu09, -Mu10, -Mu11, -Mu12, - Mu13, -Mu17, -Mu20, -Mu21, -Mu23, -Mu24, -Mu25, -Mu27, -Mu28, -Mu32, -Mu33, -Mu36, -Mu37, -Mu38, - Mu39, -Mu40, -Mu45, -Mu46, -Mu47, -Mu48, -Mu49, and -Mu50 (Figure 7A). Similarly, the results of binding to RT-112 cells are shown in Figure 7B. Compared to the parent single-chain antibody Hu2117HK, most single-chain antibody mutants retained their binding activity to RT-112 cells, including Hu2117HK-Mu01, -Mu02, -Mu03, - Mu04, -Mu05, -Mu06, -Mu08, -Mu09, -Mu10, -Mu11, -Mu12, -Mu13, -Mu14, -Mu23, -Mu24, -Mu25, -Mu27, - Mu28, -Mu29, -Mu32, -Mu34, -Mu37, -Mu38, -Mu39, -Mu40, -Mu41, -Mu42, -Mu45, -Mu46, -Mu48, -Mu49, and -Mu50.

### 2.3 Optimization of anti-HER2 humanized antibody Hu2117HK by combinatorial mutation

Based on the preferred single-/multi- point mutations identified above, combinatorial mutations were introduced into the variable region sequences of the humanized single-chain antibody Hu2117HK to construct antibody variants containing different combinatorial mutations, including Hu2117-HK201 to -HK203 (abbreviated as HK201~HK203) and Hu2117-HK301 to -HK312 (abbreviated as HK301~HK312). The single-chain antibody samples were prepared according to the method described in Section 2.2 of this example, and the transient expression levels of each mutant and the parent single-chain antibody Hu2117HK were detected using ForteBio. The combinatorial mutations contained in each antibody variant and the results of transient expression level are summarized in Table 7.

**Table 7. Combinatorial Mutation Optimization of the Single-chain Antibody Hu2117HK and the Transient Expression Levels**

| Antibody ID | Combinatorial Mutation | | Transient Expression Level (mg/L) |
|---|---|---|---|
| | Heavy Chain Mutation | Light Chain Mutation | |
| Hu2117-HK201 | P60V, S55N, T57A | G29R | 109 (Day 3) |
| Hu2117-HK202 | A97R | | 116.6 (Day 3) |
| Hu2117-HK203 | P60V, S55N, T57A, A97R | | 125.7 (Day 3) |
| Hu2117-HK301 | P60V, S55N, T57A, A97R, D61G | | 27.3 (Day 3) |
| Hu2117-HK302 | P60V, S55N, T57A, A97R, D61A | | 0 (Day 3) |
| Hu2117-HK303 | P60V, S55N, T57A, A97R, D61E | | 317 (Day 3) |
| Hu2117-HK304 | P60V, S55N, T57A, A97R, D61P | | 263 (Day 7) |
| Hu2117-HK305 | P60V, S55N, T57A, A97R, D61Q | | 15.8 (Day 3) |
| Hu2117-HK306 | P60V, S55N, T57A, A97R, D61R | | 9.61 (Day 3) |
| Hu2117-HK307 | P60V, S55N, T57A, A97R, D61T | | 0 (Day 3) |
| Hu2117-HK308 | P60V, S55N, T57A, A97R, D61V | | 0 (Day 3) |
| Hu2117-HK309 | P60V, S55N, T57A, A97R, S62K | | 273 (Day 7) |
| Hu2117-HK310 | P60V, S55N, T57A, A97R, S62N | | 307 (Day 7) |
| Hu2117-HK311 | P60V, S55N, T57A, A97R, S62P | | 0 (Day 3) |
| Hu2117-HK312 | P60V, S55N, T57A, A97R, S62R | | 0 (Day 3) |

According to the method described in Section 1.5 of Example 1, the binding activities of the single-chain antibodies containing combinatorial mutations and the parent single-chain antibody Hu2117HK to BT474 and RT-112 cells were detected using flow cytometry. Results showed that, at a saturated concentration of 10 µg/mL, antibodies HK201, HK202, and HK203 can bind to both BT474 and RT-112 cells with binding activity comparable to that of the parent antibody Hu2117HK. HK301, HK303, HK304, HK305, HK306, HK309, and HK310 can also bind to RT-112 cells with binding activity comparable to that of the parental antibody Hu2117HK (data not shown).

To further characterize the cell-binding activity of the combinatorially optimized antibodies, HK203, HK303, HK304, HK309, and HK310 were selected and analyzed by flow cytometry according to the method described in Section 1.5 of Example 1, wherein the testing antibodies were serially diluted and then incubated with BT474 and RT-112 cells, with the parental single-chain antibody Hu2117HK serving as a control. As shown in Table 8 and Figure 8, all optimized humanized antibodies were capable of binding to BT474 cells and RT-112 cells, with the binding activities comparable to that of the parental antibody Hu2117HK.

**Table 8. Binding Activity of the Single-Chain Antibody Hu2117HK Variants Optimized by Combinatorial Mutation to BT474 and RT-112 Cells**

| Antibody ID | Cell-Binding Activity (EC50, µg/mL) | |
|---|---|---|
| | BT474 | RT-112 |
| Hu2117-HK303 | 0.1012 | 0.1289 |
| Hu2117-HK304 | 0.0922 | 0.0622 |
| Hu2117-HK309 | 0.1032 | 0.0956 |
| Hu2117-HK310 | 0.1034 | 0.1572 |
| Hu2117-HK203 | 0.1020 | 0.1436 |
| Hu2117HK | 0.1075 | 0.0593 |

### Example 3 Humanization and Optimization of Anti-HER2 Chimeric Antibody mAb2126

### 3.1 Humanization of anti-HER2 chimeric antibody mAb2126

Based on the analysis with MOE software, the heavy chain framework region sequence of the chimeric antibody mAb2126 was replaced with the framework region sequence of Germline IGHV4-4 or IGHV7-4 by CDR grafting, designated as Hu2126-H1 and Hu2126-H2, respectively; the light chain framework region sequence of mAb2126 was replaced with the framework region sequence of Germline IGKV1-39, designated Hu2126-K1. The variable region gene fragments of the humanized antibodies were synthesized, and the light and heavy chain fragments were separately ligated into linearized expression vector pcDNA3.1 to construct antibody expression plasmids (the heavy chain variable region VH was inserted into pcDNA3.1 containing the human IgG1 constant region, and the light chain variable region VL was inserted into pcDNA3.1 containing the human Cκ constant region). The expression plasmids were extracted using conventional methods and transiently transfected into ExpiCHO-S cells (Thermo) for serum-free culture. The humanized antibodies Hu2126-H1K1 and Hu2126-H2K1 in the culture supernatants were purified by Protein A affinity chromatography using an AKTA Pure system.

According to the method described in Section 1.8 of Example 1, the binding kinetics of each humanized antibody to HER2 were determined using ForteBio. As shown in Table 9, the CDR-grafted humanized antibodies retained the binding activity to HER2, among which the binding affinity of Hu2126-H2K1 was comparable to that of the parental chimeric antibody mAb2126.

**Table 9. Binding Kinetics of the Chimeric Antibody mAb2126 before and after Humanization (CDR Grafting)**

| Antibody ID | K_{D} (M) | kₐ (1/Ms) | k_{dis} (1/s) | Full R² |
|---|---|---|---|---|
| mAb2126 | 5.200×10⁻⁹ | 9.659×10⁴ | 5.023×10⁻⁴ | 0.9990 |
| Hu2126-H1K1 | 1.909×10⁻⁸ | 9.452×10⁴ | 1.804×10⁻³ | 0.9958 |
| Hu2126-H2K1 | 6.890×10⁻⁹ | 8.427×10⁴ | 5.806×10⁻⁴ | 0.9946 |

| | | | | |
|---|---|---|---|---|
| Full R² represents the goodness-of-fit between the fitted curve and the measured curve. | | | | |

### 3.2 Optimization of anti-HER2 humanized antibody Hu2126-H2K1 by single-point mutation

Using the online software abYsis (http://abysis.org/abysis/index.html) and MOE software, the light and heavy chain variable region sequences of the humanized antibody Hu2126-H2K1 were analyzed to identify amino acid residues with relatively low humaness level, potential stability liability sites, and murine-derived amino acid residues in the framework regions. A total of 63 single-point mutations were designed to improve the antibody's humanization, antigen-binding affinity, and/or stability. Specifically, each of the 63 designed single-point mutations was introduced into the humanized antibody Hu2126-H2K1 sequence via PCR and expression plasmids were then constructed. The light and heavy chain expression plasmids were co-transfected transiently into ExpiCHO-S cells for antibody expression, and cell culture supernatants of 63 antibody mutants containing single-point mutations were obtained. According to the method described in Section 2.2 of Example 2, the expression level after 3 days of transient culture and binding kinetics to HER2 of each antibody mutant were detected using ForteBio. Results are summarized in Table 10. Compared to the parent antibody Hu2126-H2K1, 42 out of the 63 designed single-point mutations did not significantly affect the antigen-binding affinity of the antibody, including 17 single-point mutations in the antibody light chain: 2126-Hu-L02, -L05, -L06, -L07, -L08, -L09, -L10, -L11, -L12, -L13, -L14, - L15, -L16, -L17, and -L18. , -L19, and -L21; and 25 single-point mutations in the antibody heavy chain: 2126-Hu-H22, -H23, -H24, -H25, -H27, -H28, -H29, -H31, -H32, -H33, -H34, -H35, -H36, -H37, -H38, -H39, -H40, -H42, - H43, -H44, -H45, -H46, -H50, -H54, and -H59.

**Table 10. Summaey of Single-Point Mutation Information of Humanized Antibody Hu2126-H2K1, Transient Expression Levels, and Binding Kinetics to HER2**

| Antibody ID | Single-Point Mutation | | Transient Expression Level (mg/L) | Antigen Binding Kinetics | | | |
|---|---|---|---|---|---|---|---|
| | Mutation Site | Mutation | | K_{D} (M) | kₐ (1/Ms) | k_{dis} (1/s) | Full R² |
| 2126-Hu-L01 | L28 | D28S | 59.2 | 1.75×10⁻⁸ | 1.12×10⁵ | 1.95×10⁻³ | 0.9988 |
| 2126-Hu-L02 | L24 | K24R | 49.6 | 5.58×10⁻⁹ | 9.87×10⁴ | 5.51×10⁻⁴ | 0.9989 |
| 2126-Hu-L03 | L30 | Y30S | 54.6 | 3.95×10⁻⁸ | 4.71×10⁴ | 1.86×10⁻³ | 0.9997 |
| 2126-Hu-L04 | L32 | A32Y | 65 | 1.39×10⁻⁸ | 4.39×10⁴ | 6.11×10⁻⁴ | 0.9971 |
| 2126-Hu-L05 | L49 | N49Y | 61.2 | 1.34×10⁻⁸ | 1.04×10⁵ | 1.39×10⁻³ | 0.9986 |
| 2126-Hu-L06 | L50 | S50A | 59.1 | 6.63×10⁻⁹ | 9.94×10⁴ | 6.59×10⁻⁴ | 0.9988 |
| 2126-Hu-L07 | L53 | Y53D | 54.8 | 6.46×10⁻⁹ | 9.43×10⁴ | 6.10×10⁻⁴ | 0.9989 |
| 2126-Hu-L08 | | Y53E | 48.8 | 6.57×10⁻⁹ | 9.43×10⁴ | 6.20×10⁻⁴ | 0.9989 |
| 2126-Hu-L09 | | Y53K | 46.7 | 5.11×10⁻⁹ | 9.59×10⁴ | 4.90×10⁻⁴ | 0.9990 |
| 2126-Hu-L10 | | Y53N | 46.5 | 5.38×10⁻⁹ | 1.04×10⁵ | 5.58×10⁻⁴ | 0.9989 |
| 2126-Hu-L11 | | Y53Q | 51.9 | 6.25×10⁻⁹ | 1.04×10⁵ | 6.51×10⁻⁴ | 0.9990 |
| 2126-Hu-L12 | | Y53S | 49.9 | 6.13×10⁻⁹ | 1.01×10⁵ | 6.19×10⁻⁴ | 0.9988 |
| 2126-Hu-L13 | | Y53T | 41.7 | 5.34×10⁻⁹ | 9.95×10⁴ | 5.31×10⁻⁴ | 0.9986 |
| 2126-Hu-L14 | L55 | Y55A | 62.5 | 6.48×10⁻⁹ | 1.03×10⁵ | 6.66×10⁻⁴ | 0.9983 |
| 2126-Hu-L15 | | Y55E | 59.5 | 8.29×10⁻⁹ | 1.09×10⁵ | 9.06×10⁻⁴ | 0.9981 |
| 2126-Hu-L16 | | Y55P | 65.7 | 4.38×10⁻⁹ | 1.06×10⁵ | 4.64×10⁻⁴ | 0.9977 |
| 2126-Hu-L17 | | Y55Q | 46 | 7.91×10⁻⁹ | 9.93×10⁴ | 7.86×10⁻⁴ | 0.9991 |
| 2126-Hu-L18 | L91 | H91S | 58.3 | 8.83×10⁻⁹ | 1.03×10⁵ | 9.12×10⁻⁴ | 0.9989 |
| 2126-Hu-L19 | | H91A | 77.9 | 8.07×10⁻⁹ | 1.05×10⁵ | 8.50×10⁻⁴ | 0.9989 |
| 2126-Hu-L20 | | H91R | No Exprssion | N/A | N/A | N/A | N/A |
| 2126-Hu-L21 | | H91Y | 51.8 | 1.77×10⁻⁹ | 1.09×10⁵ | 1.93×10⁻⁴ | 0.9988 |
| 2126-Hu-H22 | H09 | S09G | 48.3 | 5.38×10⁻⁹ | 1.01×10⁵ | 5.43×10⁻⁴ | 0.9987 |
| 2126-Hu-H23 | H18 | V18L | 57.6 | 5.90×10⁻⁹ | 1.06×10⁵ | 6.23×10⁻⁴ | 0.9985 |
| 2126-Hu-H24 | H33 | S33A | 59.1 | 7.70×10⁻⁹ | 1.08×10⁵ | 8.30×10⁻⁴ | 0.9983 |
| 2126-Hu-H25 | H53 | E53N | 61.3 | 7.57×10⁻⁹ | 9.90×10⁴ | 7.49×10⁻⁴ | 0.9985 |
| 2126-Hu-H26 | | E53D | 43 | 1.11×10⁻⁸ | 1.07×10⁵ | 1.19×10⁻³ | 0.9989 |
| 2126-Hu-H27 | | E53G | 50.9 | 1.67×10⁻⁹ | 9.79×10⁴ | 1.64×10⁻⁴ | 0.9986 |
| 2126-Hu-H28 | | E53Y | 60.6 | 3.08×10⁻⁹ | 1.14×10⁵ | 3.50×10⁻⁴ | 0.9978 |
| 2126-Hu-H29 | | E53I | 46.7 | 2.61×10⁻⁹ | 9.43×10⁴ | 2.46×10⁻⁴ | 0.9990 |
| 2126-Hu-H30 | H56 | E56N | 38 | 1.31×10⁻⁸ | 1.15×10⁵ | 1.50×10⁻³ | 0.9979 |
| 2126-Hu-H31 | | E56D | 34.9 | 8.81×10⁻⁹ | 1.07×10⁵ | 9.42×10⁻⁴ | 0.9982 |
| 2126-Hu-H32 | | E56S | 56.3 | 8.96×10⁻⁹ | 1.11×10⁵ | 9.98×10⁻⁴ | 0.9980 |
| 2126-Hu-H33 | H62 | D62K | 21.4 | 2.83×10⁻⁹ | 1.09×10⁵ | 3.10×10⁻⁴ | 0.9975 |
| 2126-Hu-H34 | | D62N | 38.9 | 4.31×10⁻⁹ | 1.05×10⁵ | 4.54×10⁻⁴ | 0.9988 |
| 2126-Hu-H35 | H63 | F63V | 53.8 | 5.87×10⁻⁹ | 1.03×10⁵ | 6.03×10⁻⁴ | 0.9989 |
| 2126-Hu-H36 | H68 | V68T | 60.1 | 5.30×10⁻⁹ | 1.05×10⁵ | 5.58×10⁻⁴ | 0.9987 |
| 2126-Hu-H37 | | V68S | 52.4 | 5.31×10⁻⁹ | 1.03×10⁵ | 5.45×10⁻⁴ | 0.9988 |
| 2126-Hu-H38 | H69 | F69I | 42.5 | 8.40×10⁻⁹ | 1.04×10⁵ | 8.74×10⁻⁴ | 0.9986 |
| 2126-Hu-H39 | H71 | L71V | 50.3 | 4.87×10⁻⁹ | 1.05×10⁵ | 5.09×10⁻⁴ | 0.9984 |
| 2126-Hu-H40 | | L71R | 35.3 | 1.92×10⁻⁹ | 1.04×10⁵ | 2.00×10⁻⁴ | 0.9980 |
| 2126-Hu-H41 | H75 | V75I | No Expression | N/A | N/A | N/A | N/A |
| 2126-Hu-H42 | | V75K | 54.1 | 5.11×10⁻⁹ | 1.07×10⁵ | 5.46×10⁻⁴ | 0.9975 |
| 2126-Hu-H43 | | V75T | 48.9 | 7.20×10⁻⁹ | 1.07×10⁵ | 7.69×10⁴ | 0.9988 |
| 2126-Hu-H44 | H82 | I82L | 66.6 | 5.30×10⁻⁹ | 1.06×10⁵ | 5.62×10⁻⁴ | 0.9988 |
| 2126-Hu-H45 | | I82M | 49.3 | 5.76×10⁻⁹ | 1.04×10⁵ | 5.98×10⁻⁴ | 0.9984 |
| 2126-Hu-H46 | H91 | F91Y | 43.4 | 5.70×10⁻⁹ | 1.05×10⁵ | 5.97×10⁻⁴ | 0.9986 |
| 2126-Hu-H47 | H97 | R97F | 53.2 | 1.78×10⁻⁸ | 2.04×10⁵ | 3.62×10⁻³ | 0.9186 |
| 2126-Hu-H48 | | R97K | 50.4 | 3.41×10⁻⁸ | 1.15×10⁵ | 3.92×10⁻³ | 0.9958 |
| 2126-Hu-H49 | | R97N | 52.5 | 3.59×10⁻⁸ | 1.49×10⁵ | 5.33×10⁻³ | 0.9856 |
| 2126-Hu-H50 | | R97Y | 59.1 | 8.00×10⁻⁹ | 1.13×10⁵ | 9.02×10⁻⁴ | 0.9434 |
| 2126-Hu-H51 | H98 | Y98F | 46.4 | 2.38×10⁻⁸ | 1.03×10⁵ | 2.44×10⁻³ | 0.9989 |
| 2126-Hu-H52 | | Y98S | 23.6 | 1.93×10⁻⁸ | 1.52×10⁵ | 2.92×10⁻³ | 0.9207 |
| 2126-Hu-H53 | | Y98N | 45.8 | 4.69×10⁻⁸ | 1.01×10⁵ | 4.75×10⁻³ | 0.9917 |
| 2126-Hu-H54 | | Y98G | 43 | 7.96×10⁻⁹ | 1.18×10⁵ | 9.36×10⁻⁴ | 0.9401 |
| 2126-Hu-H55 | H99 | D99A | 52.4 | 3.37×10⁻⁸ | 1.34×10⁵ | 4.52×10⁻³ | 0.9839 |
| 2126-Hu-H56 | | D99E | 48.6 | 3.62×10⁻⁸ | 1.31×10⁵ | 4.76×10⁻³ | 0.9915 |
| 2126-Hu-H57 | | D99G | 51.6 | 1.45×10⁻⁸ | 1.50×10⁵ | 2.17×10⁻³ | 0.9272 |
| 2126-Hu-H58 | | D99Q | 108 | 2.63×10⁻⁸ | 1.55×10⁵ | 4.09×10⁻³ | 0.9834 |
| 2126-Hu-H59 | | D99S | 47.5 | 2.53×10⁻⁸ | 1.13×10⁵ | 2.87×10⁻³ | 0.9971 |
| 2126-Hu-H60 | | D99Y | 52.8 | 2.38×10⁻⁸ | 1.52×10⁵ | 3.61×10⁻³ | 0.9604 |
| 2126-Hu-H61 | H100B | M100F | 39.8 | 2.20×10⁻⁸ | 9.14×10⁴ | 2.01×10⁻³ | 0.9989 |
| 2126-Hu-H62 | | M100W | 45.8 | 3.48×10⁻⁸ | 1.43×10⁵ | 4.99×10⁻³ | 0.9600 |
| 2126-Hu-H63 | | M100Y | 40 | 2.69×10⁻⁸ | 1.07×10⁵ | 2.87×10⁻³ | 0.9981 |
| Hu2126-H2K1 | N/A | N/A | 44 | 4.71×10⁻⁹ | 9.96×10⁴ | 4.69×10⁻⁴ | 0.9983 |

| | | | | | | | |
|---|---|---|---|---|---|---|---|
| L indicates light chain; H indicates heavy chain; N/A indicates not applicable; Full R² represents the goodness-of-fit between the fitted curve and the measured curve. | | | | | | | |

### 3.3 Optimization of anti-HER2 humanized antibody Hu2126-H2K1 by combinatorial mutation

The selected single-point mutations in the light chain mentioned above were combined and introduced into Hu2126-K1, and the selected single-point mutations in the heavy chain mentioned above were combined and introduced into Hu2126-H2. Light chain combinatorial mutation expression plasmids and heavy chain combinatorial mutation expression plasmids were constructed accordingly. The light and heavy chain expression plasmids were then paired and transiently transfected into ExpiCHO-S cells. After culture, expression and purification, a total of 10 antibody mutants were obtained, designated as Hu2126-H2K1-L71-H72b-Mu10 to -Mu19 (abbreviated as 7172b-Mu10 to -Mu19). According to the method described in Section 1.8 of Example 1, binding kinetics of the humanized antibodies containing combinatorial mutations to HER2 were determined using ForteBio.

The combinatorial mutations contained in each antibody mutant and the results of antigen-binding kinetics are summarized in Table 11. The binding affinities of antibodies 7172b-Mu10, 7172b-Mu14, 7172b-Mu17 and 7172b-Mu19 to HER2 were significantly higher than that of the parental antibody Hu2126-H2K1.

**Talbe 11. Optimization of Antibody Hu2126-H2K1 by Combinatorial Mutation and Binding Kinetics to HER2**

| Antibody ID | Combinatorial Mutation | | Antigen Binding Kinetics | | | |
|---|---|---|---|---|---|---|
| | Combinatorial Mutation in Light Chain | Combinatorial Mutation in Heavy Chain | K_{D} (M) | kₐ (1/Ms) | k_{dis} (1/s) | Full R² |
| 7172b-Mu10 | K24R, Y53S, Y55P, H91Y, S50A | E53G, V68T, F69I, L71R, F91Y, V75K | 3.38×10⁻⁹ | 1.05×10⁵ | 3.56×10⁻⁴ | 0.9981 |
| 7172b-Mu11 | K24R, Y53S, Y55P, H91Y, S50D | | 5.35×10⁻⁹ | 9.01×10⁴ | 4.82×10⁻⁴ | 0.9990 |
| 7172b-Mu12 | K24R, Y53S, Y55P, H91Y, S50E | | 6.74×10⁻⁹ | 7.83×10⁴ | 5.28×10⁴ | 0.9991 |
| 7172b-Mu13 | K24R, Y53S, Y55P, H91Y, S50K | | 7.23×10⁻⁹ | 9.78×10⁴ | 7.07×10⁻⁴ | 0.9985 |
| 7172b-Mu14 | K24R, Y53S, Y55P, H91Y, S50L | | 8.25×10⁻¹⁰ | 1.01×10⁵ | 8.31×10⁻⁵ | 0.9978 |
| 7172b-Mu15 | K24R, Y53S, Y55P, H91Y, S50Q | | 4.49×10⁻⁹ | 1.06×10⁵ | 4.77×10⁴ | 0.9974 |
| 7172b-Mu16 | K24R, Y53S, Y55P, H91Y, S50R | | 5.77×10⁻⁹ | 1.00×10⁵ | 5.78×10⁻⁴ | 0.9971 |
| 7172b-Mu17 | K24R, Y53S, Y55P, H91Y | | 2.80×10⁻⁹ | 9.85×10⁴ | 2.76×10⁻⁴ | 0.9968 |
| 7172b-Mu18 | K24R, Y53S, Y55P, H91Y, S50W | | 7.96×10⁻⁹ | 1.24×10⁵ | 9.88×10⁴ | 0.9967 |
| 7172b-Mu19 | K24R, Y53S, Y55P, H91Y, S50Y | | 2.90×10⁻⁹ | 1.08×10⁵ | 3.14×10⁻⁴ | 0.9978 |

| | | | | | | |
|---|---|---|---|---|---|---|
| Full R² represents the goodness-of-fit between the fitted curve and the measured curve. | | | | | | |

### Example 4 Construction, Optimization, and Functional Activity Analysis of Anti-HER2 Biparatopic Antibody 4.1 Construction of anti-HER2 biparatopic antibody

In this example, a pair of monospecific antibodies (Hu2117-HK304 and Hu2126-H2K1-L71-H72b-Mu14) that recognize distinct epitopes on the HER2 extracellular domain without competing with each other for binding were selected for construction of an exemplary biparatopic antibody. As shown in Figure 9, the biparatopic antibody is in a DVD-IgG configuration, wherein the Fv domain consists of the variable region sequences of Hu2117-HK304, and the IgG domain consists of the sequence of Hu2126-H2K1-L71-H72b-Mu14. The heavy chain variable region of the Fv domain is fused to the heavy chain of the IgG domain via a (G₄S)₁ linker, and the light chain variable region of the Fv domain is fused to the light chain of the IgG domain via a (G₄S)₃ linker. To prevent the effector function mediated by the antibody Fc region from causing a potential killing of non-target cells, the L234F/L235E/P331S mutations (EU Numbering, referred to as "TM mutations") were introduced into the Fc region of the biparatopic antibody to reduce the binding to FcγRs and C1q, thereby eliminating the Fc effector function. The constructed biparatopic antibody was designated 04BS-109-WT. The expression plasmid of the 04BS-109-WT was transiently transfected into ExpiCHO-S cells for protein expression, and the antibody was purified by Protein A affinity chromatography. During purification, it was observed that the 04BS-109-WT bispecific antibody formed colloidal precipitates upon elution (using an eluent without NaCl), resulting in a turbid eluted product. However, after adding 0.1 M NaCl, the colloidal precipitates gradually dissolved, ultimately yielding a clear antibody solution.

### 4.2 Optimization of the anti-HER2 biparatopic antibody

Based on the analysis by MOE software, a series of mutations were designed in the HCDR2 sequence of the Fv domain of the biparatopic antibody 04BS-109-WT to improve the molecular stability of the bispecific antibody. The designed mutations are listed in Table 12 and designated as 04BS-1123-ST01 to -ST06. Mutations were introduced into the 04BS-109-WT sequence via PCR. The constructed expression plasmids of each bispecific antibody variant were transiently transfected into ExpiCHO-S cells and cultured in serum-free medium for antibody protein expression. According to the method described in Section 2.2 of Example 2, antibody expression levels in the culture supernatant were measured using ForteBio. Results showed that the expression level of the antibody 04BS-1123-ST03 was too low (<30 mg/L), while the expression levels of the other bispecific antibody variants exceeded 300 mg/L after 10 days of culture. After purified by Protein A affinity chromatography, the antibodies were analyzed by SEC-HPLC and data indicated that the monomer content of each bispecific antibody variant was close to 100%. However, significant differences in molecular stability were observed among the bispecific antibody variants during the purification process (as shown in Table 12). After the elution step in Protein A affinity chromatography, the sample solution of the bispecific antibody 04BS-1123-ST06 remained clear regardless of whether the eluent contained 0.1 M NaCl, suggesting that the molecular stability of 04BS-1123-ST06 is superior to the other variants and the parent antibody 04BS-109-WT as well.

**Table 12. Stability of the Exemplary Anti-HER2 Biparatopic Antibody 04BS-109-WT and Its Variants**

| Antibody ID | HCDR2 Amino acid sequence in the Fv domain | Molecular Stability before and after Protein A Affinity Chromatography Elution | | |
|---|---|---|---|---|
| | | Conventional Eluent (without NaCl) | | Eluent containing 0.1 M NaCl |
| | | Without adding NaCl after elution | After adding NaCl to 0.1 M after elution | |
| 04BS-109-WT | SISIGGGNYAKYVPSVKG | Colloidal precipitate, Opacity score: 5 | Clear and transparent, Opacity score: 0 | Clear and transparent, Opacity score: 0 |
| 04BS-1123-ST01 | SISIGGGSYTKYVPSVKG | Precipitation occurs after neutralization, Opacity score: 3 | Precipitation occurs, Opacity score: 2 | Not detected |
| 04BS-1123-ST02 | SISIGGGSYTKYVGSVKG | Precipitation occurs after neutralization, Opacity score: 3 | Precipitation occurs, Opacity score: 2 | Not detected |
| 04BS-1123-ST03 | SISIGGGSYTKYVESVKG | Expression level too low; molecular stability not assessed | | |
| 04BS-1123-ST04 | SISIGGGSYTKYPESVKG | Turbidity occurs after neutralization, Opacity score: 2 | Gradually dissolved after adding NaCl, but some precipitate remained | Clear and transparent, Opacity score: 0 |
| 04BS-1123-ST05 | SISIGGGSYTKYPDKVKG | Turbidity occurs after neutralization, Opacity score: 1 | Clear and transparent, Opacity score: 0 | Clear and transparent, Opacity score: 0 |
| 04BS-1123-ST06 | SISIGGGSYTKYVDKVKG | Clear and transparent, Opacity score: 0 | Clear and transparent, Opacity score: 0 | Clear and transparent, Opacity score: 0 |

| | | | | |
|---|---|---|---|---|
| Note: The underlined amino acid residues indicate the amino acid mutations introduced into the HCDR2 sequence of Hu2117-HK304. | | | | |

### 4.3 Cell-based competitive binding assay of anti-HER2 humanized antibodies Hu2117-HK304-06 and Hu2126-H2K1-L71-H72b-Mu14

The anti-HER2 monospecific antibody derived from the Fv domain sequence of 04BS-1123-ST06 was designated Hu2117-HK304-06. To further confirm whether the optimized antibodies Hu2117-HK304-06 and Hu2126-H2K1-L71-H72b-Mu14 retained the property of non-competitive binding to HER2 of their parent antibodies (the chimeric antibodies mAb2117 and mAb2126), a competitive binding assay was performed according to the method described in Section 1.5 of Example 1. The two optimized antibodies, serially diluted from 800 µg/mL, were each mixed with an equal volume of 8 µg/mL biotin-labeled Hu2117-HK304-06 antibody, and then incubated with BT474 cells. AF488-labeled streptavidin (Jackson Immuno) was used as a secondary antibody, and competitive inhibitory activity of these two antibodies on HER2-expressing tumor cells was assessed by flow cytometry. As shown in Figure 10, Hu2117-HK304-06 and Hu2126-H2K1-L71-H72b-Mu14 do not compete for binding of HER2 expressed on the cell surface.

### 4.4 Analysis of internalization and lysosomal trafficking/degradation efficiency of the anti-HER2 biparatopic antibody

In this example, the internalization rate of the aforementioned exemplary anti-HER2 biparatopic antibody in cells expressing different levels of HER2 (including BT474, JIMT-1, and RT-112 cells) was determined using flow cytometry. The specific method is as follows: cells were harvested by trypsinization, washed with ice-cold FACS buffer, and then resuspended to 6-10×10⁶ cells/mL. The cell suspension was transferred to a 96-well U-bottom deep-well plate at 300 µL/well. Then, 300 µL of the testing antibody (final antibody concentration: 20 µg/mL) was added to each well and mixed and incubated on ice for 30-60 minutes. After washing twice with ice-cold FACS buffer, the cells in each well were thoroughly resuspended with 600 µL/well ice-cold FACS buffer, and then the cell suspension was evenly dispensed into 5 replicate 96-well U-bottom plates at 100 µL/well. Within the 5 replicate plates, one plate was designated as the control group and incubated on ice, and the other 4 plates were designated as the experimental groups and incubated at 37°C and each transferred to ice for cooling to terminate the internalization reaction at 30 minutes, 1 hour, 2 hours, and 4 hours, respectively.

At the end of incubation, cells in each well were washed twice with ice-cold FACS buffer and fixed overnight with 100 µL per well of fixative (2% paraformaldehyde) at 4°C. After washing twice with ice-cold FACS buffer, 100 µL per well of AF488-labeled anti-human IgG (H+L) antibody (Jackson Immuno) diluted at 1:1000 in ice-cold FACS buffer was added to each plate. The cells were resuspended and incubated on ice in the dark for 40 minutes. After washing twice with ice-cold FACS buffer, cells were resuspended in 100 µL per well of FACS buffer and analyzed by flow cytometry. The antibody internalization rate (expressed as a percentage) was calculated using the following formula: (MFI_{control} - MFI ₑₓₚₑᵣᵢₘₑₙₜₐₗ) / MFI _{control} × 100%. As shown in Figure 11, compared to the control antibody trastuzumab and the corresponding monospecific antibodies Hu2117-HK304-06 and Hu2126-H2K1-L71-H72b-Mu14, the anti-HER2 biparatopic antibodies 04BS-1123-ST04, 04BS-1123-ST05, 04BS-1123-ST06, and their parent antibody 04BS-109-WT induced more rapid and robust internalization in BT474, JIMT-1, and RT-112 cells, wherein the internalization rates of the biparatopic antibodies reached approximately 60% in JIMT-1 and RT-112 cells, and up to approximately 80% in BT474 cells.

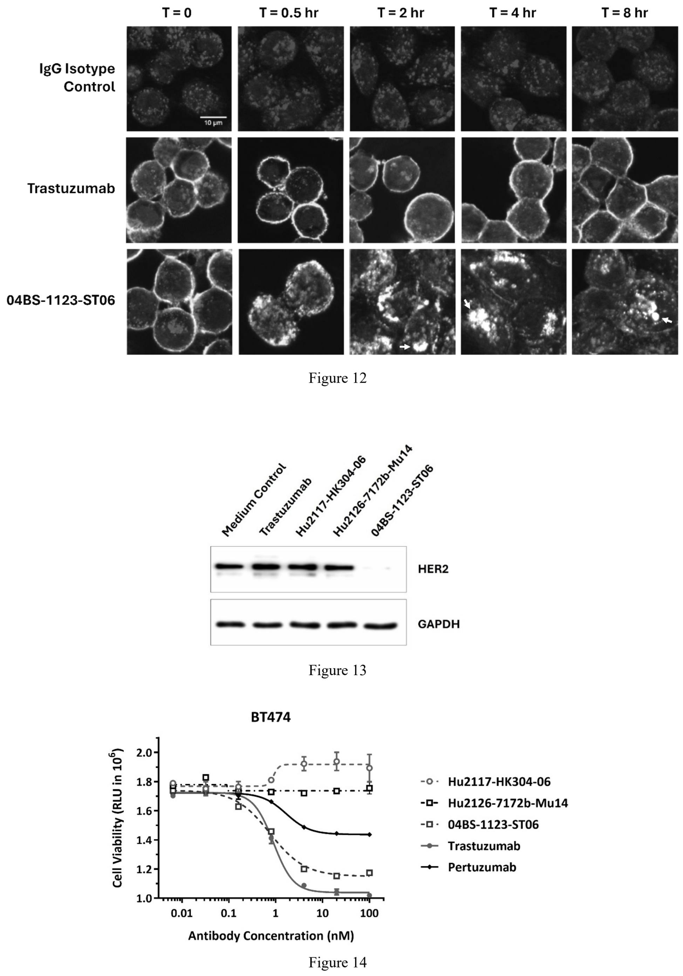

To further verify that the anti-HER2 biparatopic antibody is transported into lysosomes after internalization, the subcellular localization of the antibody was detected using confocal microscopy in this example. The specific method is as follows: SKBR-3 cells were harvested and seeded on five 8-well chamber slides (Thermo). After the cells were grown into a uniform monolayer, the medium was discarded and the cells were rinsed once with PBS; 500 µL of 10 µg/mL anti-HER2 biparatopic antibody 04BS-1123-ST06, the control antibody trastuzumab, or human IgG isotype control antibody (such as the anti-HIV/gp120 antibody described in WO2003106496A1) was added to each well of the chamber slides and incubated on ice for 1 hour. One chamber slide was served as the 0-hour control, which was rinsed once with ice-cold PBS and then fixed with 2% paraformaldehyde. The other 4 slides were incubated at 37°C for 0.5 hours, 2 hours, 4 hours, and 8 hours respectively, and then rinsed once with ice-cold PBS and fixed overnight with 2% paraformaldehyde at 4°C. All slides were rinsed twice with PBST (PBS containing 0.05% Tween-20) and then incubated at room temperature with 250 µL of fixation and permeabilization solution (PBS containing 2% goat serum and 0.5% Triton X-100) for 10 minutes. After rinsing twice with PBST, 250 µL of mouse anti-LAMP1 antibody (1:50 dilution, BD) diluted in FACS buffer (PBS containing 2% FBS) and AF488-labeled goat anti-human IgG antibody (2 µg, Molecular Probes) were added to each slide and incubated at room temperature in the dark for 1 hour. Slides were rinsed twice with PBST and incubated at room temperature in the dark with 250 µL of FACS buffer containing AF647-labeled goat anti-mouse IgG antibody (1:250 dilution, Molecular Probes) for 1 hour. After rinsing twice with PBST, DAPI was added to each slide at a final concentration of 1 µg/mL and stained for 1 minute. Slides were rinsed once with PBST and sealed with the mounting medium (Thermo), and then left at room temperature in the dark overnight. Scanning and analysis were performed using a confocal microscope, and representative images are shown in Figure 12. No green fluorescent signal was detected in cells incubated with the human IgG isotype control antibody at any time point, indicating that there was no nonspecific antibody binding to the cells; green fluorescent signals were detected in cells incubated with the positive control antibody trastuzumab, with no significant difference at any time point, showing a uniform ring-like distribution on the cell membrane and no overlap with the red fluorescence-labeled lysosomes, indicating that trastuzumab can bind to HER2 on SKBR-3 cells but no significant internalization and lysosomal trafficking occurred. In contrast, in cells incubated with the anti-HER2 biparatopic antibody 04BS-1123-ST06, as the treatment time progressed, the green fluorescent signal gradually shifted from the cell membrane to the intracellular compartment and overlapped with the red fluorescence-labeled lysosomes (appearing yellow, as indicated by arrows in the figure), after which the intensity of the yellow fluorescent signal gradually weakened or even disappeared over time, indicating that the internalized anti-HER2 biparatopic antibody 04BS-1123-ST06 was transported into the lysosomes and degraded, with a lysosomal trafficking efficiency of almost 100%.

Detection of HER2 degradation by Western blot further confirmed that, after binding to HER2 on the cell surface, the anti-HER2 biparatopic antibody is internalized and transported into lysosomes for degradation. The specific method is as follows: BT474 cells were harvested and resuspended in complete medium (RPMI-1640 medium containing 10% FBS) to a density of 1×10⁶ cells/mL, and the cell suspension was then aliquoted into 1.5-mL EP tubes at 500 µL per tube. The testing antibodies (including the biparatopic antibody 04BS-1123-ST06, the control antibody trastuzumab, and the monospecific antibodies Hu2117-HK304-06 and Hu2126-H2K1-L71-H72b-Mu14 corresponding to the biparatopic antibody) were diluted to 300 µg/mL with complete medium, and then added to the aforementioned EP tubes at 100 µL/tube (the final antibody concentration: 50 µg/mL). The EP tubes were incubated at 37°C/5% CO₂ for 24 hours. The cells were collected by centrifugation, washed twice with ice-cold PBS, and then lysed in 100 µL of RIPA lysis buffer (Proteintech) by incubating on ice for 30 minutes. After highspeed centrifugation at 4°C for 10 minutes, the supernatant of the cell lysate was collected, and the protein concentration in the extract was quantified using a BCA kit (Thermo). According to the protein concentration, each protein sample was diluted to 0.125 µg/µL with RIPA lysis buffer, and then 5× SDS-PAGE sample buffer containing DTT was added. The mixture was heated at 95°C for 5 minutes to prepare SDS-PAGE loading samples. The loading samples were loaded onto a 10% SDS-PAGE gel at 10 µL per lane (i.e., 1 µg/lane) for electrophoresis. At the end of electrophoresis, the proteins in gel were transferred onto a PVDF membrane at a voltage of 80V. The PVDF membrane was blocked with 5% skim milk at room temperature for 1 hour and then cut into two halves at the line of 55 kDa molecular weight marker. A rabbit anti-human HER2 antibody (CST) diluted at 1:5000 was added to the upper half of the PVDF membrane and incubated at 4°C overnight, and a mouse anti-human GAPDH antibody (Proteintech) diluted at 1:10,000 was added to the lower half of the PVDF membrane and incubated at room temperature for 1 hour. At the end of incubation, the membranes were washed three times with PBST, the goat anti-rabbit IgG-HRP antibody (Jackson Immuno) and goat anti-mouse IgG-HRP antibody (Jackson Immuno) diluted at 1:5000 were added to the corresponding membrane halves and incubated at room temperature for 1 hour. The membranes were washed three times with PBST and then exposed and developed using ECL solution (Vazyme). As shown in Figure 13, the biparatopic antibody 04BS-1123-ST06 induced near-complete degradation of HER2 in BT474 cells, whereas the control antibodies failed to induce HER2 degradation.

The effect of the biparatopic antibody 04BS-1123-ST06 on the proliferation of BT474 cells was also examined in this example. The specific method is as follows: BT474 cells were seeded in 96-well white-wall plates (10,000 cells/well). The testing antibodies (including 04BS-1123-ST06, trastuzumab, pertuzumab, Hu2117-HK304-06, and Hu2126-H2K1-L71-H72b-Mu14) were serially diluted and then added to the plates. The cells were incubated at 37°C/5% CO₂ for 5 days and the detection reagent CellCounting-Lite 2.0 Luminescent Reagent (NanoVizon) was then added. Chemiluminescence values were measured using a multi-function microplate reader (Varioskan, Thermo), and obtained data were analyzed using GraphPad Prism 9 software. As shown in Figure 14, the biparatopic antibody 04BS-1123-ST06 effectively inhibited the proliferation of BT474 cells, whereas the corresponding monospecific antibodies showed no inhibitory activities on BT474 cell proliferation. Since the BT474 cell proliferation is depending on the signaling activation triggered by the homodimers formed by the overexpressed HER2 on cell membrane, it is suggested that the inhibitory activity of the biparatopic antibody on the cell proliferation is attributed to the internalization and degradation of HER2 on cell surface, which suppreses the formation of HER2 homodimers.

### 4.5 Effect of the anti-HER2 biparatopic antibody on NRG-1-induced HER2/HER4 dimerization

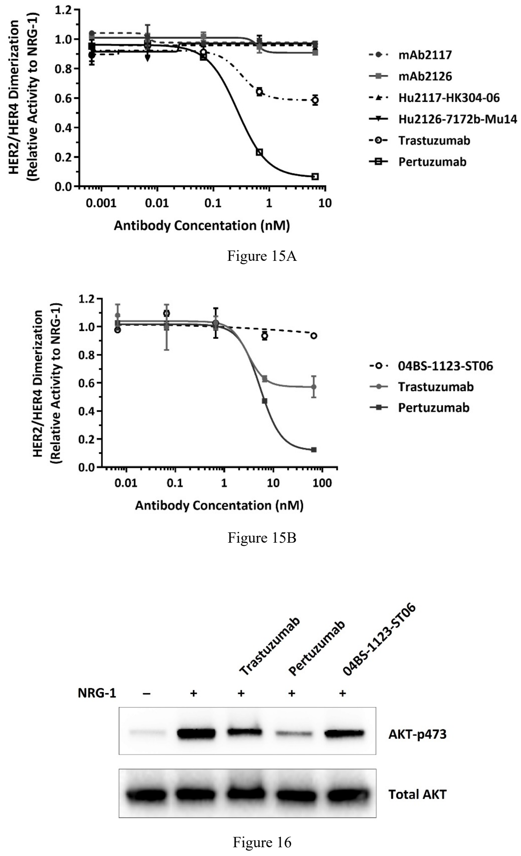

The NanoBiT structural complementation reporter system from Promega was used to further verify whether the anti-HER2 biparatopic antibody affects NRG-1-induced HER2/HER4 dimerization. The NanoBiT system consists of an LgBiT subunit and an SmBiT subunit, which can be fused to two target proteins to be tested, respectively. If the two target proteins interact and form a dimer, the LgBiT and SmBiT subunits will structurally complement to form a functional luciferase, which then reacts with the substrate to generate a luminescent signal. In this example, DNA sequences encoding the extracellular and transmembrane regions of HER2 and HER4 were first inserted into the vectors pBiT1.3-C and pBiT2.3-C of the NanoBiT system using molecular cloning techniques. These two plasmids were then co-transfected into U2OS cells. Cells were harvested 24 hours post-transfection and seeded into a 96-well white-wall plate, followed by overnight culture at 37°C. The biparatopic antibody 04BS-1123-ST06, the humanized antibodies Hu2117-HK304-06 and Hu2126-H2K1-L71-H72b-Mu14, the chimeric antibodies mAb2117 and mAb2126, and the control antibodies trastuzumab and pertuzumab were serially diluted and then mixed with NRG-1 at a volume ratio of 1:1 for use (final concentration of NRG-1 was 1 nM). Nano-Glo Live Cell Reagent (Promega) was added to the 96-well white-wall plate, and the chemiluminescence value was measured as the background. Subsequently, the prepared samples mentioned above were added to each well. After incubation at 37°C for 6 minutes, the chemiluminescence values were measured, and the experimental data were analyzed using GraphPad Prism 9 software. As shown in Figure 15, the control antibodies trastuzumab and pertuzumab significantly inhibited NRG-1-induced HER2/HER4 dimerization. In contrast, the chimeric antibodies mAb2117 and mAb2126, the monospecific humanized antibodies Hu2117-HK304-06 and Hu2126-H2K1-L71-H72b-Mu14 (Figure 15A), and the biparatopic antibody 04BS-1123-ST06 constructed based on these two monospecific humanized antibodies (Figure 15B) all showed no inhibitory effect. This result further indicates that the anti-HER2 biparatopic antibody does not interfere with the normal biological function of HER2, and thus has a very low risk of inducing cardiotoxicity *in vivo.*

### 4.6 Effect of the anti-HER2 biparatopic antibody on NRG-1-induced activation of HER2 downstream signaling pathways

Human induced pluripotent stem cells (iPSCs) and related culture kit were purchased from Fujifilm Cellular Dynamics. According to the manufacturer's instructions, iPSCs were seeded in a 6-well plate and cultured at 37°C with 5% CO₂ for one week to allow fully differentiated into human cardiomyocytes (iCell Cardiomyocytes). The effect of the anti-HER2 biparatopic antibody 04BS-1123-ST06 on NRG-1-induced AKT phosphorylation in human cardiomyocytes was detected using the method described in Section 1.10 of Example 1. As shown in Figure 16, unlike the positive control antibody pertuzumab, 04BS-1123-ST06 had no inhibitory effect on NRG-1-induced AKT phosphorylation in human cardiomyocyte, indicateing that the biparatopic antibody does not interfere with NRG-1-induced HER2 dimerization and the regulation of downstream signaling pathways in cardiomyocytes.

### 4.7 Modification of the Fc-mediated effector function in anti-HER2 biparatopic antibody

To reduce the safety risks associated with Fc-mediated effector functions, three point mutations, L234F, L235E, and P331S (referred to as TM mutations), were introduced into the Fc region of the anti-HER2 biparatopic antibody 04BS-1123-ST06 to decrease the binding activity of the antibody to Fc receptors and C1. ForteBio analysis confirmed that the biparatopic antibody 04BS-1123-ST06 containing the TM mutations showed no significant binding to FcγRI, FcγRIIa (167H), FcγRII, FcγRIIIa (176V), FcγRIIIa (176F), or C1q. In contrast, the binding affinity of the biparatopic antibody to FcRn showed no significant difference compared to the control antibody trastuzumab (data not shown).

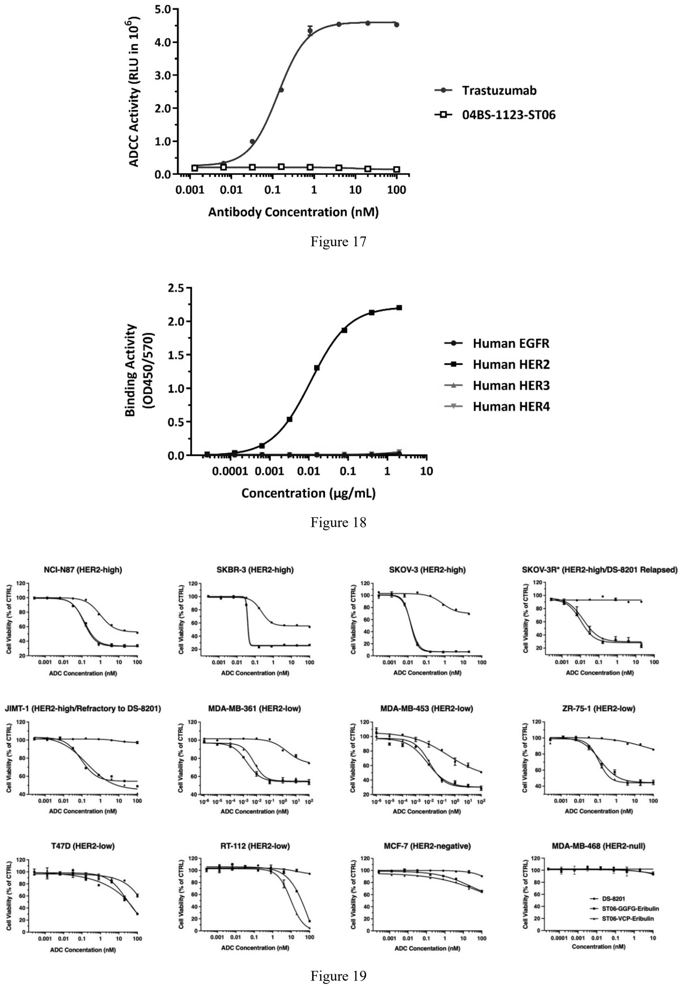

In addition, an ADCC assay was conducted to further verify whether the biparatopic antibody 04BS-1123-ST06 containing the TM mutations possessed ADCC activity, wherein the Jurkat cells expressing the FcγRIIIa-176V receptor and firefly luciferase under the control of NFAT response elements (Promega) were used as effector cells, the HER2-overexpressing BT474 cells were used as target cells, and trastuzumab was served as positive control antibody. The specific method is as follows: BT474 cells were harvested by centrifugation, resuspended in ADCC assay buffer (RPMI-1640 medium containing 0.5% FBS), and added to a 96-well white-wall plate at a density of 1.5×10⁴ cells/well. The testing antibodies were serially diluted in ADCC assay buffer and added to the 96-well plate at 25 µL/well. Effector cells were harvested by centrifugation, resuspended in ADCC assay buffer, and added to the aforementioned 96-well plate at a density of 1.5×10⁵ cells/well, and the cells were then incubated overnight at 37°C and 5% CO₂. The next day, the 96-well plate was equilibrated to room temperature and the detection reagent Bio-Lite Luciferase Reagent (Vazyme, Nanjing) was then added. Chemiluminescence values were measured using a multi-function microplate reader (Varioskan, Thermo), and obtained data were analyzed using GraphPad Prism 9 software. As shown in Figure 17, unlike the positive control antibody trastuzumab, 04BS-1123-ST06 did not induce any luciferase expression even at the highest concentration, indicating that the anti-HER2 biparatopic antibody with TM mutations introduced into the Fc region does not have ADCC activity.

### Example 5 Preparation and Functional Activity Characterization of Anti-HER2 Biparatopic ADC

### 5.1 Preparation of the anti-HER2 biparatopic antibody-drug conjugate

This example describes the preparation of anti-HER2 biparatopic ADC through conjugation of the anti-HER2 biparatopic antibody 04BS-1123-ST06 to eribulin, a cytotoxic small-molecule compound with antitumor activity, via a cleavable linker (e.g., Mal-(PEG)₂-VC-PAB or Mal-(PEG)₂-GGFG). Eribulin was prepared according to the methods described in ZL201910197071.8 or ZL201910509222.9, and WO1999065894.

The payload Mal-(PEG)₂-VC-PAB-Eribulin was prepared according to the method described in Example 2.1.1 of WO2017151979.

The preparation method of the payload Mal-(PEG)₂-GGFG-Eribulin is as follows: NH2-(PEG)₂-COOH and maleic anhydride were mixed in acetic acid, heated under reflux overnight, and then the acetic acid was removed by rotary evaporation. The residue was purified by HPLC and lyophilized to obtain Mal-(PEG)₂-COOH. Using 2Cl Trt Resin as the solid-phase carrier, the Fmoc protecting group was removed with 20% piperidine/DMF (v/v). With HOBT/DIC as the condensation system and DMF as the reaction solvent, Fmoc-Gly-OH, Fmoc-Phe-OH, Fmoc-Gly-OH, and Fmoc-Gly-OH were sequentially coupled. The product was then cleaved in a solution of DCM:TFE:AcOH = 7:2:1, precipitated with methyl tert-butyl ether, centrifuged, dried, and further purified by HPLC to obtain Fmoc-GGFG-OH. Fmoc-GGFG-OH and eribulin were placed in a single-necked flask, dissolved in DMF, and cooled to 0°C. DIPEA (161.3 mg, 1.25 mmol) and DECP (122.3 mg, 0.75 mmol) were added, and the reaction was carried out at room temperature and monitored by HPLC. After completion, the reaction solution was poured into MTBE (100 mL), stirred at room temperature for 1 hour, filtered, and the filter cake was washed with MTBE to obtain crude Fmoc-GGFG-Eribulin. The crude Fmoc-GGFG-Eribulin was placed in a single-necked flask, added with THF, and cooled to 0°C. Two equivalents of LiOH aqueous solution were added, and the reaction was carried out at room temperature, which was monitored by HPLC. After completion, the pH was adjusted to 6-7 with 50% acetic acid. THF was removed by rotary evaporation, and the product was purified by HPLC to obtain pure NH2-GGFG-Eribulin. HATU was added to a DMF solution containing Mal-(PEG)₂-COOH and sodium bicarbonate at room temperature. After stirring for 30 minutes, an equivalent amount of free NH₂-GGFG-Eribulin was added to the solution and stirred for another hour until the reaction was complete as determined by TLC. The solid was removed by filtration, and the filtrate was purified by HPLC to obtain Mal-(PEG)₂-GGFG-Eribulin.

The preparation method of the anti-HER2 biparatopic ADC is as follows: ZnCl₂ (3.0 mM) and TCEP (6.0 mM) were slowly added to the anti-HER2 biparatopic antibody 04BS-1123-ST06 (1.5 mM, fomulated in 50 mM Histidine-HCl, 8% sucrose, pH 5.5) in an ice-water bath. The final reaction concentrations of ZnCl₂, TCEP, and the 04BS-1123-ST06 antibody were 0.10 mM, 0.20 mM, and 0.05 mM, respectively. After mixing, the mixture was reacted by shaking at 8°C for approximately 16 hours. Mal-(PEG)₂-VC-PAB-Eribulin or Mal-(PEG)₂-GGFG-Eribulin dissolved in DMSO was added to a final concentration of 0.32 mM in an ice-water bath. After the reaction was continued at 8°C for 3 hours, cysteine was added to a final concentration of 0.05 mM to exhaust any excess Mal-(PEG)₂-VC-PAB-Eribulin or Mal-(PEG)₂-GGFG-Eribulin. EDTA was then added to a final concentration of 0.15 mM to chelate Zn²⁺. Related impurities were removed using a Pellicon XL Ultrafiltration membrane filter (Module Ultracel 30 kDa/0.005 m², purchased from Merck). Finally, the drug-to-antibody ratio (DAR) and heterogeneity of the prepared ADC were analyzed by HIC-HPLC (BioCore HIC-Butyl 5 µm/4.6 × 100 mm, purchased from NanoChrom), and the purity of the ADC product was analyzed by SEC-HPLC (Zenix-C SEC-300, purchased from Sepax Technologies).

The average DAR value of the prepared ADC can be calculated using the following formula: Average DAR = [AUC₊₁ + 2(AUC₊₂) + 3(AUC₊₃) + ... + n(AUC₊ₙ)] /ΣAUCₜₒₜₐₗ], wherein AUC₊₁ refers to the area under the curve of the peak corresponding to the ADC conjugated with one small-molecule compound; AUC₊₂ refers to the area under the curve of the peak corresponding to the ADC conjugated with two small-molecule compound, and so on; Σ AUCₜₒₜₐₗ represents the total sum of the area under the curve of all peaks. The average DAR value of the ADC prepared in this example was 4.0 + 0.5, with a purity of >95%.

### 5.2 Verification of antigen-binding specificity of the anti-HER2 biparatopic ADC

According to the method described in Section 1.4 of Example 1, the binding activity of the exemplary anti-HER2 biparatopic ADC (ST06-VCP-Eribulin) to the recombinant HER2 protein and its cross-reactivity to the other members of the human ErbB/HER family were verified by ELISA, wherein the antigens included the recombinant proteins of human HER2, EGFR, HER3, and HER4 extracellular domains. As shown in Figure 18, the anti-HER2 biparatopic ADC (ST06-VCP-Eribulin) can specifically bind to HER2 and exhibits no cross-reactivity to the other members of the human ErbB/HER family. The antigen-binding specificity of the anti-HER2 biparatopic ADC was identical to its corresponding unconjugated antibody 04BS-1 123-ST06 (data not shown), indicating that conjugation with the small-molecule toxin compound did not alter the antigen-binding specificity of the biparatopic antibody.

### 5.3 Detection of in vitro tumor cell-killing activity of anti-HER2 biparatopic ADC

In this example, a panel of tumor cell lines expressing varying levels of HER2 were utilized to evaluate the *in vitro* cell-killing activity of anti-HER2 biparatopic ADC. The HER2 expression level and drug resistance status of the tumor cell lines used in the assay are presented in Table 13, wherein JIMT-1 is a cell line with HER2 overexpression and exhibits intrinsic resistance to DS-8201 (*in vitro* activity), and SKOV-3R* is a cell line with HER2 overexpression and exhibits acquired resistance to DS-8201.

The procedures for examining the *in vitro* tumor cell-killing activity are as follows: Cells were harvested by trypsinization, resuspended in medium containing 10% FBS, and then seeded in 96-well white-wall plates at a density of 5,000 or 10,000 cells per well. Cells were cultured in an incubator at 37°C with 5% CO₂. After the cells had fully adhered, ST06-VCP-Eribulin, ST06-GGFG-Eribulin, or DS-8201 serially diluted in the corresponding culture medium were added to the wells. Depending on the growth rate of each cell line, the plates were incubated at 37°C and 5% CO₂ for 3 to 5 days. At the end of incubation, the cell culture plates were taken out of the incubator and allowed to equilibrate to room temperature, and CellCount Lite 2.0 reagent (Vazyme, Nanjing) was added and the plates were then shaken for 2 to 5 minutes to fully lyse the cells. After incubation at room temperature for 10 minutes, chemiluminescence values were measured using a multi-function microplate reader (Varioskan, Thermo). Data were analyzed using GraphPad Prism 9 software and results were expressed as a percentage value relative to the chemiluminescence value of the untreated control wells.

The results are summarized in Figure 19 and Table 13. In HER2-overexpressing tumor cell lines (NCI-N87, SKBR-3, and SKOV-3), the anti-HER2 biparatopic ADCs (including ST06-GGFG-Eribulin and ST06-VCP-Eribulin) and the benchmark molecule DS-8201 exhibited potent cytotoxicity, with the biparatopic ADCs exhibting significantly higher cell-killing activity than DS-8201. In HER2-low-expressing tumor cell lines (MDA-MB-361 and MDA-MB-453), the cell-killing activity of the biparatopic ADCs was markedly higher than that of DS-8201. In the HER2-low-expressing cell line ZR-75-1, the biparatopic ADCs demonstrated effective cell-killing activity, but DS-8201 showed almost no cytotoxic effect. In tumor cell lines expressing even lower levels of HER2 (T47D, RT-112, MCF-7) and in the HER2-null tumor cell line MDA-MB-468, the biparatopic ADCs showed no significant cytotoxicity, indicating a favorable safety window for the biparatopic ADCs. Additionally, in JIMT-1 cells, a cell line with over-expressing HER2 and innate/intrinsic resistance to DS-8201, the biparatopic ADCs (including ST06-GGFG-Eribulin and ST06-VCP-Eribulin) also exhibited significant cytotoxicity. Therefore, the anti-HER2 biparatopic ADCs possess a broader spectrum of cell-killing activity, capable of killing not only HER2-overexpressing tumor cells but also those with low HER2 expression, and even the tumor cells unresponsive to DS-8201.

To further validate whether the anti-HER2 biparatopic ADC can kill tumor cells that have developed resistance or are relapsed/refractory to the DS-8201 treatment, a tumor cell line with acquired resistance to DS-8201 (named SKOV-3R*) was isolated in this example by maintaining and passaging HER2-overexpressing SKOV-3 cells in a culture medium with a gradually increasing concentration of DS-8201, until the cells could proliferate at a normal rate in the culture medium containing 100 nM DS-8201. *In vitro* cytotoxicity assay showed that the biparatopic ADCs (including ST06-GGFG-Eribulin and ST06-VCP-Eribulin) could effectively kill the SKOV-3R* cells which had acquired drug-resistant.

**Table 13. In Vitro Cytotoxicity of Anti-HER2 Biparatopic ADC in Comparison to DS-8201**

| Tumor Cell | Cancer Type | HercepTest ^{®} / FISH | HER2 Expression Level on the Cell Surface | In Vitro Cytotoxicity (EC50, Nm) | | |
|---|---|---|---|---|---|---|
| | | | | DS-8201 | ST06-GGFG-Eribulin | ST06-VCP-Eribulin |
| NCI-N87 | Gastric | 3⁺ | 1,493,288 | 0.9841 | 0.1337 | 0.1273 |
| SKBR-3 | Breast | 3⁺ | 1,105,178 | 0.2103 | 0.0376 | 0.0387 |
| SKOV-3 | Ovarian | 3⁺ | 781,484 | 0.7408 | 0.0135 | 0.0140 |
| MDA-MB-361 | Breast | 2⁺ | 378,707 | 2.099 | 0.0019 | 0.0062 |
| SKOV-3R* | Ovarian | 2⁺ | 271,851 | - | 0.0104 | 0.0143 |
| JIMT-1 | Breast | 2⁺ / FISH⁺ | 167,741 | - | 0.0873 | 0.1754 |
| MDA-MB-453 | Breast | 2⁺ | 148,810 | 0.292 | 0.0079 | 0.0115 |
| ZR-75-1 | Breast | 1⁺ | 104,577 | - | 0.1292 | 0.1094 |
| T47D | Breast | 1⁺ | 81,195 | - | - | - |
| RT-112 | Bladder | 1⁺ | 46,068 | - | - | - |
| MCF-7 | Breast | 0 | 29,169 | - | - | - |
| MDA-MB-468 | Breast | Not expressing | Not detected | - | - | - |

| | | | | | | |
|---|---|---|---|---|---|---|
| -: inactive. | | | | | | |

### 5.4 Bystander killing activity of anti-HER2 biparatopic ADC

The bystander killing activity of the anti-HER2 biparatopic ADC ST06-GGFG-Eribulin was evaluated using the HER2-overexpressing cell line BT474 and the HER2-null cell line MDA-MB-468. The specific method was as follows: On Day 0, BT474 cells were seeded at 10,000 cells/100 µL/well in a 96-well white-wall plate and cultured overnight at 37°C. On Day 1, ST06-GGFG-Eribulin was diluted to 20 nM in culture medium, followed by 5-fold serial dilutions, and then added to the plate at 100 µL/well (i.e., the starting concentration of 10 nM). The plate was incubated at 37°C. On Day 3, MDA-MB-468 cells were seeded at 10,000 cells/well in a new 96-well white-wall plate and cultured overnight at 37°C. On Day 4, the MDA-MB-468 cell culture medium was removed and replaced with 100 µL/well of the conditioned media collected from the BT474 cell culture plate to assess the bystander effect of the ADC. Simultaneously, freshly prepared ST06-GGFG-Eribulin with 5-fold serial dilution starting from 10 nM was added to MDA-MB-468 cells at 100 µL/well as a control. In addition, BT474 cell cultures from Day 4 were assayed for cell viability using CellCount Lite 2.0 reagent. The plates with MDA-MB-468 cells were incubated at 37°C for 72 hours, and the CellCount Lite 2.0 reagent was added to detect the cell viability. Obtained data were analyzed using GraphPad Prism 9 software. As shown in Figure 20A, ST06-GGFG-Eribulin caused significant cell killing in BT474 cells after 3 days of treatment, and MDA-MB-468 cultured with the BT474-conditioned medium also exhibited significant cell death, whereas the freshly prepared ADC solution had no cytotoxic activity against MDA-MB-468 cells. These results suggest that, while ST06-GGFG-Eribulin kills BT474 cells, the small-molecule toxin compound eribulin released into the BT474 cell culture medium is capable of killing the HER2-null MDA-MB-468 cells.

Additionally, the bystander killing activity of ST06-GGFG-Eribulin was further verified by co-culturing BT474 cells and HER2-null Jurkat cells in this example. The specific method is as follows: BT474 cells and GFP-expressing Jurkat cells were harvested, and both cell types were seeded in a 24-well cell culture plate at 1×10⁵ cells/well, eigher in separate wells or in the same well. ST06-GGFG-Eribulin was added to a final concentration of 1 nM. After incubation at 37°C for 72 hours, the cells in each well were collected in flow cytometry tubes. After centrifugation and supernatant removal, the cells were resuspended in 100 µL FACS buffer (PBS containing 1% BSA), and 3 µL of 7-AAD was added for dead cell staining, followed by flow cytometric analysis. For each tube, 10 µL of the cell suspension was collected and analyzed. After excluding 7-AAD-positive dead cells, BT474 and Jurkat cells were distinguished using the side scatter channel and the GFP channel. Finally, the bystander activity of the biparatopic ADC was assessed by comparing the viable cell counts after treated with either ST06-GGFG-Eribulin or blank culture medium (i.e., a control group, designated as "Medium"). As shown in Figure 20B, ST06-GGFG-Eribulin demonstrated significant cytotoxic activity against BT474 cells but showed no cytotoxicity against Jurkat cells in mono-cultures. However, ST06-GGFG-Eribulin caused significant killing of both BT474 and Jurkat cells in co-cultures of the two cell types.

In summary, the results of both experiments demonstrate that ST06-GGFG-Eribulin exhibits a bystander killing activity.

### Example 6 Evaluation of Antitumor Activity of Anti-HER2 Biparatopic ADC in Mouse Subcutaneous Xenograft Models

### 6.1 Antitumor activity of anti-HER2 biparatopic ADC in mouse subcutaneous xenograft models established with tumor cell lines

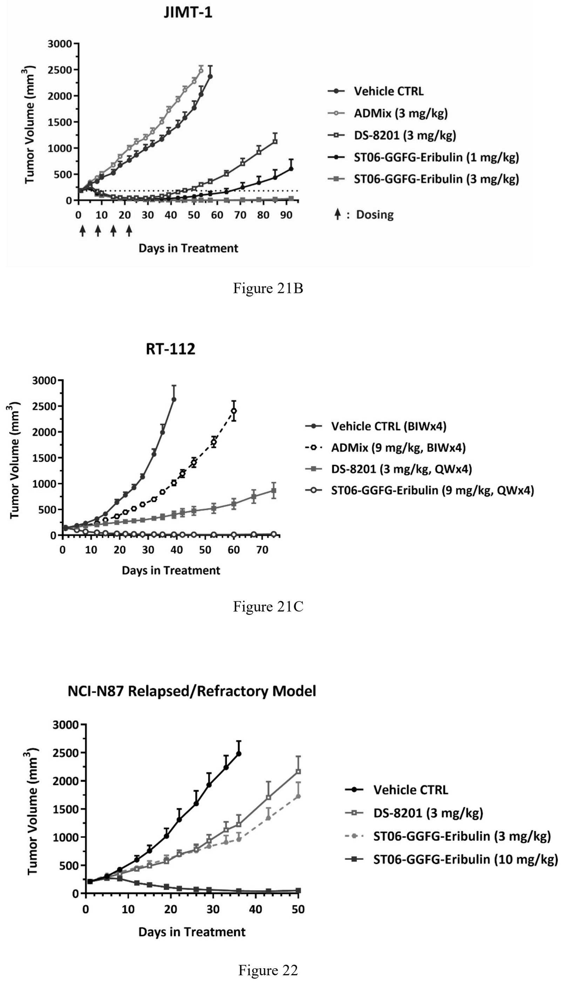

The antitumor activity of the anti-HER2 biparatopic ADC was evaluated in three mouse subcutaneous xenograft tumor models established using human tumor cell lines in this example, wherein DS-8201 (brand name Enhertu, purchased from Daiichi Sankyo) was used as the reference control drug. The three tumor models were as follows: a HER2-overexpressing NCI-N87 gastric cancer model, a HER2-intermediate-expressing JIMT-1 breast cancer model, and a HER2-low-expressing RT-112 bladder cancer model. The specific method is as follows: After the cell lines (NCI-N87, JIMT-1, and RT-112) were cultured to the logarithmic growth phase, cells were harvested and inoculated subcutaneously into the dorsal right forelimb of immunodeficient mice (Nude or SCID) at a density of 5-10×10⁶ cells per mouse. When tumors reached a volume of 200±50 mm³, the mice were randomized into groups (8 tumor-bearing mice per group), including the ST06-GGFG-Eribulin test group, the DS-8201 reference control group, the vehicle control group (50 mM Histidine-HCl solution containing 8% sucrose, pH 5.5), and the antibody-small molecule drug mixture ADMix control group (ADMix was prepared by mixing the unconjugated antibody 04BS-1123-ST06 and eribulin at a molar ratio of 1:4). All tumor-bearing mice were dosed via intravenous injection. The dose and dosing frequency for each group in different models are shown in Figure 21. After administration, the body weight and tumor dimensions (length and width) of the mice were measured and recorded twice weekly. Tumor volumes were calculated using the following formula: tumor volume = (length) × (width)² × 0.5.

As shown in Figure 21, compared to the vehicle control, both DS-8201 and ST06-GGFG-Eribulin demonstrated significant tumor growth inhibition activities in the NCI-N87 (Figure 21A), JIMT-1 (Figure 21B), and RT-112 (Figure 21C) tumor models. Furthermore, the antitumor activity of ST06-GGFG-Eribulin showed a dose-dependent trend in both NCI-N87 and JIMT-1 models. At the same dose level (3 mg/kg), ST06-GGFG-Eribulin exhibited an antitumor activity comparable to DS-8201 in the HER2-overexpressing NCI-N87 model, but demonstrated significantly stronger antitumor activity than DS-8201in the HER2-intermediate-expressing JIMT-1 model (p < 0.01). In the HER2-low-expressing RT-112 model, considering the differences in molecular weight and DAR value, ST06-GGFG-Eribulin showed markedly superior antitumor activity to DS-8201 when administered at a DAR-equivalent dose (9 mg/kg), and the difference in antitumor activity was statistically significant (p < 0.01). No body weight loss or other adverse toxic side effects were observed in the tumor-bearing mice throughout the study (data not shown).

### 6.2 Antitumor activity of anti-HER2 biparatopic ADC in a mouse subcutaneous xenograft tumor model with acquired resistance to DS-8201

To further verify whether the anti-HER2 biparatopic ADC is capable of killing tumors that have developed resistant or relapsed from DS-8201 treatment, a mouse xenograft tumor model with acquired resistance to DS-8201 was established using the NCI-N87 tumor cell line in this example. The method is as follows: Wild-type NCI-N87 cells were maintained and passaged in culture medium containing gradually increasing concentrations of DS-8201, and a subpopulation of cell lines with certain resistance to DS-8201 was isolated and designated as NCI-N87(R). NCI-N87(R) cells were expanded and cultured in RPMI-1640 medium containing 10% serum until reaching the exponential growth phase, and then harvested and resuspended in a mixture of PBS and Matrigel (1:1 volume ratio). BALB/c Nude mice were inoculated subcutaneously with 1×10⁷ cells/mouse at the dorsal right forelimb, with five female mice inoculated at each time. *In vivo* screening of tumors with acquired drug-resistance was performed as follows: When the tumor volume reached 200+50 mm³, DS-8201 (3 mg/kg) was dosed via intravenous injection once weekly to screen for tumor-bearing mice with tumors continuing to grow stably after dosing, wherein the given number of doses was 3-5, depending on the tumor growth rate. When tumors reached to the range of 900-1000 mm³, mice were euthanized and tumors were harvested. After removing necrotic tissue, the tumors were cut into small pieces of approximately 30 mm³ and inoculated subcutaneously into the dorsal side near the right forelimb of five new female Nude mice for the next round of DS-8201 treatment and screening. After three rounds of screening, tumors in all five tumor-bearing mice showed stable growth after administration of DS-8201 (at a dose of 3 mg/kg), with a relatively consistent growth pace. When tumors reached to approximately 1000 mm³, two mice were euthanized and the tumors were harvested, and the tumors were cut into approximately 30 mm³ pieces after necrotic tissue was removed, and then inoculated subcutaneously into the dorsal side near the right forelimb of 50 female Nude mice.

From these 50 tumor-bearing mice, 29 mice with relatively uniform tumor growth and an average volume of approximately 210 mm³ were selected and randomized into groups, including two ST06-GGFG-Eribulin treatment groups (3 mg/kg dose group and 10 mg/kg dose group), one DS-8201 control group (at dose of 3 mg/kg), and one vehicle control group. The ST06-GGFG-Eribulin 3 mg/kg treatment group, the DS-8201 control group, and the vehicle control group consisted of eight mice each, and the ST06-GGFG-Eribulin 10 mg/kg treatment group consisted of five mice. Mice in each group were administered via intravenous injection for a total of four weeks, wherein the DS-8201 group and the vehicle group were administered once weekly, and the ST06-GGFG-Eribulin treatment groups were administered once in the first week and then twice weekly for the remaining 3 weeks. Mouse body weight and tumor dimensions were measured and recorded twice weekly for each group. Tumor volumes were calculated using the following formula: Tumor Volume = (length) × (width)² × 0.5.

As shown in Figure 22, mouse tumors exhibited a continued and stable growth after DS-8201 treatment, indicating the successful establishment of model, i.e., the tumor model was confirmed to be a xenograft model with acquired resistance to DS-8201. ST06-GGFG-Eribulin demonstrated antitumor activity at both 3 mg/kg and 10 mg/kg doses; particularly, at the 10 mg/kg dose (equivalent to the DAR of DS-8201), ST06-GGFG-Eribulin induced a near-complete tumor regression, indicating that the biparatopic ADC (ST06-GGFG-Eribulin) could overcome the acquired resistance to DS-8201. These results suggest a therapeutic potential of the biparatopic ADC in treating the tumors relapsed/refractory from DS-8201 therapy. No body weight loss or other adverse toxic side effects were observed in the tumor-bearing mice during the study (data not shown).

## Claims

1. An isolated anti-HER2 antibody or antigen-binding fragment thereof, comprising at least one of the following characteristics:
(1) the anti-HER2 antibody or antigen-binding fragment thereof is capable of specifically binding to subdomains 1, 3, and/or 4 of the HER2 extracellular region;
(2) the anti-HER2 antibody or antigen-binding fragment thereof does not affect HER2 and downstream signaling pathways mediated thereby, including not inducing, blocking or inhibiting HER2-tyrosine residue phosphorylation and/or dephosphorylation, and/or not inducing, blocking or inhibiting ligand-dependent or ligand-independent HER2 dimerization and its downstream signaling pathways, wherein the HER2 dimerization includes HER2/HER4 dimerization and HER2/HER3 dimerization, and the ligands include NRG-1 and heregulins; and
(3) the anti-HER2 antibody or antigen-binding fragment thereof does not cross-react with other members of the human ErbB/HER family (including EGFR, HER3, and HER4).

2. The anti-HER2 antibody or antigen-binding fragment thereof of claim 1, wherein the anti-HER2 antibody or antigen-binding fragment thereof is capable of specifically binding to subdomain 3 of the HER2 extracellular region, comprising: (1) HCDR1 having an amino acid sequence of X₁YGMS (SEQ ID NO: 217), wherein X₁=S, N, or D; (2) HCDR2 having an amino acid sequence of SISGX₂GX₃YX₄KYX₃X₆X₇VKG (SEQ ID NO: 218), wherein X₂=G or S; X₃=S or N; X₄= T or A; X₅=P, A, G, or V; X₆ = D, G, E, P, Q, or R; X₇ = S, K, or N; (3) HCDR3 having an amino acid sequence of DYX₈GFFDV (SEQ ID NO: 219), wherein Xs = A, I, N, R, S, or V; (4) LCDR1 having an amino acid sequence of RSSQSLX₉X₁₀SNX₁₁NTYLH (SEQ ID NO: 220), wherein X₉ = V or L; X₁₀ = H or S; X₁₁ = G, A, I, S, R, or T; (5) LCDR2 having an amino acid sequence of KVSNRX₁₂S (SEQ ID NO: 221), wherein X₁₂ = F, D, or P; (6) LCDR3 having an amino acid sequence X₁₃QSTHVPX₁₄T (SEQ ID NO: 222), wherein X₁₃ = S or Q; X₁₄ = Y or W, or amino acid sequences having at least 85%, 86%, 87%, 88%, 89%, 90%, 91%, 92%, 93%, 94%, 95%, 96%, 97%, 98%, 99% or 100% identical to the HCDR1, HCDR2, HCDR3, LCDR1, LCDR2, and LCDR3, respectively.

3. The anti-HER2 antibody or antigen-binding fragment thereof of claim 1 or 2, comprising: (1) HCDR1 as set forth in SEQ ID NO: 11, 12, or 13; (2) HCDR2 as set forth in SEQ ID NO: 14, 15, 16, 17, 18, 19, 20, 21, 22, 23, 24, 25, 26, 27, 28, 29, 30, or 31; and (3) HCDR3 as set forth in SEQ ID NO: 32, 33, 34, 35, 36, or 37; and (4) LCDR1 as set forth in SEQ ID NO: 38, 39, 40, 41, 42, 43, 44, or 45; (5) LCDR2 as set forth in SEQ ID NO: 46, 47, or 48; and (6) LCDR3 as set forth in SEQ ID NO: 49, 50, or 51;
furthermore, the anti-HER2 antibody or antigen-binding fragment thereof comprising:
(1) HCDR1 as set forth in SEQ ID NO: 11, HCDR2 as set forth in SEQ ID NO: 14, 15, 16, 17, 18, 19, or 20, HCDR3 as set forth in SEQ ID NO: 32, and LCDR1 as set forth in SEQ ID NO: 38, LCDR2 as set forth in SEQ ID NO: 46 (wherein X₁₂=F), LCDR3 as set forth in SEQ ID NO: 49; or
(2) HCDR1 as set forth in SEQ ID NO: 12 or 13, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 46, LCDR3 as set forth in SEQ ID NO: 49; or
(3) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 47 or 48, LCDR3 as set forth in SEQ ID NO: 49; or
(4) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 39, 40, 41, 42, 43, 44, or 45, LCDR2 as set forth in SEQ ID NO: 46, LCDR3 as set forth in SEQ ID NO: 49; or
(5) HCDR1 as set forth in SEQ ID NO: 11, HCDR2 as set forth in SEQ ID NO: 14, HCDR3 as set forth in SEQ ID NO: 32; and LCDR1 as set forth in SEQ ID NO: 38, LCDR2 as set forth in SEQ ID NO: 46, LCDR3 as set forth in SEQ ID NO: 50 or 51; or
(6) HCDR1 as set forth in SEQ ID NO: 11, HCDR2 as set forth in SEQ ID NO: 14, HCDR3 as set forth in SEQ ID NO: 33, 34, 35, 36, or 37; and LCDR1 as set forth in SEQ ID NO: 38, LCDR2 as set forth in SEQ ID NO: 46, LCDR3 as set forth in SEQ ID NO: 49; or
(7) HCDR1 as set forth in SEQ ID NO: 11, HCDR2 as set forth in SEQ ID NO: 14, 21, 22, 23, 24, 25, 26, 27, 28, 29, 30, or 31, HCDR3 as set forth in SEQ ID NO: 37; and LCDR1 as set forth in SEQ ID NO: 42, LCDR2 as set forth in SEQ ID NO: 46, LCDR3 as set forth in SEQ ID NO: 49; or
(8) HCDR1 as set forth in SEQ ID NO: 11, HCDR2 as set forth in SEQ ID NO: 21, HCDR3 as set forth in SEQ ID NO: 32; and LCDR1 as set forth in SEQ ID NO: 42, LCDR2 as set forth in SEQ ID NO: 46, LCDR3 as set forth in SEQ ID NO: 49; or
(9) HCDR1 as set forth in SEQ ID NO: 11, HCDR2 as set forth in SEQ ID NO: 21, 23, 24, 27, 28, 29, 30 or 31, HCDR3 as set forth in SEQ ID NO: 37; and LCDR1 as set forth in SEQ ID NO: 42, LCDR2 as set forth in SEQ ID NO: 46, LCDR3 as set forth in SEQ ID NO: 49; or
(10) HCDR1 as set forth in SEQ ID NO: 11, HCDR2 as set forth in SEQ ID NO: 24, 29, 30 or 31, HCDR3 as set forth in SEQ ID NO: 37; and LCDR1 as set forth in SEQ ID NO: 42, LCDR2 as set forth in SEQ ID NO: 46, LCDR3 as set forth in SEQ ID NO: 49.

4. The anti-HER2 antibody or antigen-binding fragment thereof of claim 1, wherein the anti-HER2 antibody or antigen-binding fragment thereof is capable of specifically binding to subdomain 1 of the HER2 extracellular region, comprising: (1) HCDR1 having an amino acid sequence of DYX₁₅MH (SEQ ID NO: 223), wherein X₁₅=S or A; (2) HCDR2 having an amino acid sequence of WINTX₁₆TGX₁₇PTYADX₁₈X₁₉KG (SEQ ID NO: 224), wherein X₁₆=E, N, G, Y or I; X₁₇=E, D or S; X₁₈=D, K or N; X₁₉=F or V; (3) HCDR3 having an amino acid sequence of VGX₂₀X₂₁X₂₂YAMDY (SEQ ID NO: 225), wherein X₂₀=R or Y; X₂₁=Y or G; X₂₂=D or S; (4) LCDR1 having an amino acid sequence of X₂₃ASQDVYTAVA (SEQ ID NO: 226), wherein X₂₃=K or R; (5) LCDR2 having an amino acid sequence of X₂₄ASX₂₅RX₂₆T (SEQ ID NO: 227), wherein X₂₄=S, A, D, E, K, L, Q, R, W or Y; X₂₅=Y, D, E, K, N, Q, S or T; X₂₆=Y, A, E, P or Q; (6) LCDR3 having an amino acid sequence QQX₂₇YSTPPT (SEQ ID NO: 228), wherein X₂₇=H, S, A or Y, or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

5. The anti-HER2 antibody or antigen-binding fragment thereof of claim 1 or 4, comprising: HCDR1 as set forth in SEQ ID NO: 98 or 99; HCDR2 as set forth in SEQ ID NO: 100, 101, 102, 103, 104, 105, 106, 107, 108, or 109; HCDR3 as set forth in SEQ ID NO: 110, 111, 112, or 113; and LCDR1 as set forth in SEQ ID NO: 114 or 115; LCDR2 as set forth in SEQ ID NO: 116, 117, 118, 119, 120, 121, 122, 123, 124, 125, 126, 127, 128, 129, 130, 131, 132, 133, 134, 135, 136, 137, or 138; and LCDR3 as set forth in SEQ ID NO: 139, 140, 141, or 142;
furthermore, the anti-HER2 antibody or antigen-binding fragment thereof comprising:
(1) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 114, LCDR2 as set forth in SEQ ID NOs: 116, 117, 118, 119, 120, 121, 122, 123, 124, 125, 126, 127, or 128, LCDR3 as set forth in SEQ ID NO: 139; or
(2) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 140, 141, or 142; or
(3) HCDR1 as set forth in SEQ ID NO: 98, HCDR2 as set forth in SEQ ID NO: 101, 102, 103, 104, 105, 106, 107, 108, or 109, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 139; or
(4) HCDR1 as set forth in SEQ ID NO: 98, HCDR2 as set forth in SEQ ID NO: 100, HCDR3 as set forth in SEQ ID NO: 111, 112, or 113, LCDR1 as set forth in SEQ ID NO: 114, LCDR2 as set forth in SEQ ID NO: 116, LCDR3 as set forth in SEQ ID NO: 139; or
(5) HCDR1 as set forth in SEQ ID NO: 98, HCDR2 as set forth in SEQ ID NO: 100, HCDR3 as set forth in SEQ ID NO: 110, LCDR1 as set forth in SEQ ID NO: 115, LCDR2 as set forth in SEQ ID NO: 116, LCDR3 as set forth in SEQ ID NO: 139; or
(6) HCDR1 as set forth in SEQ ID NO: 99, HCDR2 as set forth in SEQ ID NO: 100, HCDR3 as set forth in SEQ ID NO: 110, LCDR1 as set forth in SEQ ID NO: 114, LCDR2 as set forth in SEQ ID NO: 116, LCDR3 as set forth in SEQ ID NO: 139; or
(7) HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 130, 131, 132, 133, 134, 135, 136, 137, or 138, LCDR3 as set forth in SEQ ID NO: 142;
preferably, the anti-HER2 antibody or antigen-binding fragment thereof comprising: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 133, 136, or 138, LCDR3 as set forth in SEQ ID NO: 142.

6. The anti-HER2 antibody or antigen-binding fragment thereof of claim 1, wherein the anti-HER2 antibody or antigen-binding fragment thereof is capable of specifically binding to subdomain 4 of the HER2 extracellular region, comprising: HCDR1 as set forth in SEQ ID NO: 199, HCDR2 as set forth in SEQ ID NO: 200, HCDR3 as set forth in SEQ ID NO: 201, LCDR1 as set forth in SEQ ID NO: 202, LCDR2 as set forth in SEQ ID NO: 203, LCDR3 as set forth in SEQ ID NO: 204; or
wherein the anti-HER2 antibody or antigen-binding fragment thereof is capable of specifically binding to subdomain 1 of the HER2 extracellular region, comprising: HCDR1 as set forth in SEQ ID NO: 205, HCDR2 as set forth in SEQ ID NO: 206, HCDR3 as set forth in SEQ ID NO: 207, LCDR1 as set forth in SEQ ID NO: 208, LCDR2 as set forth in SEQ ID NO: 209, LCDR3 as set forth in SEQ ID NO: 210; or
wherein the anti-HER2 antibody or antigen-binding fragment thereof is capable of specifically binding to subdomain 1 of the HER2 extracellular region, comprising: HCDR1 as set forth in SEQ ID NO: 211, HCDR2 as set forth in SEQ ID NO: 212, HCDR3 as set forth in SEQ ID NO: 213, LCDR1 as set forth in SEQ ID NO: 214, LCDR2 as set forth in SEQ ID NO: 215, LCDR3 as set forth in SEQ ID NO: 216.

7. The anti-HER2 antibody or antigen-binding fragment thereof of any one of claims 1-3, comprising: a heavy chain variable region VH having at least 80% identity to the amino acid sequence as set forth in SEQ ID NO: 1, 52, 53, 54, 55, 56, 57, 58, 59, 60, 61, 62, 63, 64, 65, 66, 67, 68, 69, 70, 71, 72, 73, 74, 75, 76, 77, 78, 79, or 80; and a light chain variable region VL having at least 80% identity to the amino acid sequence as set forth in SEQ ID NO: 2, 81, 82, 83, 84, 85, 86, 87, 88, 89, 90, 91, 92, 93, 94, 95, 96, or 97;
furthermore, the anti-HER2 antibody or antigen-binding fragment thereof comprising:
(1) amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 1 and the VL as set forth in SEQ ID NO: 2, respectively; or
(2) amino acid sequences having at least 85%, 86%, 87%, 88%, 89%, 90%, 91%, 92%, 93%, 94%, 95%, 96%, 97%, 98%, 99%, or 100% identity to the VH as set forth in SEQ ID NO: 52 and the VL as set forth in SEQ ID NO: 81, 82, 83, 84, 85, 86, 87, 88, 89, 90, 91, 92, 93, 94, 95, 96, or 97, respectively; or
(3) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 53, 54, 55, 56, 57, 58, 59, 60, 61, 62, 63, 64, 65, 66, 67, or 68 and the VL set forth in SEQ ID NO: 81, respectively; or
(4) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 68, 69, 70, 71, 72, 73, 74, 75, 76, 77, 78, 79, or 80 and the VL set forth in SEQ ID NO: 92, respectively;
preferably, the anti-HER2 antibody or antigen-binding fragment thereof comprising amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 73, 78, 79, or 80 and the VL set forth in SEQ ID NO: 92, respectively.

8. The anti-HER2 antibody or antigen-binding fragment thereof of claim 1, 4 or 5, comprising: a heavy chain variable region VH having at least 80% identity to the amino acid sequence as set forth in SEQ ID NO: 3, 143, 144, 145, 146, 147, 148, 149, 150, 151, 152, 153, 154, 155, 156, 157, 158, 159, 160, 161, 162, 163, 164, 165, 166, 167, 168, 169, or 170; and a light chain variable region VL having at least 80% identity to the amino acid sequence as set forth in SEQ ID NO: 4, 171, 172, 173, 174, 175, 176, 177, 178, 179, 180, 181, 182, 183, 184, 185, 186, 187, 188, 189, 190, 191, 192, 193, 194, 195, 196, 197, or 198;
furthermore, the anti-HER2 antibody or antigen-binding fragment thereof comprising:
(1) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 3 and the VL set forth in SEQ ID NO: 4, respectively; or
(2) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 143 and the VL set forth in SEQ ID NO: 171, 172, 173, 174, 175, 176, 177, 178, 179, 180, 181, 182, 183, 184, 185, 186, 187, or 188, respectively; or
(3) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 144, 145, 146, 147, 148, 149, 150, 151, 152, 153, 154, 155, 156, 157, 158, 159, 160, 161, 162, 163, 164, 165, 166, 167, 168, or 170 and the VL set forth in SEQ ID NO: 171, respectively; or
(4) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 169 and the VL set forth in SEQ ID NO: 189, 190, 191, 192, 193, 194, 195, 196, 197, or 198, respectively;
preferably, the anti-HER2 antibody or antigen-binding fragment thereof comprising amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 169 and the VL set forth in SEQ ID NO: 189, 190, 191, 192, 193, 194, 195, 196, 197, or 198, respectively;
more preferably, the anti-HER2 antibody or antigen-binding fragment thereof comprising amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 169 and the VL set forth in SEQ ID NO: 189, 193, 196, or 198, respectively.

9. The anti-HER2 antibody or antigen-binding fragment thereof of claim 1 or 6, comprising a heavy chain variable region VH and a light chain variable region VL, wherein the heavy chain variable region VH and the light chain variable region VL comprise:
(1) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 5 and the VL set forth in SEQ ID NO: 6, respectively; or
(2) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 7 and the VL set forth in SEQ ID NO: 8, respectively; or
(3) amino acid sequences having at least 85%, 86%, 87%, 88%, 89%, 90%, 91%, 92%, 93%, 94%, 95%, 96%, 97%, 98%, 99%, or 100% identity to the VH set forth in SEQ ID NO: 9 and the VL set forth in SEQ ID NO: 10, respectively.

10. An anti-HER2 biparatopic antibody, comprising two antigen-binding domains capable of non-competitively binding to the HER2 extracellular domain simultaneously, wherein the first antigen-binding domain and the second antigen-binding domain each specifically binds to different epitopes on the HER2 extracellular domain, the epitopes are located in subdomains 1, 3 and 4 of the HER2 extracellular region, and the biparatopic antibody comprises at least one of the following characteristics:
(1) the biparatopic antibody is capable of cross-linking/clustering HER2 on the tumor cell surface, thereby triggering rapid and robust internalization of the cross-linked HER2 complexes, as manifested by an internalization rate of more than 60% in HER2-low-expressing tumor cells and more than 80% in HER2-overexpressing tumor cells;
(2) the biparatopic antibody can effectively trigger lysosomal trafficking and degradation of HER2 on the tumor cell surface, significantly reducing the expression of HER2 on cell surface, thereby significantly inhibiting the proliferation of HER2-overexpressing tumor cells;
(3) the biparatopic antibody does not affect HER2 and downstream signaling pathways mediated thereby, including neither inducing, blocking, nor inhibiting ligand-induced HER2 dimerization and the activation of its downstream signaling pathways, and thus the biparatopic antibody does not interfere with the function and regulation of HER2 and downstream signaling pathways mediated thereby in normal tissues/cells including cardiomyocytes, wherein the ligand includes NRG-1;
(4) the biparatopic antibody is capable of specifically binding to HER2 without cross-reacting with the other members of human ErbB/HER family (including EGFR, HER3, and HER4).

11. The biparatopic antibody of claim 10, within the first antigen-binding domain and the second antigen-binding domain, one specifically binds to subdomain 1 of the HER2 extracellular region and the other one specifically binds to subdomain 3 of the HER2 extracellular region;
further, the first antigen-binding domain comprising: (1) HCDR1 having an amino acid sequence of X₁YGMS, wherein X₁ = S, N, or D; (2) HCDR2 having an amino acid sequence of SISGX₂GX₃YX₄KYX₅X₆X₇VKG, wherein X₂ = G or S; X₃ = S or N; X₄ = T or A; X₅ = P, A, G, or V; X₆ = D, G, E, P, Q, or R; X₇ = S, K, or N; (3) HCDR3 having an amino acid sequence of DYX₈GFFDV, wherein X₈ = A, I, N, R, S, or V; (4) LCDR1 having an amino acid sequence of RSSQSLX₉X₁₀SNX₁₁NTYLH, wherein X₉ = V or L; X₁₀ = H or S; X₁₁ = G, A, I, S, R, or T; (5) LCDR2 having an amino acid sequence of KVSNRX₁₂S, wherein X₁₂ = F, D, or P; (6) LCDR3 having an amino acid sequence of X₁₃QSTHVPX₁₄T, wherein X₁₃ = S or Q; X₁₄ = Y or W; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively;
the second antigen-binding domain comprising: (1) HCDR1 having an amino acid sequence of DYX₁₅MH, wherein X₁₅ = S or A; (2) HCDR2 having an amino acid sequence of WINTX₁₆TGX₁₇PTYADX₁₈X₁₉KG, wherein X₁₆ = E, N, G, Y, or I; X₁₇ = E, D, or S; X₁₈ = D, K, or N; X₁₉ = F or V; (3) HCDR3 having an amino acid sequence of VGX₂₀X₂₁X₂₂YAMDY, wherein X₂₀ = R or Y; X₂₁ = Y or G; X₂₂ = D or S; (4) LCDR1 having an amino acid sequence of X₂₃ASQDVYTAVA, wherein X₂₃ = K or R; (5) LCDR2 having an amino acid sequence of X₂₄ASX₂₅RX₂₆T, wherein X₂₄ = S, A, D, E, K, L, Q, R, W, or Y; X₂₅ = Y, D, E, K, N, Q, S, or T; X₂₆ = Y, A, E, P, or Q; (6) LCDR3 having an amino acid sequence of QQX₂₇YSTPPT, wherein X₂₇ = H, S, A, or Y; or amino acid sequences having at least 85%, 86%, 87%, 88%, 89%, 90%, 91%, 92%, 93%, 94%, 95%, 96%, 97%, 98%, 99%, or 100% identity to the HCDR1, HCDR2, HCDR3, LCDR1, LCDR2, and LCDR3, respectively.

12. The anti-HER2 biparatopic antibody of claim 10 or 11, wherein the first antigen-binding domain comprises: HCDR1 as set forth in SEQ ID NO: 11, HCDR2 as set forth in SEQ ID NO: 21, 23, 24, 27, 28, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; the second antigen-binding domain comprises: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 129, 130, 131, 132, 133, 134, 135, 136, 137, or 138, and LCDR3 as set forth in SEQ ID NO: 142;
furthermore, wherein the first antigen-binding domain comprises: HCDR1 as set forth in SEQ ID NO: 11, HCDR2 as set forth in SEQ ID NO: 24, 29, 30, or 31, HCDR3 as set forth in SEQ ID NO: 37, LCDR1 as set forth in SEQ ID NO: 42, LCDR2 as set forth in SEQ ID NO: 46, and LCDR3 as set forth in SEQ ID NO: 49; the second antigen-binding domain comprises: HCDR1 as set forth in SEQ ID NO: 98, HCDR2 as set forth in SEQ ID NO: 102, HCDR3 as set forth in SEQ ID NO: 110, LCDR1 as set forth in SEQ ID NO: 115, LCDR2 as set forth in SEQ ID NO: 133, and LCDR3 as set forth in SEQ ID NO: 142.

13. The anti-HER2 biparatopic antibody of claim 10 or 11, wherein the first antigen-binding domain comprises: a VH as set forth in SEQ ID NO: 70, 72, 73, 76, 77, 78, 79, or 80, and a VL as set forth in SEQ ID NO: 92; or amino acid sequences having at least 85%, 86%, 87%, 88%, 89%, 90%, 91%, 92%, 93%, 94%, 95%, 96%, 97%, 98%, 99%, or 100% identity to the VH and VL, respectively; the second antigen-binding domain comprises: a VH as set forth in SEQ ID NO: 169, and a VL as set forth in SEQ ID NO: 189, 190, 191, 192, 193, 194, 195, 196, 197, or 198; or amino acid sequences having at least 85%, 86%, 87%, 88%, 89%, 90%, 91%, 92%, 93%, 94%, 95%, 96%, 97%, 98%, 99%, or 100% identity to the VH and VL, respectively;
preferably, wherein the first antigen-binding domain comprises: a VH as set forth in SEQ ID NO: 73, 78, 79, or 80, and a VL as set forth in SEQ ID NO: 92; or amino acid sequences having at least 85%, 86%, 87%, 88%, 89%, 90%, 91%, 92%, 93%, 94%, 95%, 96%, 97%, 98%, 99%, or 100% identity to the VH and VL, respectively; the second antigen-binding domain comprises: a VH as set forth in SEQ ID NO: 169, and a VL as set forth in SEQ ID NO: 193; or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

14. The anti-HER2 biparatopic antibody of any one of claims 10-12, having a DVD-Ig format, wherein, with the first antigen-binding domain and the second antigen-binding domain, one is an Fv domain and the other one is a Fab or IgG domain, and the Fv domain is linked to the Fab or IgG domain via a linker;
furthermore, the first antigen-binding domain specifically binds to subdomain 3 of the HER2 extracellular region and is an Fv domain, and the second antigen-binding domain specifically binds to subdomain 1 of the HER2 extracellular region and is a Fab domain or an IgG domain, wherein the first or second antigen-binding domain comprises a heavy chain variable region VH and a light chain variable region VL, the C-terminus of the VH domain in the Fv domain is fused to the N-terminus of the VH domain in the Fab or IgG domain via a linker, and the C-terminus of the VL domain in the Fv domain is fused to the N-terminus of the VL domain in the Fab or IgG domain via a linker.

15. The anti-HER2 biparatopic antibody of any one of claims 10-14, comprising four polypeptide chains:
(1) two of the polypeptide chains comprise VH1-L1-VH2-C-(Fc)n, wherein VH1 represents the heavy chain variable region of the first antigen-binding domain specifically binding to subdomain 3 of the HER2 extracellular region, L1 represents a linker, VH2 represents the heavy chain variable region of the second antigen-binding domain specifically binding to subdomain 1 of the HER2 extracellular region, C represents the heavy chain constant region CH1, Fc represents the heavy chain constant region Fc domain, and n is 0 or 1; and
(2) the other two polypeptide chains comprise VL1-L2-VL2-CL, wherein VL1 represents the light chain variable region of the first antigen-binding domain specifically binding to subdomain 3 of the HER2 extracellular region, L2 represents a linker, VL2 represents the light chain variable region of the second antigen-binding domain specifically binding to subdomain 1 of the HER2 extracellular region, and CL is the IgG light chain constant region;
the L1 and L2 linkers comprising a sequence of (G₄S)ₙ, wherein n represents the copy number of G₄S and is an integer greater than 0, and the copy numbers n of the L1 and L2 linkers may be the same or different.

16. The anti-HER2 biparatopic antibody of claim 15, wherein the VH1 comprises HCDR1 as set forth in SEQ ID NO: 11, HCDR2 as set forth in SEQ ID NO: 24, 29, 30, or 31, and HCDR3 as set forth in SEQ ID NO: 37; the VH2 comprises HCDR1 as set forth in SEQ ID NO: 98, HCDR2 as set forth in SEQ ID NO: 102, and HCDR3 as set forth in SEQ ID NO: 110; the VL1 comprises LCDR1 as set forth in SEQ ID NO: 42, LCDR2 as set forth in SEQ ID NO: 46, and LCDR3 as set forth in SEQ ID NO: 49; and the VL2 comprises LCDR1 as set forth in SEQ ID NO: 115, LCDR2 as set forth in SEQ ID NO: 133, and LCDR3 as set forth in SEQ ID NO: 142.

17. The anti-HER2 biparatopic antibody of claim 15 or 16, wherein the VH1 comprises a VH as set forth in SEQ ID NO: 73, 78, 79, or 80, or an amino acid sequence having at least 85%, 86%, 87%, 88%, 89%, 90%, 91%, 92%, 93%, 94%, 95%, 96%, 97%, 98%, 99%, or 100% identity thereto; the VH2 comprises a VH as set forth in SEQ ID NO: 169, or an amino acid sequence having at least 85%, 86%, 87%, 88%, 89%, 90%, 91%, 92%, 93%, 94%, 95%, 96%, 97%, 98%, 99%, or 100% identity thereto; the VL1 comprises a VL as set forth in SEQ ID NO: 92, or an amino acid sequence having at least 85%, 86%, 87%, 88%, 89%, 90%, 91%, 92%, 93%, 94%, 95%, 96%, 97%, 98%, 99%, or 100% identity thereto; and the VL2 comprises a VL as set forth in SEQ ID NO: 193, or an amino acid sequence having at least 85%, 86%, 87%, 88%, 89%, 90%, 91%, 92%, 93%, 94%, 95%, 96%, 97%, 98%, 99%, or 100% identity thereto.

18. The anti-HER2 biparatopic antibody of any one of claims 15-17, wherein the L1 comprises the amino acid sequence as set forth in SEQ ID NO: 229; the L2 comprises the amino acid sequence as set forth in SEQ ID NO: 230; the Fc comprises an engineered Fc domain, preferably comprising the amino acid sequence of a human IgG1 Fc having amino acid substitutions L234F/L235E/P331S (EU numbering system) as set forth in SEQ ID NO: 232; and the CL is selected from a human κ constant region or a human λ constant region, preferably the human κ constant region having an amino acid sequence as set forth in SEQ ID NO: 233.

19. An anti-HER2 biparatopic antibody-drug conjugate (ADC), comprising the anti-HER2 biparatopic antibody of any one of claims 10-18, a small-molecule toxin compound, and a cleavable linker, wherein the ADC has at least one of the following characteristics:
(1) the ADC is capable of binding to two different epitopes of HER2 on the tumor cell surface, cross-linking HER2 into clusters, and inducing rapid internalization and lysosomal trafficking of the ADC, thereby significantly improving the release of small-molecule toxin compound in the ADC into the target cells, which enables the ADC to gain a broader-spectrum cytotoxic activity, having not only stronger cytotoxic activity than existing HER2-targeting drugs in HER2-overexpressing tumor cells but also direct cytotoxic effects in HER2-low-expressing tumor cells, wherein the existing HER2-targeting drugs include trastuzumab, pertuzumab, T-DM1, and DS-8201;
(2) the ADC exhibits cytotoxic effects against tumors that have developed resistance to or relapsed from treatment of the existing HER2-targeting drugs;
(3) the ADC does not affect the biological activity of HER2 and the regulation of signaling pathways mediated thereby, does not interfere with the normal function of HER2 in cardiomyocytes, and lacks Fc effector functions, thereby exhibiting significantly improved safety in comparison to the existing HER2-targeting drugs;
furthermore, the ADC is represented by formula (I):
Ab-(L-D)ₚ (I)
wherein Ab represents the anti-HER2 biparatopic antibody of any one of claims 10-18, comprising a first and a second antigen-binding domains, wherein the first antigen-binding domain specifically binds to subdomain 3 of the HER2 extracellular region and is an Fv domain, and the second antigen-binding domain specifically binds to subdomain 1 of the HER2 extracellular region and is an IgG domain, and the first or second antigen-binding domain comprises a heavy chain variable region and a light chain variable region;
D represents a small-molecule toxin compound;
L represents a cleavable linker; and
p ranges from 2 to 8.

20. The ADC of claim 19, wherein the first antigen-binding domain comprises: (1) HCDR1 as set forth in SEQ ID NO: 11, (2) HCDR2 as set forth in SEQ ID NO: 24, 29, 30, or 31, (3) HCDR3 as set forth in SEQ ID NO: 37, (4) LCDR1 as set forth in SEQ ID NO: 42, (5) LCDR2 as set forth in SEQ ID NO: 46, and (6) LCDR3 as set forth in SEQ ID NO: 49; the second antigen-binding domain comprises: (1) HCDR1 as set forth in SEQ ID NO: 98, (2) HCDR2 as set forth in SEQ ID NO: 102, (3) HCDR3 as set forth in SEQ ID NO: 110, (4) LCDR1 as set forth in SEQ ID NO: 115, (5) LCDR2 as set forth in SEQ ID NO: 133, and (6) LCDR3 as set forth in SEQ ID NO: 142.

21. The ADC of claim 19 or 20, wherein the first antigen-binding domain comprises a VH as set forth in SEQ ID NO: 73, 78, 79, or 80 and a VL as set forth in SEQ ID NO: 92, or amino acid sequences having at least 85%, 86%, 87%, 88%, 89%, 90%, 91%, 92%, 93%, 94%, 95%, 96%, 97%, 98%, 99%, or 100% identity to the VH and VL, respectively; the second antigen-binding domain comprises a VH as set forth in SEQ ID NO: 169 and a VL as set forth in SEQ ID NO: 193, or amino acid sequences having at least 85%, 86%, 87%, 88%, 89%, 90%, 91%, 92%, 93%, 94%, 95%, 96%, 97%, 98%, 99%, or 100% identity to the VH and VL, respectively.

22. The ADC of any one of claims 19-21, wherein the Ab further comprises constant regions, including a heavy chain constant region and a light chain constant region, the heavy chain constant region comprising the human IgG1 Fc as set forth in SEQ ID NO: 232, and the light chain constant region comprising the human κ constant region as set forth in SEQ ID NO: 233.

23. The ADC of any one of claims 19-22, wherein the small-molecule toxin compound includes eribulin, auristatin derivatives, tubulysins, cryptomycins, maytansinoid derivatives, topoisomerase inhibitors, pyrrolobenzodiazepines (PBD), calicheamicin and derivatives thereof, duocarmycins, alkylating chemotherapeutic agents and other compounds with alkylating forms, antimetabolites, mitotic inhibitors, and cytotoxic antibiotics;
furthermore, the auristatin derivatives including MMAE, MMAF, and MMAD; the maytansinoid derivatives including DM1, DM2, DM3, and DM4; the topoisomerase inhibitors including camptothecin derivatives SN-38, exatecan, and DXd; the alkylating chemotherapeutic agents and other compounds with alkylating forms including nitrogen mustards, ethylenimine compounds, alkyl sulfonates, nitrosoureas, cisplatin, and dacarbazine; the antimetabolites including folic acid, purine, or pyrimidine antagonists; the mitotic inhibitors including vinca alkaloids and derivatives of podophyllotoxin; the cytotoxic antibiotics including anthracycline antibiotics, actinomycins, and bleomycin; a preferred small-molecule toxin compound being eribulin.

24. The ADC of any one of claims 19-23, wherein the cleavable linker comprises a cleavable peptide moiety and at least one spacer;
furthermore, the cleavable peptide moiety comprising amino acid units, including amino acid sequences of Phe-Lys, Val-Cit (VC), Glu-Val-Cit, or Gly-Gly-Phe-Gly (GGFG), cleavable by cathepsin B;
the spacer comprising a spacer conjugated to the antibody, and/or a second spacer conjugated to the small-molecule toxin compound, wherein the spacer conjugated to the antibody is a hydrophilic spacer comprising one or more polyethylene glycols (PEG), and the second spacer comprises a p-aminobenzyl unit.

25. The ADC of claim 24, wherein the amino acid units include VC and GGFG; the hydrophilic spacer comprises 2 PEG moieties, and the hydrophilic spacer and maleimide (Mal) form a Mal-spacer that attaches to the antibody; the second spacer comprises p-aminobenzyl carbonyl (PAB).

26. The ADC of claim 24 or 25, wherein the cleavable linker comprises a Mal-spacer and a cleavable peptide moiety, the Mal-spacer comprising 2 PEGs, and the cleavable peptide moiety including the dipeptide VC and the tetrapeptide GGFG; or the cleavable linker comprises a Mal-spacer, a cleavable peptide moiety, and a second spacer, the Mal-spacer comprising 2 PEGs, the cleavable peptide moiety comprising the dipeptide VC, and the second spacer comprising PAB.

27. The ADC of any one of claims 24-26, wherein the cleavable linker includes Mal-(PEG)₂-VC, Mal-(PEG)₂-GGFG, or Mal-(PEG)₂-VC-PAB.

28. The ADC of any one of claims 24-27, wherein the cleavable peptide moiety of the cleavable linker can be coupled directly or via the second spacer to the small-molecule toxin compound to prepare an ADC comprising a drug payload, the small-molecule toxin compound being eribulin.

29. The ADC of claim 28, wherein the cleavable linker comprises Mal-(PEG)₂-GGFG, and the drug payload comprises a compound Mal-(PEG)₂-GGFG-Eribulin obtained by coupling the cleavable peptide moiety GGFG of the cleavable linker to the C-35 amine of eribulin via a carboxyl group; or
the cleavable linker comprises Mal-(PEG)₂-VC-PAB, and the drug payload comprises a compound Mal-(PEG)₂-VC-PAB-Eribulin obtained by coupling the cleavable linker to the C-35 amine of eribulin via the second spacer PAB.

30. The ADC of any one of claims 19-29, wherein p is 4 to 8.

31. A nucleic acid encoding the anti-HER2 antibody or antigen-binding fragment thereof of any one of claims 1-9, or the anti-HER2 biparatopic antibody of any one of claims 10-18, or the antibody component of the ADC of any one of claims 19-30.

32. An expression vector capable of expressing the nucleic acid of claim 31.

33. A host cell comprising the nucleic acid of claim 31 or the expression vector of claim 32.

34. A method of preparing the anti-HER2 antibody or antigen-binding fragment thereof of any one of claims 1-9, the anti-HER2 biparatopic antibody of any one of claims 10-18, or the antibody component of the ADC of any one of claims 19-30 using the host cell of claim 33, comprising: (1) expressing the anti-HER2 antibody or antigen-binding fragment thereof, the anti-HER2 biparatopic antibody, or the antibody component of the ADC in the host cell; and (2) isolating the anti-HER2 antibody or antigen-binding fragment thereof, the anti-HER2 biparatopic antibody, or the antibody component of the ADC from the host cell or cell culture.

35. A method for preparing an ADC of any one of claims 19 to 30, comprising: reacting a nucleophilic group or an electrophilic group of the small-molecule toxin compound with a cleavable linker to form a drug payload via a covalent bond, followed by reacting with a nucleophilic group or an electrophilic group of the antibody; or preferably, reacting free cysteine residues generated by reduction of interchain disulfide bonds in the hinge region of the antibody with a reactive functional group of the drug payload to form a covalent bond, wherein the reactive functional group comprises a maleimide group.

36. A pharmaceutical composition comprising the anti-HER2 antibody or antigen-binding fragment thereof of any one of claims 1-9, the anti-HER2 biparatopic antibody of any one of claims 10-18, or the anti-HER2 biparatopic ADC of any one of claims 19-30, and a pharmaceutically acceptable carrier.

37. A kit comprising an effective amount of the anti-HER2 antibody or antigen-binding fragment thereof of any one of claims 1-9, the anti-HER2 biparatopic antibody of any one of claims 10-18, the anti-HER2 biparatopic ADC of any one of claims 19-30, or the pharmaceutical composition of claim 36, and optionally at least one additional tumor therapeutic agent;
furthermore, wherein the additional tumor therapeutic agent includes a HER2 antagonist, an EGFR antagonist, a HER3 antagonist, a MET antagonist, an IGF1R antagonist, a B-Raf inhibitor, a PDGFR-α inhibitor, a PDGFR-β inhibitor, a PDGF ligand inhibitor, a VEGF antagonist, a VEGF receptor kinase inhibitor, a DLL4 antagonist, an Ang2 antagonist, a FOLH1 antagonist, a STEAP1 or STEAP2 antagonist, a TMPRSS2 antagonist, a MSLN antagonist, a MUC16 antagonist, a CLEC12A antagonist, a PD-1 or PD-L1 blocker, a hormone receptor modulator, an aromatase inhibitor, a kinase inhibitor, a cytokine agonist or a cytokine inhibitor, and a chemotherapeutic agent.

38. A method of treating HER2-expressing cancers, comprising administering to a subject in need thereof an effective amount of the anti-HER2 biparatopic antibody of any one of claims 10-18, the anti-HER2 biparatopic ADC of any one of claims 19-30, the pharmaceutical composition of claim 36, or the kit of claim 37.

39. The method of claim 38, wherein the cancers include breast cancer, ovarian cancer, cervical cancer, colorectal cancer, gastric cancer, esophageal cancer, lung cancer, head and neck cancer, melanoma, pancreatic cancer, liver cancer, bile duct cancer, kidney cancer, bladder cancer, thyroid cancer, prostate cancer, and endometrial cancer, wherein the cancers also include cancers at all stages, such as early-stage cancer, non-metastatic cancer, primary cancer, advanced cancer, locally advanced cancer, metastatic cancer, or cancer in remission; wherein the cancers further include HER2-overexpressing cancer, cancers expressing relatively low levels of HER2, cancers unresponsive or poorly responsive to existing HER2-targeting therapeutics, and/or cancers that are resistant to or relapsed from existing HER2-targeting therapeutics, wherein the existing HER2-targeting therapeutics include trastuzumab, pertuzumab, T-DM1, and DS-8201;
wherein the subject can be a human, a non-human primate, or other mammals such as dogs, mice, or rats; the subject further includes patients who are ineligible for or refractory to existing HER2-targeting therapies, or resistant to or relapsed from existing HER2-targeting therapies.

40. A method for detecting and/or measuring HER2 or HER2-expressing tumor cells in a sample, and a method for screening cancer patients potentially responsive to treatment with the ADC of any one of claims 19-30, comprising incubating the anti-HER2 antibody or antigen-binding fragment thereof of any one of claims 1-9, or the anti-HER2 biparatopic antibody of any one of claims 10-18 with the sample or a biological specimen isolated from the patient, and detecting whether the antibody binds to the sample or biological specimen.
